(19) Europäisches Patentamt
European Patent Office
Office européen des brevets

(11) Veröffentlichungsnummer: **0 062 001**
**B1**

# (12) EUROPÄISCHE PATENTSCHRIFT

(45) Veröffentlichungstag der Patentschrift: **27.05.87**

(21) Anmeldenummer: **82810126.1**

(22) Anmeldetag: **18.03.82**

(51) Int. Cl.$^4$: **C 07 D 215/56,**
C 07 D 215/48,
C 07 D 401/02,
C 07 D 405/02,
C 07 D 409/02, A 61 K 31/47

## (54) Acyl-chinolinonderivate, Verfahren zu ihrer Herstellung, diese enthaltende pharmazeutische Präparate und ihre Verwendung.

(30) Priorität: **24.03.81 CH 1986/81**

(43) Veröffentlichungstag der Anmeldung:
**06.10.82 Patentblatt 82/40**

(45) Bekanntmachung des Hinweises auf die Patenterteilung:
**27.05.87 Patentblatt 87/22**

(84) Benannte Vertragsstaaten:
**AT BE CH DE FR IT LI LU NL SE**

(56) Entgegenhaltungen:
**DE-A-1 670 464**
**DE-A-2 658 544**
**DE-A-2 806 879**
**GB-A- 830 832**
**GB-A-1 334 705**

**J.Med.Chem. 17 685-690 (1974)**

**J.Med.Chem. 21, 984-988 (1978)**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Göschke, Richard, Dr.**
**Felixhäglistrasse 21**
**CH-4103 Bottmingen (CH)**
Erfinder: **Ferrini, Pier Giorgio, Dr.**
**Im Rehwechsel 22**
**CH-4102 Binningen (CH)**
Erfinder: **Sallmann, Alfred, Dr.**
**Joachimsackerstrasse 12**
**CH-4103 Bottmingen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 062 001 B1

**Beschreibung**

Die Erfindung betrifft neue Chinolinonderivate der Formel (I),

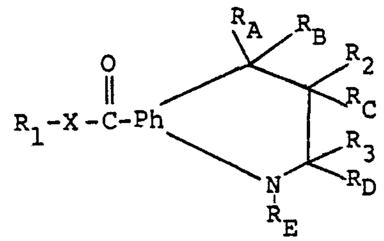

(I),

worin $R_1$ gegebenenfalls durch Hydroxy, Niederalkoxy, Hydroxyniederalkoxy, Niederalkoxyniederalkoxy, Hydroxyniederalkoxyniederalkoxy, Niederalkoxyniederalkoxyniederalkoxy, Niederalkylthio, Niederalkansulfinyl oder Niederalkansulfonyl substituiertes Niederalkyl, Niederalkanoyloxymethyl, Niederalkenyl oder Cycloalkyl, gegenbenenfalls im Phenylteil durch Niederalkyl, Niederalkoxy und/oder Halogen substituiertes Phenyl oder Phenylniederalkyl, Furyl, Thienyl oder Pyridyl oder Furylniederalkyl, Thienylniederalkyl, Pyridylniederalkyl oder Wasserstoff bedeutet, X ein direkte Bindung oder Oxy darstellt, Ph gegebenenfalls zusätzlich durch Niederalkyl, Niederalkoxy, Hydroxy und/oder Halogen substituiertes 1,2-Phenylen bedeutet, $R_2$ Carboxy, Niederalkoxycarbonyl, Hydroxyniederalkoxycarbonyl, Niederalkoxyniederalkoxycarbonyl, Diniederalkylaminoniederalkoxy- oder 5- bis 7-gliedriges Niederalkylenamino- bzw. Aza-, Oxa- oder Thianiederalkylenaminoniederalkoxycarbonyl oder amidiertes Carboxy bedeutet, das als Aminogruppe Amino, Hydroxyamino, Niederalkylamino, Diniederalkylamino oder 5- bis 7-gliedriges Niederalkylen- bzw. Aza-, Oxa-, Thianiederalkylenamino aufweist, und worin entweder $R_A$ und $R_B$ gemeinsam Oxo darstellen, $R_C$ und $R_D$ gemeinsam eine zusätzliche Bindung bedeuten und $R_3$ Hydroxy ist bze. $R_C$ Wasserstoff ist und $R_3$ und $R_D$ gemeinsam Oxo darstellen und $R_E$ Wasserstoff, Niederalkyl, Hydroxyniederalkyl, Niederalkoxyniederalkyl, Diniederalkylaminoniederalkyl, 5- bis 7-gliedriges Niederalkylenamino- bzw. Aza-, Oxa- oder Thianiederalkylenaminoniederalkyl, Niederalkenyl, Niederalkinyl, Cycloalkyl mit 3 bis 8, vor allem 5 bis 7, Ringgliedern oder gegebenenfalls durch Niederalkyl, Niederalkoxy und/oder Halogen substituiertes Phenylniederalkyl, Furylniederalkyl, Thienylniederalkyl oder Pyridylniederkyl darstellt, oder $R_A$ Hydroxy oder Niederalkoxy bedeutet, $R_B$ gemeinsam mit $R_C$ sowie $R_D$ gemeinsam mit $R_E$ jeweils eine zusätzliche Bindung darstellen und $R_3$ Hydroxy ist bzw. $R_B$ und $R_C$ gemeinsam eine zusätzliche Bindung und $R_3$ und $R_D$ gemeinsam Oxo darstellen und $R_E$ Wasserstoff, Niederalkyl, Hydroxyniederalkyl, Niederalkoxyniederalkyl, Diniederalkylaminoniederalkyl, 5- bis 7-gliedriges Niederalkylenamino- bzw. Aza-, Oxa- oder Thianiederalkylenaminoniederalkyl, Niederalkenyl, Niederalkinyl, Cycloalkyl mit 3—8 Ringgliedern oder gegebenenfalls durch Niederalkyl, Niederalkoxy und/oder Halogen substituiertes Phenylniederalkyl, Furylniederalkyl, Thienylniederalkyl oder Pyridylniederalkyl darstellt, wobei "niedere" Reste jeweils bis und mit 7 Kohlenstoffatome aufweisen, und Salze von salzbildenden Verbindungen der Formel (I), Verfahren zu ihrer Herstellung, Verbindungen der Formel (I) oder ihre Salze enthaltende pharmazeutische Präparate und die Verwendung von Verbindungen der Formel (I) und ihrer Salze als Arzneimittel oder zur Herstellung von Arzneimitteln auf nicht-chemischen Wege.

Vor- und nachstehend werden unter "niederen" organischen Resten und Verbindungen solche verstanden, die bis und mit 7, vor allem bis und mit 4, Kohlenstoffatome aufweisen.

Niederalkyl bedeutet z.B. Methyl, Aethyl, Propyl, Isopropyl, Butyl, Isobutyl, Secundärbutyl, Tertiärbutyl, Pentyl, Hexyl oder Heptyl.

Niederalkenyl bedeutet z.B. Vinyl, 1-Methylvinyl, 1-Aethylvinyl, Allyl oder 2- oder 3-Methallyl.

Niederalkinyl bedeutet z.B. Aethinyl oder Prop-2-inyl (Propargyl).

Niederalkoxy sowie Niederalkoxy in Niederalkoxycarbonyl bedeutet z.B. Methoxy, Aethoxy, Propyloxy, Isopropyloxy, Butyloxy, Isobutyloxy, Secundärbutyloxy, Tertiärbutyloxy, Pentyloxy, Hexyloxy oder Heptyloxy. Niederalkylthio ist. z.B. Methylthio, Aethylthio, Propylthio, Butylthio, Pentylthio, Hexylthio oder Heptylthio.

Hydroxyniederalkyl ist beispielsweise 2- und/oder 3-Hydroxyniederalkyl, z.B. 2-Hydroxyäthyl, 3-Hydroxypropyl oder 2,3-Dihydroxypropyl. Entsprechend ist Hydroxyniederalkoxy sowie Hydroxyniederalkoxy in Hydroxyniederalkoxycarbonyl insbesondere 2- und/oder 3-Hydroxyniederalkoxy, z.B. 2-Hydroxyäthoxy, 3-Hydroxypropyloxy oder 2,3-Dihydroxypropyloxy, und Hydroxyniederalkoxyniederalkoxy insbesondere ω-(ω-Hydroxyniederalkoxy)-niederalkoxy, z.B. 2-(2-Hydroxyäthoxy)-äthoxy.

Niederalkoxyniederalkyl ist beispielsweise 2-und/oder 3-Niederalkoxyniederalkyl, z.B. 2-Methoxyäthyl, 2-Aethoxyäthyl oder 3-Methoxypropyl. Entsprechend ist Niederalkoxyniederalkoxy sowie solches in Niederalkoxyniederalkoxycarbonyl insbesondere 2- und/oder 3-Niederalkoxyniederalkoxy z.B. 2-Methoxyäthoxy, 2-Aethoxyäthoxy oder 3-Methoxypropyloxy, und Niederalkoxyniederalkoxyniederalkoxy insbesondere ω-(ω-Niederalkoxyniederalkoxy)-niederlalkoxy, z.B. 2-(2-methoxyäthoxy)-äthoxy.

Diniederalkylaminoniederalkyl ist beispielsweise 2- und/oder 3-Diniederalkylaminoniederalkyl, z.B. 2-Dimethylaminoäthyl, 2-Diäthylaminoäthyl oder 3-Dimethylaminopropyl. Entsprechend ist Diniederalkylaminoniederalkoxy sowie solches in Diniederalkylaminoniederalkoxycarbonyl insbesondere 2- und/oder 3-Diniederalkylaminoniederalkoxy, z.B. 2-Dimethylaminoäthoxy, 2-Diäthylaminoäthoxy oder oder 3-Dimethylaminopropyloxy.

Phenylniederalkyl ist beispielsweise Benzyl oder 1- oder 2-Phenyläthyl und Phenylniederalkoxy sowie solches in Phenylniederalkoxycarbonyl z.B. Benzyloxy oder 1- oder 2-Phenyläthoxy.

Cycloalkyl ist insbesondere Cycloalkyl mit 3 bis 8, vor allem 5 bis 7, Ringgliedern, z.B. Cyclopentyl, Cyclohexyl oder Cycloheptyl, ferner Cyclopropyl, Cyclobutyl, oder Cyclooctyl.

Furyl ist z.B. 2-Furyl, entsprechend Thienyl- z.B. 2-Thienyl, wohingegen Pyridyl 2-, 3- oder 4-Pyridyl sein kann.

Furylniederalkyl, Thienylniederalkyl, bzw. Pyridylniederalkyl weist als Alkylteil insbesondere Methyl auf und stellt beispielsweise Furfuryl, 2-Thenyl oder Pyridylmethyl, wie 2- oder 4-Pyridylmethyl, dar.

Mono- oder Diniederalkylamino bedeutet z.B. Methylamino, Diäthylamino, Aethylamino, Diäthylamino, Propylamino oder Butylamino.

Niederalkylenamino bzw. Aza-, Oxa- oder Thianiederalkylenamino sowie solches in entsprechend substituierten Niederalkyl- bzw. Niederalkoxyresten bedeutet z.B. Pyrrolidino, Piperidino, Morpholino, Thiamorpholino, oder N-Niederalkyl-, wie N'-Methylpiperazino.

Niederalkanoyloxy ist z.B. Acetoxy, Propionyloxy, Butyryloxy oder Pivaloyloxy.

Halogen ist insbesondere Halogen bis und mit Auomnummer 35 wie Fluor, Chlor oder Brom.

Salzbildende Verbindungen der Formel (I) sind beispielsweise Carboxy $R_2$ bzw. $R_1$—X—C(=O)— und/oder enolische Hydroxygruppen, z.B. Hydroxy $R_A$, aufweisende saure Verbindungen oder basische Gruppen, gegebenenfalls neben einer geringeren Zahl saurer Gruppen, aufweisende basische Verbindungen der Formel (I). Salze derselben sind beispielsweise Salze von sauren Verbindungen mit Basen, insbesondere pharmazeutisch verwendbare Salze, wie Alkalimetall- oder Erdalkalimetall-, z.B. Natrium Kalium, Magnesium- oder Calciumsalze, ferner Ammoniumsalze mit Ammoniak oder Aminen, wie Niederalkyl- oder Hydroxyniederalkylaminen, z.B. Trimethylamin, Träthylamin oder Di- oder Tri(2-hydroxyäthyl)-amin bzw. Säureadditionssalze, insbesondere pharmazeutisch verwendbare Mineralsäure, z.B. Halogenwasserstoffsäure, Schwefelsäure oder Phosphorsäure, Additionssalze oder Säureadditionssalze mit geeigneten organischen Sulfon- oder Carbonsäuren, beispielsweise Hydrochloride, Hydrobromide, Sulfate, Phosphate, Maleate, Maleinate, Fumarate oder Tartrate.

Die Erfindung betrifft beispielsweise solche Verbindungen der Formel I, worin X eine direkte Bindung darstellt und $R_1$, $R_2$, $R_3$, $R_A$, $R_B$, $R_C$, $R_D$ und $R_E$ die eingangs angegebenen Bedeutungen haben, $R_1$ jedoch von Wasserstoff verschieden ist, oder worin X für Oxy steht, $R_1$ Niederalkyl oder gegebenenfalls durch Niederalkyl, Niederalkoxy, Hydroxy und/oder Halogen substituiertes Phenyl darstellt, und $R_2$, $R_3$, $R_A$, $R_B$, $R_C$, $R_D$ und $R_E$ die eingangs angegebenen Bedeutungen haben, und ihre Salze, Verfahren zu ihrer Herstellung, sie enthaltende pharmazeutischen Präparate und ihre Verwendung als Arzneimittlewirkstoffe.

Die Erfindung betrifft in erster Linie Verbindungen der Formel

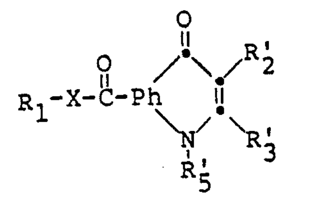

(Ia),

worin $R_1$ Niederalkyl mit bis zu 7 Kohlenstoffatomen, z.B. Propyl, Isopropyl, Butyl, Isobutyl, Tertiärbutyl, Secundärbutyl oder Pentyl, Niederalkoxy-, Niederalkylthio-, Niederalkansulfinyl-, oder Niederalkansulfonylniederalkyl mit jeweils bis zu 4 Kohlenstoffatomen in den Alkylteilen, z.B. Methansulfinyl- oder Methansulfonylmethyl, Niederalkenyl mit bis zu 4 Kohlenstoffatomen, z.B. allyl, 3-8-gliedriges Cycloalkyl, z.B. Cyclopentyl, Cyclohexyl oder Cycloheptyl, gegebenenfalls durch Niederalkyl mit bis zu 4 Kohlenstoffatomen, z.B. Methyl, Niederalkoxy mit bis zu 4 Kohlenstoffatomen, z.B. Methoxy, und/oder Halogen bis einschliesslich Atomnummer 35, z.B. Chlor oder Brom, substituiertes Phenyl bzw. Phenylniederalkyl mit bis zu 4 Kohlenstoffatomen im Alkylteil, z.B. entsprechendes Benzyl oder 1- oder 2-Phenyläthyl, Furyl, Thienyl oder Pyridyl, z.B. 2-Furyl, 2-Thienyl oder 2-, 3- oder 4-Pyridyl, oder Furyl-, Thienyl- oder Pyridylniederalkyl mit bis zu 4 Kohlenstoffatomen im Alkylteil, z.B. Furfuryl, 2-Thenyl oder 2- oder 4-Picolyl, und X eine direkte Bindung bedeutet oder $R_1$ Wasserstoff oder Niederalkyl mit bis zu 7 Kohlenstoffatomen und X Oxy darstellt, und worin Ph die Gruppe $R_1$—X—C(=O)— aufweisendes, gegebenenfalls zusätzlich durch Niederalkyl bis zu 4 Kohlenstoffatomen, z.B. Methyl, Niederalkoxy mit bis zu 4 Kohlenstoffatomen, z.B. Methoxy, Hydroxy oder Halogen bis einschliesslich Atomnummer 35, z.B. Chlor, substituiertes 1,2- Phenylen darstellt, $R_2'$ für Carboxy, Niederalkoxycarbonyl mit bis zu 5 Kohlenstoffatomen, z.B. Methoxy- oder Aethoxycarbonyl, Hydroxyniederalkoxycarbonyl mit bis zu 5 Kohlenstoffatomen, z.B. 2-Hydroxyäthoxycarbonyl, Niederalkoxyniederalkoxycarbonyl mit bis zu 4 Kohlenstoffatomen in den Alkoxyteilen, z.B. 2-Methoxy- oder 2-Aethoxyäthoxycarbonyl, Diniederalkylaminoniederalkoxycarbonyl mit jeweils bis zu 4 Kohlenstoffatomen im Alkyl- und Alkoxyteil, z.B. 2-Dimethylaminoäthoxy- oder 2-Diäthylaminoäthoxycarbonyl, Carbamoyl, N-Hydroxycarbamoyl oder N-Niederalkyl- bzw. N,N-Diniederalkylcarbamoyl mit bis zu 4 Kohlenstoffatomen

im Alkylteil, z.B. N-Methyl-, N-Aethyl- oder N,N-Dimethylcarbarbamoyl, steht, $R_3'$ Hydroxy darstellt und $R_4'$ Wasserstoff, Niederalkyl oder Niederalkenyl mit bis zu 4 Kohlenstoffatomen, z.B. Methyl, Aethyl oder Allyl, gegebenenfalls durch Niederalkyl mit bis zu 4 Kohlenstoffatomen, z.B. Methyl, Niederalkoxy mit bis zu 4 Kohlenstoffatomen, z.B. Methoxy, und/oder Halogen bis und mit Atomnummer 35, z.B. Chlor, substituiertes Phenyl-, Furyl-, Thienyl- oder Pyridylniederalkyl mit bis zu 4 Kohlenstoffatomen im Alkylteil, z.B. entsprechendes Benzyl, 1- oder 2-Phenyläthyl, Furfuryl, 2-Thenyl- oder 2- oder 4-Picolyl, Niederalkoxyniederalkyl mit bis zu 4 Kohlenstoffatomen im Alkoxy- bzw. Alkylteil, z.B. 2-Methoxy- oder 2-Aethoxyäthyl, oder Diniederalkylaminoniederalkyl, worin Niederalkyl bis zu 4 Kohlenstoffatome aufweist, z.B. 2-Dimethylamino- oder 2-Diäthylaminoäthyl, bedeutet, sowie Tautomere derselben, in denen eine Hydroxygruppe $R_3$ in der tautomeren Oxoform vorliegt, und Salze von salzbildenden Verbindungen der Formel Ia oder ihrer Tautomeren.

Die Erfindung betrifft insbesondere Verbindungen der Formel

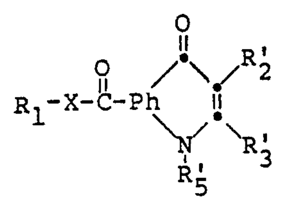

(Ia),

worin $R_1$ Wasserstoff, Niederalkyl mit bus zu 7 Kohlenstoffatomen, z.B. Propyl, Isopropyl, Butyl, Isobutyl, Tertiärbutyl, Secundärbutyl oder Pentyl, Niederalkoxy-, Niederalkylthio-, Niederalkansulfinyl- oder Niederalkansulfonylniederalkyl mit jeweils bis zu 4 Kohlenstoffatomen in den Alkylteilen, z.B. Methansulfinyl- oder Methansulfonylmethyl, Niederalkenyl mit bis zu 4 Kohlenstoffatomen, z.B. Allyl, oder 3-8-gliedriges Cycloalkyl, z.B. Cyclopentyl, Cyclohexyl oder Cycloheptyl, und X eine direkte Bindung oder $R_1$ Wasserstoff, Niederalkyl mit bis zu 7 Kohlenstoffatomen, z.B. Propyl, Isopropyl, Butyl, Isobutyl, Tertiärbutyl, Secundärbutyl oder Pentyl, Niederalkoxy-, Niederalkylthio-, Niederalkansulfinyl-, oder Niederalkansulfonylniederalkyl mit jeweils bis zu 4 Kohlenstoffatomen in den Alkylteilen, z.B. Methansulfinyl- oder Methansulfonylmethyl, Niederalkenyl mit bis zu 4 Kohlenstoffatomen, z.B. Allyl, 3-8-gliedriges Cycloalkyl, z.B. Cyclopentyl, Cyclohexyl oder Cycloheptyl, Niederalkanoyloxymethyl mit bis und mit 7 Kohlenstoffatomen im Alkanoylteil, wie Acetoxy- oder Pivaloyloxymethyl, oder ω-(ω-Hydroxyniederalkoxy)-niederalkyl, ω-(ω-Niederalkoxyniederalkoxy)-niederalkyl, ω-[ω-(ω-Hydroxyniederalkoxy)-niederalkoxy]-niederalkyl oder ω-[ω-(ω-Niederalkoxyniederalkoxy)-niederalkoxy]-niederalkyl mit jeweils 1 bis 4 Kohlenstoffatomen im Alkyl- und 2 bis 4 Kohlenstoffatomen im Alkylenteil, wie 2-(2-Hydroxyäthoxy)-äthyl, 2-(2-Methoxyäthoxy)-äthyl, 2-[2-(2-Hydroxyäthoxy)-äthoxy]-äthyl oder 2-[2-(2-Methoxyäthoxy)-äthoxy]-äthyl, und X Oxy darstellt, und worin Ph die Gruppe $R_1$—X—CO— aufweisendes, gegebenenfalls, zusätzlich durch Niederalkyl mit bis zu 4 Kohlenstoffatomen, z.B. Methyl, Niederalkoxy mit bis zu 4 Kohlenstoffatomen, z.B. Methoxy, Hydroxy oder Halogen bis einschliesslich Atomnummer 35, z.B. Chlor, substituiertes 1,2-Phenylen darstellt, $R_2'$ für Carboxy, Niederalkoxycarbonyl mit bis zu 5 Kohlenstoffatomen, z.B. Methoxy- oder Aethoxycarbonyl, Niederalkoxyniederalkoxycarbonyl mit bis zu 4 Kohlenstoffatomen in den Alkoxyteilen, z.B. 2-Methoxy- oder 2-Aethoxyäthoxycarbonyl, Carbamoyl, N-Hydroxycarbamoyl oder N-Niederalkyl- bzw. N,N-Diniederalkylcarbamoyl mit bis zu 4 Kohlenstoffatomen im Alkylteil, z.B. N-Methyl-, N-Aethyl- oder N,N-Dimethylcarbamoyl, steht, $R_3'$ Hydroxy darstellt und $R_5'$ Wasserstoff, Niederalkyl mit bis und mit 4 Kohlenstoffatomen, z.B. Aethyl, Niederalkenyl mit bis und mit 4 Kohlenstoffatomen, wie Allyl, oder Niederalkinyl mit bis und mit 4 Kohlenstoffatomen, wie Proparyl, bedeutet, sowie Tautomere derselben, in denen eine Hydroxygruppe $R_3'$ in der tautomeren Oxoform vorliegt, und Salze von salzbildenden Verbindungen der Formel Ia oder ihrer Tautomeren.

Die Erfindung betrifft vor allem Verbindungen der Formel (II)

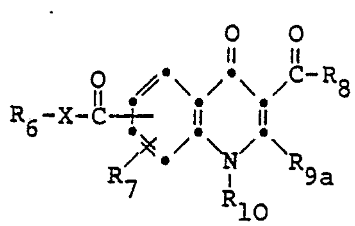

(II),

worin X eine direkte Bindung und $R_6$ Wasserstoff oder Niederalkyl mit bis zu 7 Kohlenstoffatomen, z.B. Propyl, Isopropyl oder insbesondere Butyl, ferner Isobutyl, Sekundärbutyl oder Pentyl, bedeutet, oder X für Oxy und $R_6$ für Wasserstoff, Niederalkyl, mit bis zu 7 Kohlenstoffatomen, z.B. Propyl, Isopropyl oder insbesondere Butyl, ferner Isobutyl, Sekundärbutyl oder Pentyl, ω-Hydroxyniederalkyl mit 2 bis 4 Kohlenstoffatomen, wie 2-Hydroxyäthyl, Niederalkanoyloxymethyl mit bis und mit 7 Kohlenstoffatomen im

Alkanoylteil, wie Acetoxy- oder Pivaloyloxymethyl, oder ω-(ω-Hydroxyniederalkoxy)-niederalkyl, ω-(ω-Niederalkoxyniederalkoxy)-niederalkyl, ω-[ω-(ω-Hydroxyniederalkoxy)-niederalkoxy]-niederalkyl oder ω-[ω-(ω-Niederalkoxyniederalkoxy)-niederalkoxy]-niederalkyl mit jeweils 1 bis 4 Kohlenstoffatomen im Alkyl- und 2 bis 4 Kohlenstoffatomen im Alkylenteil, wie 2-(2-Hydroxyäthoxy)-äthyl, 2-(2-Methoxyäthoxy)-äthyl, 2-[2-(2-Hydroxyäthoxy)-äthoxy]-äthyl oder 2-[2-(2-Methoxyäthoxy)-äthoxy]-äthyl, $R_7$ Wasserstoff oder insbesondere Niederalkyl mit bis und mit 4 Kohlenstoffatomen, z.B. Methyl, oder ferner Niederalkoxy mit bis und mit 4 Kohlenstoffatomen, z.B. Methoxy, Halogen der Atomnummer bis und mit 35, z.B. Chloro, oder Hydroxy darstellt, $R_8$ Hydroxy oder Niederalkoxy mit bis zu 4 Kohlenstoffatomen, z.B. Methoxy oder Aethoxy, bedeutet, $R_{9a}$ Hydroxy darstellt und $R_{10}$ Niederalkyl, Niederalkenyl oder Niederalkinyl mit jeweils bis und mit 4 Kohlenstoffatomen, z.B. Aethyl, Allyl oder Propargyl, bedeutet, wobei 2-Hydroxy-4-oxo-1,4-dihydrochinolinverbindungen der Formel II auch in der tautomeren 2-Oxo-4-hydroxy-1,2-dihydro- oder 2,4-Dioxo-1,2,3,4,-tetrahydrochinolinform vorliegen Können, ganz besonders solche, in denen sich der Rest der Formel $R_6$—X—C(=O)— in 6-Stellung und ein von Wasserstoff verschiedener Rest $R_7$ sich in 5- oder 7-Stellung des Chinolin-Ringgerüstes befindet, und Salze von salzbildenden Verbindungen der Formel II bzw. ihrer Tautomeren.

Die Erfindung betrifft im besonderen Masse einerseits Verbindungen der Formel (II), worin X Oxy darstellt, die Gruppe $R_6$—O—C(=O)— in 6-Stellung gebunden ist, $R_6$ Wasserstoff, Niederalkyl mit bis zu 7 Kohlenstoffatomen, z.B. Butyl, Isobutyl oder Sekundärbutyl, ω-Hydroxyniederalkyl mit 2 bis 4 Kohlenstoffatomen, wie 2-Hydroxyäthyl, Niederalkanoyloxymethyl mit bis und mit 7 Kohlenstoffatomen im Alkanoylteil, wie Acetoxy- oder Pivaloyloxymethyl, oder ω-(ω)Hydroxyniederalkoxy)-niederalkyl, ω-(ω-Niederalkoxyniederalkoxy)-niederalkyl, ω-[ω-(ω-Hydroxyniederalkoxy)-niederalkoxy]-niederalkyl oder ω[ω-(ω-Niederalkoxyniederalkoxy)-niederalkoxy]-niederalkyl mit jeweils 1 bis 4 Kohlenstoffatomen im Alkyl- und 2 bis 4 Kohlenstoffatomen im Alkylenteil, wie 2-(2-Hydroxyäthoxy)-äthyl, 2-(2-Methoxyäthoxy)-äthyl, 2-[2-(2-Hydroxyäthoxy)-äthoxy]-äthyl oder 2-[2-(Methoxyäthox)-äthoxy]-äthyl, bedeutet, $R_7$ Wasserstoff darstellt, $R_8$ Hydroxy oder Niederalkoxy mit bis und mit 4 Kohlenstoffatomen, z.B. Methoxy oder Aethoxy, bedeutet, $R_{9a}$ Hydroxy darstellt, und $R_{10}$ Niederalkenyl mit bis und mit 4 Kohlenstoffatomen, z.B. Allyl, ist, oder X eine direkte Bindung darstellt, $R_6$ Wasserstoff oder Niederalkyl mit bis und mit 7 Kohlenstoffatomen, wie Propyl, $R_7$ Niederalkyl mit bis und mit 4 Kohlenstoffatomen, wie Methyl, darstellt, wobei die Gruppe $R_6$—C(=O)— die 6- und die Niederalkylgruppe, $R_7$ die 7-Stellung einnimmt, $R_8$ Hydroxy oder Niederalkoxy mit bis und 4 Kohlenstoffatomen, wie Methoxy oder Aethoxy, darstellt, $R_{9a}$ Hydroxy bedeutet und $R_{10}$ Niederalkyl mit bis und mit 4 Kohlenstoffatomen, wie Propyl, bedeutet, bzw. das 2-Oxo-4-hydroxy-1,2-dihydrochinolin- oder 2,4-Dioxo-1,2,3,4-tetrahydrochinolin-Tautomere davon, in freier Form oder in Salzform.

Die Erfindung betrifft in ganz besonderem mMasse Verbindungen der Formel II, worin X eine direkte Bindung darstellt und $R_6$ Niederalkyl mit bis zu 7 Kohlenstoffatomen, z.B. Propyl bedeutet, oder X Oxy darstellt und $R_6$ Wasserstoff oder Niederalkyl mit bis und mit 4 Kohlenstoffatomen, wie Methyl, bedeutet, und worin $R_7$ Wasserstoff oder vor allem Niederalkyl mit bis und mit 4 Kohlenstoffatomen, wie Methyl, bedeutet, wobei die Gruppe $R_6$—C(=O)— bzw. $R_6$—O—C(=O)— die 6- und eine Niederalkylgruppe $R_7$ die 7-Stellung einnimmt, $R_8$ Hydroxy oder Niederalkoxy mit bis zu 4 Kohlenstoffatomen, wie Aethoxy, bedeutet, $R_{9a}$ für Hydroxy steht und $R_{10}$ Niederalkyl oder Niederalkenyl mit bis zu 4 Kohlenstoffatomen, wie Methyl, Aethyl, Propyl oder Allyl, darstellt, und deren 2-Oxo-4-hydroxy-1,2-dihydrochinolin- bzw. 2,4-Dioxo-1,2,3,4-tetrahydrochinolin-tautomeren und gegebenenfalls der Salze, vor allem pharmazeutisch verwendbaren Salze, von salzbildenden Verbindungen der genannten Art.

Die Erfindung betrifft namentlich die Herstellung der in den Beispielen genannten Verbindungen der Formel (I).

Die Verbindungen der Formel (I) können nach an sich bekannten Methoden hergestellt werden, beispielsweise indem man eine Verbindung der Formel (IV)

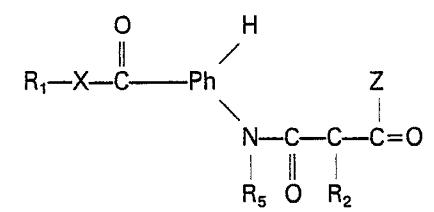

(IV)

worin Z einen abspaltbaren Rest bedeutet, oder ein Tautomeres und/oder Salz davon intramolekular cyclisiert und gewünschtenfalls die erhaltene Verbindung in eine andere Verbindung der Formel (I) überführt und/oder eine erhaltene freie Verbindung in ein Salz oder ein erhaltenes Salz in die freie Verbindung oder in ein anderes Salz umwandelt.

Der abspaltbare Rest Z ist beispeilsweise eine gegebenenfalls verätherte oder veresterte Hydroxygruppe. Verätherte Hydroxygruppen sind beispielsweise mit aliphatischen, araliphatischen oder aromatischen Alkoholen verätherte Hydroxygruppen, wie Niederalkoxy, z.B. Methoxy. oder Aethoxy, oder gegebenenfalls substituiertes, wie Niederalkyl, Niederalkoxy oder insbesondere Halogen und/oder Nitro aufweisendes Phenoxy, z.B. Phenoxy, 4-Chlorphenoxy, 4-Nitrophenoxy, 2,4-Dinitrophenoxy und 3,5-Dichlorphenoxy. Veresterte Hydroxygruppen sind beispielsweise mit organischen Carbonsäuren, wie

Niederalkansäuren, oder mit monofunktionellen Kohlensäurederivaten, wie Kohlensäuremonoestern oder -monohalogeniden, insbesondere aber mit Mineralsäuren, wie Halogenwasserstoffsäuren, veresterte Hydroxygruppen, beispielsweise Formyloxy, Acetoxy, Chlorcarbonyloxy, Niederalkoxy-, wie Aethoxycarbonyloxy, oder insbesondere Halogenatome, wie Chor, Brom oder Jod, ferner Sulfonyloxygruppen, wie von organischen Sulfonsäuren oder Halogensulfonsäuren abgeleitete Sulfonyloxygruppen, z.B. Fluorsulfonyloxy, Chlorsulfonyloxy, Methansulfonyloxy, Benzolsulfonyloxy, p-Toluolsulfonyloxy oder p-Bromsulfonyloxy. Tautomere von Verbindungen der Formel (IV) sind insbesondere solche, in denen die Hydroxygruppe $R_3$ in der tautomeren Oxoform vorliegt. Salze von Verbindungen der Formel (IV) sind insbesondere Salze mit Basen, wie Alkalimetallsalze, von Verbindungen der Formel (IV), in denen Z Hydroxy und/oder $R_2$ Carboxy ist, oder Säureadditionssalze von basischen Verbindungen der Formel (IV).

Die intramolekulare Kondensation kann in üblicher Weise durchgeführt werden, vorzugsweise durch Erhitzen, beispielsweise auf etwa 150°C bis etwa 250°C, in Gegenwart eines geeigneten Kondensationsmittels und erforderlichenfalls eines unter den Reaktionsbedingungen inerten Lösungsmittels, und wenn notwendig, unter Inertgas, wie Stickstoff, und/oder im geschlossenen Gefäss. Unter den Reaktionsbedingungen inerter Lösungsmittel sing beispielsweise höher siedende Kohlenstoffstoffe, wie Toluol oder Xylol, Aether, wie diphenyläther oder tertiärer Carbonsäureamide, wie Dimethylformamid oder N-Methylpyrrolidon. Geeignete Kondensationsmittel sing beispielsweise saure Mittel, wie Protonensäuren, z.B. Mineralsäuren, u.a. Schwefelsäure, Phosphorsäure oder Polyphosphorsäure, oder saure Mineralsäureester wie Mono- oder Diniederalkylphosphate oder -phosphite u.a. Triäthylphosphat, Triäthylphosphit oder Tetraäthylpyrophosphat, ferner Lewissäurene, wie beispielsweise Aluminiumchlorid, Aluminiumbromid, Zinkchlorid, Bortrifluorid oder Antimonpentachlorid.

Die Ausgangsstoffe der Formel (IV) können, soweit sie neu sind, nach an sich bekannten Methoden erhalten werden, beispielsweise ausgehend von entsprechenden Anilinverbindungen der Formel $R_1—X—C(=O)—Ph(NHR_E)—H$ (V). So kann man Ausgangsstoffe der Formel (IV), in denen Z eine verätherte Hydroxygruppe bedeutet, z.B. erhalten, indem man die obengenannte Anilinverbindung in üblicher Weise mit einem Niederalkoxy-$R_3$-Methylenmalonsäurediniederalkylester, d.h. mit einem Methantricarbonsäuretriniederalkylester, kondensiert. Aus den so erhältlichen Estern der Formel (IV) können in üblicher Weise, z.B. hydrolytisch, die entsprechenden Säuren und aus diesen in üblicher Weise andere funktionelle Derivate derselben hergestellt werden.

Die Verbindungen der Formel (I) bzw. Tautomere derselben, können ferner hergestellt werden, indem man eine Verbindung der Formel (VI)

```
         O        C(=O)—Z1
         ‖       /
R1—X—C———Ph                    (VI)
                 \
                  N(RE)—C(=O)—Z2
```

worin einer der Reste $Z_1$ und $Z_2$ eine Gruppe der Formel $CH_2—R_2$ und der andere eine gegebenenfalls reaktionsfähig verätherte oder veresterte Hydroxygruppe bedeutet, oder ein Tautomeres und/oder Salz davon intramolekular cyclisiert und gewünschtenfalls die erhaltene Verbindung in eine andere Verbindung der Formel (I) überführt und/oder eine erhaltene freie Verbindung in ein Salz oder ein erhaltenes Salz in die freie Säure oder in ein anderes Salz umwandelt.

Verätherte Hydroxygruppen sind beispielsweise mit einem aliphatischen Alkohol, wie mit einem Niederalkanol, z.B. mit Methanol oder Aethanol, oder mit einem gegebenenfalls substituierten aromatischen Alkohol, z.B. Phenol, verätherte Hydroxygruppen. Veresterte Hydroxygruppen sind beispielsweise mit einer Mineralsäure, wie einer Halogenwasserstoffsäure, z.B. mit Chlor-, Brom- oder Jodwasserstoffsäure, oder in zweiter Linie mit einer Halogensulfonsäure oder organischen Sulfonsäure, z.B. Methan-, Aethan-, Benzol-, p-Toluol-, oder Fluorsulfonsäure veresterte Hydroxygruppen.

Die intromolukulare Cyclisierung erfolgt in üblicher Weise, vorzugsweise in einem weitgehend wasserfreien Lösungsmittel, vorteilhaft in Gegenwart eines wasserentziehenden Mittels und erforderlichenfalls in Anwesenheit eines basischen Kondensationsmittels, wenn nötig, bei erhöhter Temperatur, z.B. bei etwa 50 bis 150°C, unter Inertgas wie Stickstoff, und/oder im geschlossenen Gefäss. Geeignete Lösungsmittel sind vor allem Niederalkanole, wie Methanol, Aethanol oder Butanol, Niederalkylenglykole, wie Aethylenglykol, ferner Dimethylsulfoxid, Dimethylformamid, Diphenyläther und hochsiedenden Kohlenwasserstoffe, wie Xylol. Basische Kondensationsmittel sind beispielsweise Alkalialkoholate, wie Alkaliniederalkanolate, z.B. Natriummethanolat, Natriumäthanolat oder Natrium- tert.-butanolat, oder Alkalimetallhydride, wie Natriumhydrid.

Die ausgangsstoffe der Formel (VI) können, soweit sie neu sind, nach an sich bekannten Methoden hergestellt werden, beispielsweise indem man eine Verbindung der Formel

$$R_1—X—C(=O)—Ph(NHR_E)—C(=O)—Z_1 \qquad \text{(VII)}$$

oder ein Derivat davon mit einer Verbindung der Formel $Z_2—COOH$ (VIII) oder einem reaktionsfähigen funktionellen Derivat, wie einem Ester, z.B. einem Niederalkylester, oder Anhydrid, z.B. dem Chlorid oder

dem symmetrischen oder inneren Anhydrid, davon umsetzt, und gewünschtenfalls in der erhaltenen Verbindung der Formel (VI) Gruppen $Z_1$ und/oder $Z_2$ in üblicher Weise in andere solche Gruppen überführt. So kann man beispielsweise einen entsprechenden Anthranilsäureester mit einem Malonsäureesterchlorid oder einem Malonsäurediester oder einen entsprechenden 2-Aminobenzoylessigsäureester mit Phosgen oder einem Halogenameisensäureniederalkyl- oder -phenylester umsetzen. Die Verbindungen der Formel (I) können ferner hergestellt werden, indem man eine Verbindung der Formel (IX)

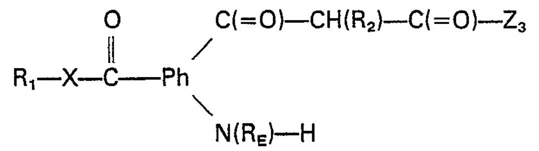

(IX)

worin $Z_3$ Hydroxy oder eine reaktionsfähige abgewandelte Hydroxygruppe bedeutet, oder ein Tautomeres und/oder Salz davon intramolekular kondensiert und gewünschtenfalls eine so erhältliche Verbindung in eine andere Verbindung der Formel (I) überführt und/oder eine erhaltene freie Verbindung in ein Salz oder ein erhaltenes Salz in die freie Verbindung oder in ein anderes Salz überführt.

Reaktionsfähige abgewandelte Hydroxygruppen sind beispielsweise verätherte oder vor allem veresterte Hydroxygruppen. Verätherte Hydroxygruppen sind z.B. Niederalkoxy oder gegebenenfalls substituiertes Phenoxy, und veresterte Hydroxygruppen insbesondere mit Mineralsäuren, wie Halogenwasserstoffsäuren, oder mit Halogensulfonsäuren bzw. organischen Sulfonsäuren veresterte Hydroxygruppen, wie Halogen, z.B. Chlor-, Brom oder Jod, oder Methan-, Benzol-, p-Toluol-, p-Brombenzol-, oder Fluorsulfonyloxy. Die intramolekulare Cyclisierung von Verbindungen der Formel (IX) kann üblicher Weise erfolgen, beispielsweise in einem unter den Reaktionsbedingungen inerten Lösungsmittel wie einem Kohlenwasserstoff, z.B. von Benzol, Toluol oder Mineralöl, einem Aether, z.B. Diäthyläther oder Tetrahydrofuran, Dimethylformamid, Dimethylsulfoxid, und dergleichen erforderlichenfalls unter Erwärmen z.B. auf etwa 50 bis 150°C, vorteilhaft in Gegenwart eines basischen Kondensationsmittels, wie eine Alkalimetallhydroxyds, z.B. von Natrium- oder Kaliumhydroxid, oder einer organischen Stickstoffbase, z.B. von Pyridin oder Triäthylamin, wenn nötig, unter Inertgas, wie Stickstoff, und/oder im geschlossenen Gefäss.

Die Ausgangsstoffe der Formel (IX) können, soweit sie neu sind, nach an sich bekannten Methoden hergestellt werden, beispielsweise indem man in üblicher Weise eine Verbindung der Formel $R_1—X—C(=O)—Ph—(NHR_E)—COOH$ (X) oder ein funktionelles Carboxyderivat, wie einen Ester oder ein Anhydrid, z.B. das symmetrische Anhydrid, ein entsprechendes Säurechlorid oder das entsprechende Isatosäureanhydrid, mit Malonsäure oder einem geeigneten funktionellen Derivat, wie einem Diester derselben, umsetzt und gewünschtenfalls in der erhaltenen Verbindung die Gruppe $Z_3$ in eine andere solche Gruppe überführt.

Die Verbindungen der Formel (I) können weiterhin hergestellt werden, indem man in einer Verbindung der Formel (XI)

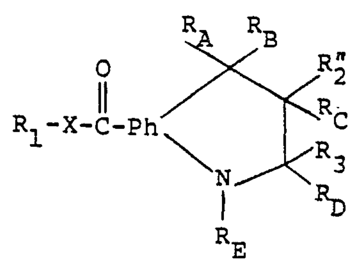

(XI),

worin $R_2''$ einen in die gegebenenfalls veresterte oder amidierte Carboxygruppe $R_2$ überführbaren Rest bedeutet, oder in einem Tautomeren und/oder Salz davon die Gruppe $R_2''$ in die gegebenenfalls veresterte oder amidierte Carboxygruppe $R_2$ überführt und gewünschtenfalls eine so erhältliche Verbindung in eine andere Verbindung der Formel (I) oder eine erhaltene salzbildende Verbindung in ein Salz oder ein erhaltenes Salz in die freie Verbindung oder ein anderes Salz überführt.

In gegebenenfalls verestertes oder amidiertes Carboxy überführbare Gruppen $R_2''$ sind beispielsweise von verestertem oder amidiertem Carboxy $R_2$ verschiedene, solvolytisch in gegebenenfalls verestertes oder amidiertes Carboxy $R_2$ überführbaren funktionell abgewandelte Carboxygruppen. Derartige Gruppen sind beispielsweise Cyano, anhydridisierte Carboxygruppen, Iminoäthergruppen, Iminoestergruppen sowie verätherte und/oder veresterte Trihydroxymethylgruppen. Anhydridisierte Carboxygruppen sind beispielsweise mit einer Mineralsäure, wie einer Halogenwasserstoffsäure, mit einer Halogensulfonsäure, wie Fluorsulfonsäure, oder einer organischen Sulfon- oder Carbonsäure, wie einer aliphatischen oder aromatischen Sulfon- oder Carbonsäure, anhydridisierte Carboxygruppe. Iminoäthergruppen sind beispeilsweise von veresterten Carboxygruppen $R_2$ abgeleitete Iminoäthergruppen, wie O-Niederalkylcarbamoyl, oder cyclische Iminoäthergruppen, wie 4,4- oder 5,5-Diniederalkyl-, z.B. 4,4- oder 5,5-Dimethyl-4,5-dihydro-oxazolyl-(2), oder 4,4,6-Triniederalkyl-, z.B. 4,4,6-Trimethyl-5,6-dihydro-oxazinyl-(2). Iminoestergruppen sind beispielsweise von amidierten Carboxygruppen $R_2$ abgeleitete, mit Halogenwasserstoffsäuren oder organischen Carbonsäuren, wie Niederalkansäuren, veresterte Iminoestergruppen, wie z.B. Chlorcarbimido oder O-Niederalkanoylcarbamoyl. Verätherte und/oder veresterte Trihydroxymethylgruppen sind beispielsweise veresterten Carboxygruppen $R_2$ entsprechende verätherte und/oder mit Mineralsäuren, wie Halogenwasserstoffsäuren, veresterte Trihydroxymethylgruppen, z.B. Triniederalkoxymethyl, Niederalkoxy-dihalogenmethyl oder Trihalogenmethyl, insbesondere solche, in denen Halogen Chlor oder Brom darstellt. Weitere solvolytisch in amidierte Carboxygruppen $R_2$ überführbare Gruppen sind beispielsweise diesen entsprechende dihalogenierte Carbamoylgruppen, d.h. entsprechende Amino-dihalogen-, insbesondere -dichlormethylgruppen.

Die genannten Gruppen können durch übliche Hydrolyse in Carboxygruppen, Iminoäthergruppen sowie verätherte Hydroxy-dihalogenmethyl-, wie Niederalkoxy-dihalogenmethylgruppen, ferner in veresterte Carboxygruppen $R_2$, Amino-dihalogenmethylgruppen in amidierte Carboxygruppen $R_2$ und Cyano in unsubstituierte Carbamoylgruppen überführt werden. Die Hydrolyse erfolgt in überlicher Weise, beispielsweise in Gegenwart eines sauren oder alkalischen Hydrolysemittels, überlicherweise in Gegenwart eines Lösungs- und/oder Verdünnungsmittels oder eines Gemisches davon, und, wenn notwendig, unter Kühlen oder Erwärmen z.B. in einem Temperaturberiech von etwa 0°C bis etwa 120°C, erforderlichenfalls unter Inertgas, wie Stickstoff, und/oder in geschlossenem Gefäss. Saure Hydrolysemittel sind beispielsweise Mineralsäuren, wie Halogenwasserstoffsäuren, z.B. Salzsäure, oder Sauerstoffsäuren des Schwefels oder Phosphors, wie Schwefel- oder Phosphorsäure, ferner organische Sulfonsäuren, z.B. p-Toluolsulfonsäure oder Mesitylensulfonsäure, oder organische Carbonsäuren, wie Niederalkancarbonsäuren, z.B. Ameisen- oder Essigsäure. Basische Hydrolysemittel sind beispielsweise Alkalimetallhydroxyde, z.B. Natrium- oder Kaliumhydroxyd, ferner Alkalimetall carbonate, z.B. Natrium- oder Kaliumcarbonat. Geeignete Lösungs- oder Verdünnungsmittel sind vorzugsweise mit Wasser mischbare Lösungsmittel, wie Niederalkanole, z.B. Aethanol oder Methanol, niedere Ketone, z.B. Aceton, oder tertiärer Alkansäureamide, z.B. Dimethylformamid oder N-Methylpyrrolidon.

Anhydridisierte Carboxygruppen sowie cyclishes Iminoäthergruppen können ferner durch Alkoholyse, d.h. Umsetzung mit einem entsprechenden Alkohol, in veresterte Carboxygruppen $R_2$ überführt werden. Dabei arbeitet man in üblicher Weise, ausgehend von anhydridisierten Carboxygruppen beispielsweise in Gegenwart eines basischen Kondensationsmittels, wie eines Alkalimetallhydroxydes oder -carbonates, z.B. von Natrium- oder Kaliumhydroxyd bzw. entsprechender Carbonate, oder von organischen Stickstoffbasen, wie Pyridin oder Triäthylamin, und ausgehend von cyclischen Iminoäthergruppen vorzugsweise unter wasserfreien Bedingungen, z.B. in Gegenwart von Chlorwasserstoff, Phosphorsäure, Schwefelsäure oder p-Toluolsulfonsäure, erforderlichenfalls unter Erwärmen, beispielsweise im Temperaturbereich von etwa 0°C bis etwa 150°C, unter Inertgas, wie Stickstoff, und/oder in einem geschlossenen Gefäss.

Anhydridisierte Carboxygruppen können ferner durch Ammono- bzw. Aminolyse, d.h. Umsetzung mit Ammoniak oder einen entsprechenden, mindestens ein Wasserstoffatom aufweisenden Amin, in üblicher Weise in amidierte Carboxygruppen $R_2$ überführt werden.

Weitere in gegebenenfalls veresterte Carboxygruppen $R_2$ überführbare Reste $R_2''$ sind oxydativ in diese überführbare Gruppen, wie die oxydativ in Carboxy überführbare, gegebenenfalls hydratisierte Formylgruppe oder oxydativ in veresterte Carboxygruppen $R_2$ überführbare verätherte Hydroxymethylgruppen. Weiere oxydativ in Carboxy überführbare Gruppen sind gegebenenfalls subsituierte, z.B. in 5-Stellung Diniederalkoxymethyl, wie Diäthoxymethyl, enthaltendes 2-Furyl, oder gegebenenfalls 2-substituiertes Vinyl, z.B. 2,2-Diphenylvinyl.

Die Oxydation kann in an sich bekannter Weise durchgeführt werden, beispielsweise durch Umsetzung mit einer oxydierenden Schwermetallverbindung, vorzugsweise einer Chrom-VI- oder Mangan-VIIenthaltenden Verbindung, z.B. mit Chromtrioxyd oder insbesondere Kaliumpermanganat, ferner mit Wismut-III-, Mangan-IV- oder Silber-I- enthaltenden Verbindungen, z.B. mit Wismutoxid, Mangandioxyd oder Silberoxid, oder durch Luftoxydation. Dabei arbeitet man vorteilhafterweise in Gegenwart eines gegenüber den Reaktionsteilnehmern inerten Lösungsmittels, z.B. von Aceton oder Pyridin, oder eines, vorzugsweise wässrigen Gemisches davon, wenn notwendig, unter Kühlen oder Erwärmen, z.B. im Temperaturebereich von etwa 0°C bis 80°C. Gegebenenfalls veräthertes Hydroxymethyl kann vorteilhaft mittels Kaliumpermanganat in wässrigem Aceton oder Pyridin zu gegenbenenfalls verestertem Carboxy oxydiert werden.

Die Ausgangsstoffe der Formel (XI) können, soweit sie neu sind, nach an sich bekannten Methoden hergestellt werden.

Verbindungen, der Formel (XI), in denen $R_2''$ Cyano, veräthertes Hydroxymethyl, Formyl oder gegebenenfalls substituiertes 2-Furyl bedeutet, kann man beispielsweise durch intramolekulare Kondensation entsprechender Verbindungen der Formel $R_1$—X—C(=O)—Ph(H)—N($R_E$)—(=O)—CH($R_2''$)—C(=O)—Z (XII), in denen Z eine reaktionsfähig veresterte oder verätherte Hydroxygruppe, z.B. Halogen oder Niederalkoxy, wie Methoxy, ist, herstellen. Dabei kann eine Formylgruppe $R_2''$ auch in intermediär geschützter, wie acetalysierter oder acylalisierter Form, z.B. als Diniederalkoxy-, Niederalkylendioxy- oder Dihalogenmethyl, vorliegen. Die, beispielsweise so erhältlichen, Nitrile der Formel (XI) können durch übliche, z.B. säurekatalysierte, Umsetzung mit den entsprechenden Alkoholen bzw. Aminoalkoholen, in Iminoäther der Formel (XI), beispielsweise mit einem Niederalkanol in offenkettige Iminoäther bzw. mit dem betreffenden

Aminoalkanol oder Alkandiol, z.B. mit 4-Amino-2-methyl-pentan-2-ol oder 2-Methyl-pentan-2,4-diol, in cyclische Iminoäther, überführt werden. Eine Trihalogenmethylgruppe $R_2''$ aufweisende Verbindungen der Formel (XI) kann man beispielsweise durch übliche Halogenierung einer entsprechenden Methylverbindung, z.B. mit N-Chlor- oder N-Bromsuccinimid, oder durch Haloform-analogen Abbau entsprechender Alkanoyl-, wie Acetylverbindungen, erhalten. Eine gegebenenfalls hydratisierte Formylgruppe $R_2''$ aufweisende Verbindungen der Formel (XI) können ferner im Verlaufe der Oxydationsreaktion *in situ*, z.B. aus der Methyl- oder Aminomethylgruppe oder der gegebenenfalls mit einer anorganischen Säure, wie einer Halogenwasserstoffsäure, oder mit einer organischen Carbonsäure, wie einer Niederalkancarbonsäure, veresterten Hydroxymethylgruppe gebildet oder aus einem ihrer Derivate, wie einem Acetal, Acylal oder Imin, z.B. einem Niederalkylen- oder Diniederalkylacetal einer Dihalogen-, wie Dichlormethylverbindung oder einem gegebenenfalls substituierten Benzylimin, Freiheit gesetzt werden. Amino- bzw. Niederalkoxydihalogenmethylgruppen werden ebenfalls vorteilhaft *in situ* durch übliche partielle Ammono- bzq. Aminolyse oder Alkoholyse der entsprechenden Trihalogenmethylverbindung hergestellt.

Die Verbindungen der Formel (I) können weiterhin hergestellt werden, indem man in einer Verbindung der Formel (XIII)

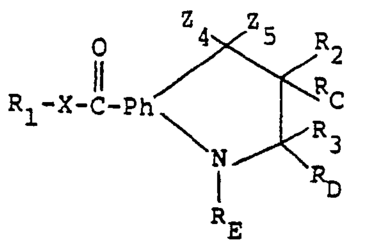

(XIII),

worin $Z_4$ ein in Hydroxy oder Niederalkoxy überführbaren Rest bedeutet, und $Z_5$ gemeinsam mit $R_C$ sowie $R_D$ gemeinsam mit $R_E$ jeweils eine zusätzliche Bindung darstellt, oder worin $Z_4$ und $Z_5$ jeweils einen einwertigen oder gemeinsam einen zweiwertigen in Oxo überführbaren Rest bedeuten und $R_C$ gemeinsam mit $R_D$ eine zusätzliche Bindung darstellt, oder in einem Salz davon einen in Hydroxy oder Niederalkoxy überführbaren Rest $Z_4$ in Hydroxy oder Niederalkoxy bzw. in Oxo überführbare Reste $Z_4$ und $Z_5$ gemeinsam in Oxo überführt und gewünschtenfalls die so erhältliche Verbindung in eine andere Verbindung der Formel (I) und/oder eine erhaltene salzbildende Verbindung in ein Salz oder ein erhaltenes Salz in die freie Verbindung oder in ein anderes Salz umwandelt.

In Hydroxy bzw. Niederalkoxy überführbare Reste sind beispielsweise veresterte Hydroxygruppen, gegebenenfalls verätherte Mercaptogruppen, Sulfinylgruppen, Sulfonylgruppen oder gegebenenfalls substituierte Aminogruppen. Einwertige, gemeinsam in die Oxogruppe überführbare Reste sind beispielsweise veresterte oder mit einem einwertigen offenkettigen Alkohol verätherte Hydroxygruppen oder mit einem offenkettigen Mercaptan verätherte Mercaptogruppen. Durch $Z_4$ und $Z_5$ gemeinsam dargestellte zweiwertige, in Oxo überführbare Reste sind beispielsweise die Thiogruppe oder mit einem 2-wertigen Alkohol, Mercaptoalkohol oder Mercaptan verätherte Hydroxygruppen oder gegebenfalls substituierte Iminogruppen.

Veresterte Hydroxygruppen sind beispielsweise mit einer Mineralsäure, einer organischen Sulfonsäure oder einer Carbonsäure veresetelte Hydroxygruppen, wie Halogen, z.B. Chlor, Brom oder Jod, aliphatisches oder aromatisches Sulfonyloxy, z.B. Methan-, Aethan-, Benzol- oder p-Toluolsulfonyloxy, oder von einer organischen Carbonsäure oder einem monofunktionellen Kohlensäurederivat, wie einem Kohlensäurehalbester, einer Halogenameisensäure oder der gegebenenfalls substituierte Carbaminsäure, abgeleitetes Acyloxy, z.B. Niederalkanoyloxy, gegebenenfalls substituiertes Benzyloxy, Niederalkoxycarbonyloxy, gegebenenfalls substituiertes Phenoxycarbonyloxy, Chlor- oder Bromcarbonyloxy oder gegebenenfalls substituiertes, wie niederalkyliertes, Carbamoyloxy. Veräthertes Mercapto ist beispielsweise eine Aliphatylthio oder Arylthiogruppe z.B. Niederalkylthio oder gegebenenfalls substiuiertes Phenylthio. Substituiertes Amino ist beispielsweise durch Hydroxy oder Amino monosubstituiertes, durch aliphatische Reste oder gegebenenfalls substituiertes Phenyl mono- oder disubstituiertes Amino, wobei aliphatische Reste ein- oder zweiwertig sein können, einwertige aliphatische Reste beispielsweise Niederalkyl und zweiwertige aliphatische Reste Niederalkylen bzw. Aza-, Oxa- oder Thianiederalkylen darstellen, z.B. Hydroxyamino, Hydrazino, Mono- oder Di-niederalkylamino, gegebenenfalls substituiertes Anilino, Pyrrolidino, Piperidino, Morpholino, Thiamorpholino oder N'-Niederalkyl-, wie N'-Methyl-piperazino. Mit einem einwertigen Alkohol veräthertes Hydroxy ist beispielsweise Niederalkoxy oder gegebenenfalls substituiertes Phenyloxy. Mit einem zweiwertigen Alkohol verätherte Hydroxygruppen $Z_4 + Z_5$ sind beispielsweise Niederalkylendioxy- oder gegebenenfalls substituierte 1,2-Phenylendioxy-gruppen, z.B. Aethylendioxy, 1,3-Propylendioxy oder 1,2-Phenylendioxy. Mit einem zweiwertigen Hercaptoalkohol oder Mercaptan verätherte Hydroxygruppen sind beispielsweise Niederalkylendithio- bzw. Niederalkylenoxythiogruppen oder gegebenenfalls substituierte 1,2-Phenyldithio- bzw. Phenoxythiogruppen, z.B. Aethylendithio, 1,3-Propylendithio oder 1,2-Phenylendithio. Gegebenenfalls substituierte Iminogruppen

sind beispielsweise durch Hydroxy, Amino, Niederalkyl oder gegebenenfalls substituiertes Phenyl substituierte Iminogruppen, z.B. Oximino, Hydrazono, Niederalkylamino oder Anilo.

Die Ueberführung der genannten Gruppen in Hydroxy, Niederalkoxy bzw. in Oxo erfolgt in üblicher Weise, vorzugsweise durch Solvolyse, insbesondere Hydrolyse oder Alkoholyse, d.h. Umsetzung mit Wasser oder dem der zu bildenden Niederalkoxygruppe entsprechenden Niederalkanol. So Kann man beispielsweise die genannten in Oxo bzw. Hydroxy überführbaren Gruppen in üblicher Weise, beispielsweise in Gegenwart eines sauren oder basischen Hydrolysemittels, vorteilhaft in einem Lösungs- oder Verdünnungsmittel und erforderlichenfalls bei erhöhter oder erniedrigter Temperatur, z.B. dem Temperaturbereich von 0—150°C, unter Inertgas wie Stickstoff, und/oder in einem geschlossenen Gefäss, in Oxo bzw. Hydroxy überführen. Saure Hydrolysemittel sind beispielsweise Protonensäuren, wie Mineralsäuren oder deren saure Salze, z.B. Chlor-, Brom- oder lodwasserstoffsäure, Schwefelsäure bzw. Alkalimetallhydrogensulfate, Sulfonsäuren z.B. p-Toluolsulfonsäure oder Sulfaminsäure oder organische Carbonsäuren, wie Niederalkansäuren, ferner saure Ionenaustauscher. Basische Kondensationsmittel sind beispielsweise Alkalimetallhydroxyde oder -carbonate z.B. Natrium- oder Kaliumhydroxyd oder -carbonat, ferner tertiäre organische Stickstoffbasen, z.B. Triäthylamino oder Pyridin. Geeignete Lösungs- oder Verdünnungsmittel sind insbesondere mit Wasser mischbare Lösungsmittel, wie Alkohole, z.B. Niederalkanole, cyclische aliphatische Aether, z.B. Dioxan oder Tetrahydrofuran, niederaliphatische Ketone, z.B. Aceton, tertiäre aliphatische Amide oder Lactame, z.B. Dimethylformamid oder N-methylpyrrolidon, oder aliphatische Sulfoxyde, z.B. Dimethylsulfoxyd. Wie bereits erwähnt, können reaktionsfähig veresterte, d.h. mit einer Mineralsäure oder einer organischen Sulfonsäure veresterte, Hydroxygruppen $Z_4$ nach üblichen Alkoholysemethoden in Niederalkoxy überführt werden, beispielsweise in Gegenwart eines basischen Kondensationsmittels, wie eines Alkalimetallhydroxydes oder -carbonates, oder indem man den betreffenden Alkohol in Form eines Alkoholates, wie des betreffenden Alkalimetallalkoholates einsetzt, vorzugsweise in einem Lösungs- oder Verdünnungsmittels, erforderlicherweise unter Erwärmen z.B. im Temperaturbereich von etwa 0—150°C, und ein Inertgas, wie Stickstoff, und/oder im geschlossenen Gefäss.

Die Ausgangsstoffe der Formel XIII können, soweit sie neu sind, nach an sich bekannten Methoden hegestellt werden.

So kann man Verbindungen der Formel XIII, in denen $Z_4$ Halogen und X eine direkte Bindung bedeutet, beispielsweise erhalten, indem eine Verbindung der Formel XIV

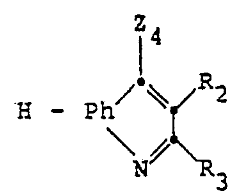

(XIV),

in Gegenwart einer Lewis-Säure, z.B. von Aluminiumtrichlorid, mit einem Halogenid der Säure der Formel $R_1$—COOH (XV), kondensiert. Aus den so erhältlichen Halogen-Verbindungen können durch Umsetzung mit einem Isothiuroniumsalz und anschliessende Hydrolyse bzw. Umsetzung mit Natriumthiolacetat und anschliessender Reduktion Verbindungen der Formel XIII, in denen $Z_4$ Mercapto ist, bzw. durch Umsetzung mit einem Alkalimetallmercaptid Verbindungen der Formel XIII, in den $Z_4$ veräthertes Mercapto ist, erhalten werden. Verbindungen der Formel XIII, in denen $Z_4$ eine gegebenenfalls substituiertes Aminogruppe bedeutet, können z.B. hergestellt werden, in dem man eine Verbindung der Formel

$$R_1—X—C(=O)—Ph(NHR_E)—C(Z_4)=CHR_2 \qquad (XVI)$$

mit Phosgen kondensiert. Verbindungen der Formel XIII, in denen X eine direckte Bindung darstellt und $Z_4$ und $Z_5$ veräthertes Hydroxy oder Mercapto bedeuten, kann man beispielsweise herstellen, indem man eine Verbindung der Formel XVIII

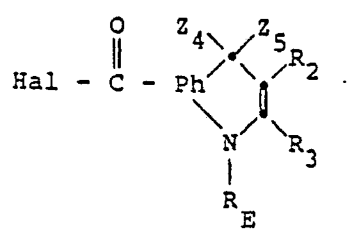

(XVIII),

worin Hal Halogen bedeutet, mit einer von dem Kohlenwasserstoff der Formel $R_1H$ abgeleiteten Metallverbindung, z.B. einer entsprechenden Halogenmagnesium- oder Lithiumverbindung umsetzt, Verbindungen der Formel XIII, in denen $Z_4 + Z_5$ Imino darstellt, können z.B. erhalten werden durch Kondensation einer Verbindung der Formel $R_1$—X—C(=)—Ph($NHR_E$)—H (V) mit Formylmalodinitril bzw. Formylmalonsäureesternitril, wobei man vorzugweise in der für die Herstellung und intramolekulare

Kondensation von Verbindungen der Formel IV bzw. VI angegebenen Weise arbeitet.

Die Verbindungen der Formel I kann man weiterhin erhalten, indem man in einer Verbindung der Formel XIX

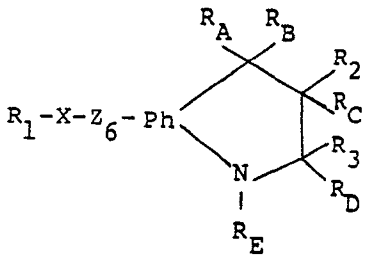

(XIX),

worin $Z_6$ einen in die Carbonylgruppen überführbaren Rest bedeutet, oder in einem Salz davon $R_6$ in die Carbonylgruppe überführt und gewünschtenfalls die so erhältliche Verbindung in eine andere Verbindung der Formel I und/oder eine erhaltende salzbildende Verbindung in ein Salz oder ein erhaltenes Salz in die freie Verbindung oder in ein anderes Salz umwandelt.

Der in die Carbonylgruppe überführbare Rest $Z_6$ ist beispielsweise ein solvolytisch, insbesondere hydrolytisch in diese überführbarer Rest, wie eine funktionell abgewandelte Carbonylgruppe, beispielsweise Thioxomethylen, gegebenenfalls substituiertes Iminomethylen oder veräthertes oder verestertes Dihydroxymethylen. Substituierte Iminomethylengruppen sind z.B. durch Hydroxy, Amino, Niederalkyl oder gegebenenfalls substituiertes Phenyl substituierte Methyleniminogruppen. Verätherte Dihydroxymethylengruppen sind beispielsweise mit aliphatischen Alkoholen, wie Niederalkanolen oder Niederalkandiolen, verätherte Dihydroxymethylengruppen. Veresterte Dihydroxymethylengruppen sind beispielsweise mit einer Halogenwasserstoffsäure veresterte Dihydroxymethylengruppen, wie Dichlor- oder Dibrommethylen.

Die Ueberführung der genannten Gruppen $Z_6$ in Carbonyl erfolgt nach üblichen Solvolyse- insbesondere Hydrolysemethoden, beispielsweise in Gegenwart eines sauren oder basischen Hydrolysemittels vorteilhaft in einem geeigneten Lösungs- oder Verdünnungsmittel, erforderlichenfalls unter Kühlen oder Erwärmen z.B. im Temperaturbereich von etwa 0 bis etwa 150°C, unter Inertgas, wie Stickstoff, und/oder in einem geschlossenen Gefäss. Saure Hydrolysemittel sind beispielsweise Protonensäuren, wie Mineralsäuren, z.B. Chlor-, Brom- oder Iodwasserstoffsäure, Schwefelsäure oder Phosphorsäure, oder deren saure Salze, z.B. Kaliumhydrogensulfat, Sulfonsäuren, wie aliphatische oder aromatische Sulfonsäuren, z.B. Methan-, Aethan- oder p-Toluolsulfonsäure, Sulfaminsäure, oder organische Carbonsäuren, wie Niederalkancarbonsäuren. Geeignete Lösungs- oder Verdünnungsmittel sind beispielsweise mit Wasser mischbare Lösungsmittel, wie aliphatische Alkohole, z.B. Niederalkanole oder Niederalkandiole, cyclische aliphatische Aether, z.B. Dioxan oder Tetrahydrofuran, Diniederalkylketone, z.B. Aceton, oder tertiäre Niederalkancarbonsäureamide oder -lactame, z.B. Dimethylformamide oder N-Methylpyrolidon.

Weiter in die Carbonylgruppe überführbare Reste sind zu dieser oxydierbare Gruppen, beispielsweise die Hydroxymethylengruppe, die auch unter den Reaktionsbedingungen, z.B. durch Oxydation der Methylengruppe, *in situ* gebildet oder aus einem ihrer funktionellen Derivate, beispielsweise einem Ester, wie einem Mineralsäure- oder Carbonsäureester, z.B. aus einer Halogenmethylengruppe, einer Niederalkanoyloxymethylengruppe oder einer gegebenenfalls substituierten Benzoyloxymethylengruppe in Freiheit gesetzt werden kann.

Die Oxydation kann in an sich bekannter Weise erfolgen, beispielsweise durch Behandeln mit oxidierenden Schwermetallverbindungen, wie Chrom-VI-, Mangan-VII- bzw. -IV-, Silber- oder Wismutenthaltenden Verbindungen z.B. mit Chromsäure, Kaliumdichromat, Kaliumpermanganat, Mangandioxyd, Silberoxyd bzw. Silberacetat oder Wismut- oxyd, mit oxydierenden anorganischen Sauerstoffsäuren oder ihren Salzen, z.B. mit Natriumhypochlorit, Natriumhypoiodit, Natriumchlorat, Natriumjodat oder Natriummetaperjodat, oder mit organischen Oxydationsmitteln, wie Chinonen, z.B. Benzochinon oder 2,6-Dicyanobenzochinon, Dimethylsulfoxyd/N-Chlorsuccinimid oder Dimethylsulfid/Trifluormethyl-trifluormethansulfonat, vorteilhaft in einem den Reaktionsteilnehmern gegenüber inerten Lösungs- oder Verdünnungsmittel, erforderlichenfalls unter Kühlen oder Erwärmen, z.B. im Temperaturbereich von etwa −20—100°C, unter Inertgas, wie Stickstoff, und/oder in einem geschlossenen Gefäss. Vorteilhaft ist insbesondere die Verwendung von Kaliumpermanganat oder von Mangandioxyd in wässrigem Aceton bzw. wässrigem Pyridin oder von Chromtrioxyd in Hexamethylphosphorsäuretriamid.

Die Erfindung betrifft ebenfalls Ausgangsstoffe der Formel

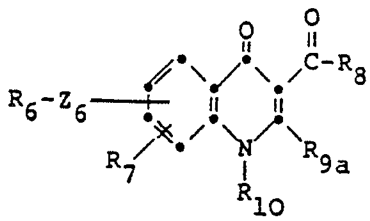

(XIXa),

worin $X_6$ Hydroxymethylen und $R_6$ Niederalkyl mit bis zu 7 Kohlenstoffatomen bedeutet, $R_7$ Wasserstoff oder Niederalkyl mit bis und mit 4 Kohlenstoffatomen darstellt, wobei die Gruppe $R_6$—$Z_6$ die 6- und eine Niederalkylgruppe $R_7$ die 7-Stellung einnimmt, $R_8$ Hydroxy oder Niederalkoxy mit bis und mit 4 Kohlenstoffatomen bedeutet, $R_{9a}$ Hydroxy bedeutet und $R_{10}$ Niederalkyl oder Niederalkenyl mit bis und mit 4 Kohlenstoffatomen bedeutet, bzw. jeweils das 2-Oxo-4-hydroxy-1,2-dihydrochinolin- oder 2,4-Dioxo-1,2,3,4-tetrahydrochinolin-Tautomere und/oder ein Salz davon, ein Verfahren zu ihrer Herstellung, diese enthaltende pharmazeutische Präparate und ihre Verwendung als Arzneimittel oder zur Herstellung von Arzneimitteln auf nicht-chemischen Wege. Die Erfindung betrifft diesbezüglich insbesondere 1-Aethyl-4-hydroxy-6-(1-hydroxybutyl)-7-methyl-carbostiryl-3-carbonsäureäthylester.

Das Verfahren zur Herstellung von Verbindungen der Formel XIXa und ihrer Salze ist dadurch gekennzeichnet, dass man eine Verbindung der Formel

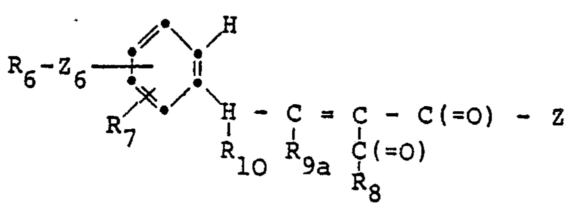

(IVa),

worin Z einen abspaltbaren Rest bedeutet, oder ein Tautomeres und/oder Salz davon intramolekular cyclisiert oder eine Verbindung der Formel

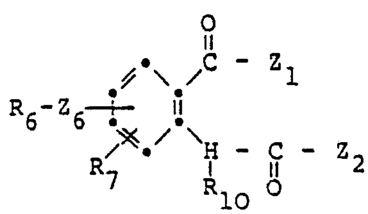

(VIa),

worin einer der Reste $Z_1$ und $Z_2$ eine Gruppe der Formel $CH_2$—C(=O)—$R_8$ und der andere eine gegebenenfalls reaktionsfähig verätherte oder veresterte Hydroxygruppe bedeutet, oder ein Tautomeres und/oder Salz davon intramolekular cyclisiert oder eine Verbindung der Formel

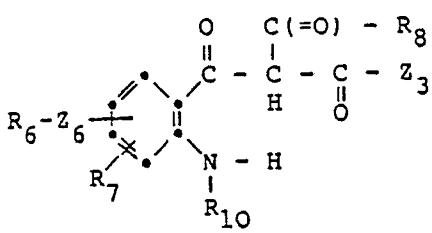

(IXa),

worin $Z_3$ Hydroxy oder eine reaktionsfähige abgewandelte Hydroxygruppe bedeutet, oder ein Tautomeres und/oder Salz davon intramolekular kondensiert oder in einer Verbindung der Formel

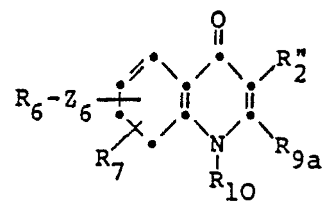

(XIa),

worin $R_2''$ einen in die gegebenenfalls veresterte oder amidierte Carboxygruppe $-C(=O)-R_8$ überführbaren Rest bedeutet, oder in einem Tautomeren und/oder Salz davon die Gruppe $R_2''$ in die gegebenenfalls veresterte oder amidierte Carboxygruppe $-C(=O)-R_8$ überführt oder in einer Verbindung der Formeln

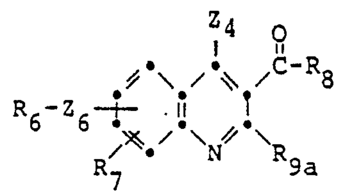

(XIIIa),

oder

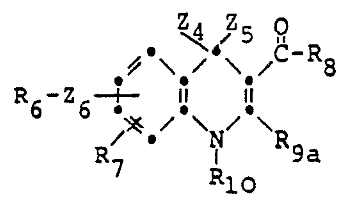

(XIIIb),

worin $Z_4$ in Formel XIIIa einen Hydroxy überführbaren Rest bedeutet oder worin in Formel XIIIb $Z_4$ und $Z_5$ jeweils einen einwertigen oder gemeinsam einen zweiwertigen in Oxo überführbaren Rest bedeuten, oder in einem Salz davon einen in Hydroxy überführbaren Rest $Z_4$ in Hydroxy bzw. in Oxo überführbare Reste $Z_4$ und $Z_5$ gemeinsam in Oxo überführt oder in einer Verbindung der Formeln

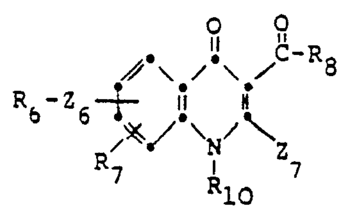

(XXIa),

oder

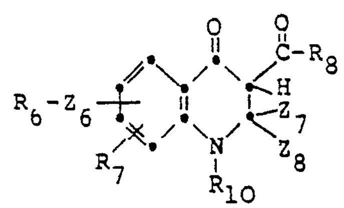

(XXIb),

worin in Formel XXIa $Z_7$ einen in Hydroxy überführbaren Rest oder in Formel XXIb $Z_7$ und $Z_8$ jeweils einen einwertigen oder gemeinsam einen zweiwertigen in Oxo überführbaren Rest bedeuten, $Z_7$ in Hydroxy bzw. $Z_7$ und $Z_8$ gemeinsam in Oxo überführt, oder eine Verbindung der Formel

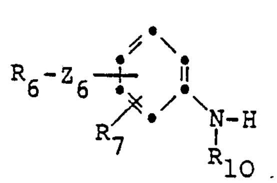

(Va)

mit einer Verbindung der Formel

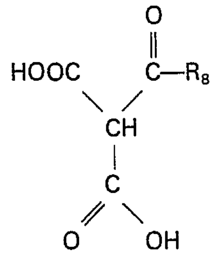

(XXa)

oder einen funktionellen Carboxyderivat bzw. Salz davon kondensiert oder in einer Verbindung der Formel

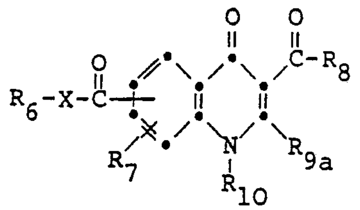

(II),

worin X eine direkte Bindung und $R_6$ Niederalkyl mit bis und mit 7 Kohlenstoffatomen ist, die Gruppe der Formel $R_6$—X—C(=O)— durch Behandeln mit einem geeigneten Reduktionsmittel, wie einem Dileichtmetallhydrid, z.B. Natriumboranat oder Natriumcyanboranat, oder mit einem sekundären Alkohol, wie einem sekundären Niederalkanol oder einem Cycloalkanol, z.B. mit Isopropanol oder Cyclohexanol, in Gegenwart eines Aluminiumalkoholates, z.B. von Aluminiumisopropanolat oder -cyclohexanolat, zu einer Gruppe der Formel $R_6$—$Z_6$ reduziert oder einen Aldehyd der Formel

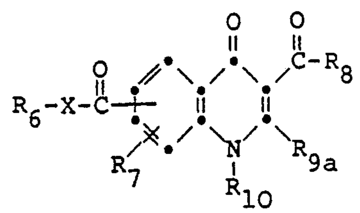

(II),

worin $R_6$ Wasserstoff und X eine direkte Bindung ist, mit einer Metallverbindung eines Niederalkans mit bis und mit 7 Kohlenstoffatomen der Formel $R_6$—H, wie einer Halogenmagnesium- oder Lithiumverbindung desselben, z.B. mit einem Niederalkyllithium, umsetzt, und gewünschtenfalls in einer verfahrensgemäss erhältlichen Verbindung der Formel XIXa Niederalkoxycarbonyl —C(=O)—$R_8$ zu Carboxy solvolysiert, Carboxy —C(=)—$R_8$ zu Niederalkoxycarbonyl verestert und/oder ein verfahrensgemäss erhältliches Salz in die freie Verbindung oder eine verfahrensgemäss erhältliche freie Verbingdung in ein Salz überführt.

Die Ausgangsstoffe der Formel XIX können, soweit sie neu sind, allgemein beispielsweise hergestellt werden, indem man eine Verbindung der Formel $R_1$—X—$Z_6$—Ph($NHR_E$)—H (V') in üblicher Weise z.B. wie für die Herstellung und intramolekulare Cyclisierung von Verbindungen der Formel IV beschrieben, mit Methantricarbonsäure oder einem funktionellen Derivat, wie einem Ester davon in üblicher Weise, z.B. wie für die Herstellung und intramolekulare Kondensation von Verbindungen der Formel IV angegeben, kondensiert, Verbindungen der Formel XIX, in denen $Z_6$ Hydroxymethlen, Iminomethylen bzw. veräthertes Dihydroxymethylen bedeutet, können ferner durch Umsetzung eines entsprechenden Aldehydes, Nitrils bzw. Orthocarbonsäureesters mit einer Metallverbindung des betreffenden Kohlenwasserstoffes der Formel $R_1H$, z.B. mit einer entsprechenden. Verbindungen der Formel XIX, in denen $Z_6$ Dihalogenmethylen bedeutet, können ferner hergestellt werden, indem man in eine entsprechende, im Benzoring unsubstituierte Verbindung den $R_1$-Dihalogenmethyl-Rest einführt, z.B. durch Umsetzung mit dem entsprechenden $R_1$-Trihalogen-methan in Gegenwart von Aluminiumchlorid.

Die Verbindungen der Formel I können ferner hergestellt werden, indem man in einer Verbindung der Formel XXI

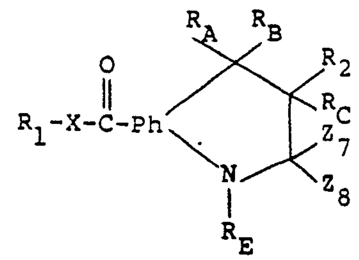

(XXI),

worin $Z_7$ einen in Hydroxy überführbaren Rest und $Z_8$ einen Rest $R_D$ bedeutet oder $Z_7$ und $Z_8$ jeweils einen einwertigen oder gemeinsam einen zweiwertigen in Oxo überführbaren Rest bedeuten, $Z_7 + Z_8$ in Oxo überführt, und gewünschtenfalls die so erhältliche Verbindung in eine andere Verbindung der Formel I und/ oder eine erhaltene salzbildende Verbindung in ein Salz oder ein erhaltenes Salz in die freie Verbindung oder in ein anderes Salz umwandelt.

In Hydroxy überführbare Reste sind beispielsweise veresterte Hydroxygruppen, gegebenenfalls verätherte Mercaptogruppen oder gegebenenfalls substituierte Aminogruppen. Einwertige, gemeinsam in die Oxogruppe überführbare Reste sind beispielsweise veresterte oder mit einem einwertigen offenkettigen Alkohol verätherte Hydroxygruppen oder mit einem offenkettigen Mercaptan verätherte Mercaptogruppen. Durch $Z_7$ und $Z_8$ gemeinsam dargestellt zweiwertige, in Oxo überführbare Reste sind beispielsweise Thioxo, mit einem 2-wertigen Alkohol, Mercaptoalkohol oder Mercaptan verätherte Hydroxygruppen, oder gegebenenfalls substituierte Iminogruppen.

Veresterte Hydroxygruppen sind beispielsweise mit einer Mineralsäure, einer organischen Sulfonsäure oder einer Carbonsäure veresterte Hydroxygruppen, wie Halogen, z.B. Chlor, Brom oder Jod, aliphatisches oder atomatisches Sulfonyloxy, z.B. Methan-, Aethan-, Benzol- oder p-Toluolsulfonyloxy, oder von einer organischen Carbonsäure oder einem monofunktionellen Kohlensäurederivat, wie einem Kohlensäurehalbester, einer Halogenameisensäure oder der gegebenenfalls substituierte Carbaminsäure, abgeleitetes Acyloxy, z.B. Niederalkanoyloxy, gegebenenfalls substituiertes Benzoyloxy, Niederalkoxycarbonyloxy, gegebenenfalls substituiertes Phenoxycarbonyloxy, Chlor- oder Bromcarbonyloxy oder gegebenenfalls substituiertes, wie niederalkyliertes, Carbamoyloxy. Veräthertes Mercapto ist beispielsweise eine Aliphatylthio oder Arylthiogruppe z.B. Niederalkylthio oder gegebenenfalls substituiertes Phenylthio. Substituiertes Amino ist beispielsweise durch Hydroxy oder Amino monosubstituiertes, durch aliphatische Reste oder gegebenenfalls substituiertes Phenyl mono- oder disubstituiertes Amino, wobei aliphatische Reste ein- oder zweiwertig sein können, einwertige aliphatische Reste beispielsweise Niederalkyl und zweiwertige aliphatische Reste, z.B. Niederalkylen bzw. Aza-, Oxa- oder Thianiederalkylen darstellen, z.B. Hydroxyamino, Hydrazino, Mono- oder Di-niederalkylamino, gegebenenfalls substituiertes Anilino, Pyrrolidino, Piperidino, Morpholino, Thiamorpholino oder N'-Niederalkyl-, wie N'-Methyl-piperazino. Mit einem einwertigen Alkohol veräthertes Hydroxy ist beispielsweise Niederalkoxy oder gegebenenfalls substituiertes Phenyloxy. Mit einem zweiwertigen Alkohol verätherte Hydroxygruppen sind beispielsweise Niederalkylendioxy- oder gegebenenfalls substituierte 1,2-Phenylendioxy-gruppen, z.B. Aethylendioxy, 1,3- Propylendioxy oder 1,2-Phenylendioxy. Mit einem zweiwertigen Hercaptoalkohol oder Mercaptan verätherte Hydroxygruppen sind beispielsweise Niederalkylenditio- bzw. Niederalkylenoxythiogruppen oder gegenbenenfalls substituierte 1,2-Phenyldithio- bzw. Phenoxythiogruppen, z.B. Aethylendithio, 1,3-Propylendithio oder 1,2-Phenylendithio. Gegebenenfalls substituierte Iminogruppen sind beispielsweise durch Hydroxy, Amino, Niederalkyl oder gegebenenfalls substituiertes Phenyl substituierte Iminogruppen, z.B. Oximino, Hydrazono, Niederalkylimino oder Anilo.

Die Ueberführung der genannten Gruppen in Hydroxy bzw. in Oxo erfolgt in üblicher Weise, beispielsweise durch Solvolyse, insbesondere Hydrolyse. Dabei arbeitet man in üblicher Weise, beispielsweise in Gegenwart eines sauren oder basischen Hydrolysemittels und/oder eines geeigneten Lösungs- oder Verdünnungsmittels, erforderlichenfalls unter Kühlen oder Erwärmen, z.B. in Temperaturbereich von etwa 0—100°C unter Inertgas, wie Stickstoff, und/oder im geschlossenen Gefäss. Saure Hydrolysemittel sind beispielsweise Protonensäuren, wie Mineralsäuren z.B. Chlor-, Brom- oder Iodwasserstoffsäure, Schwefelsäure oder Phosphorsäure, Sulfonsäuren, z.B. Methan-, Aethan-, Benzol- oder p-Toluolsulfonsäure oder Sulfaminsäure, oder organische Carbonsäuren, wie Niederalkansäuren oder gegebenenfalls substituierte Benzolsäuren. Basische Hydrolysemittel sind beispielsweise Alkalimetallhydroxyde oder -carbonate, z.B. Natriumhydroxyd bzw. -carbonat, Kaliumhydroxyd bzw. -carbonat oder Calciumhydroxyd, ferner Stickstoffbasen, wie Ammoniak oder organische Amine. Geeignete Lösungs- oder Verdünnungsmittel sind insbesondere mit Wasser mischbare Lösungsmittel.

Reaktionsfähig veresterte, d.h. mit einer Halogenwasserstoffsäure oder einer organischen Sulfonsäure veresterte, Hydroxygruppen können ferner durch übliche Alkoholyse, d.h. Umsetzung mit dem gewünschten verätherten Hydroxygruppe entsprechenden Alkohol, in veräthertes Hydroxy überführt werden, beispielsweise in Gegenwart eines basischen Mittels, erforderlichenfalls in einem Lösungs- oder Verdünnungsmittel unter Kühlen oder Erwärmen, z.B. im Temperaturenbereich von etwa 0—100°C, unter Inertgas wie Stickstoff und/oder im geschlossenen Gefäss. Geeignete basische Mittel sind beispielsweise Alkalimetallhydroxyde oder -carbonate, z.B. Natriumhydroxyd bzw. -carbonat oder Kaliumhydroxyd bzw. -carbonat, organische insbesondere tertiäre organische, Stickstoffbasen, wie Triniederalkylamine, z.B. Triäthylamin, oder Pyrridin. Statt ein basisches Mittel zu verwenden, kann man den betreffenden Alkohol vorteilhafterweise auch in Form eines Alkoholates, wie Alkalimetallalkoholates z.B. des Natrium- oder Kaliumalkoholates einsetzen.

Die Ausgangsstoffe der Formel XXI können, soweit sie neu sind, nach an sich bekannten Methoden erhalten werden, beispielsweise indem man eine Verbindung der Formel

$$R_1—X—C(=O)—Ph(H)—N(R_E)—C(Z_7)(Z_8)—C(R_4)—(R_C)—COOH \qquad (XXII)$$

oder ein geeignetes funktionelles Derivat, wie einen Ester davon cyclisiert. Die Herstellung von Verbindungen der Formel XXII sowie ihre Cyclisierung erfolgt insbesondere wie für die Herstellung von Verbindungen der Formel IV und ihre Cyclisierung angegeben.

Verbindungen der Formel I, in denen X Oxy bedeutet, und ihre Tautomeren können weiterhin hergestellt werden, indem man in einer Verbindung der Formel XXIII

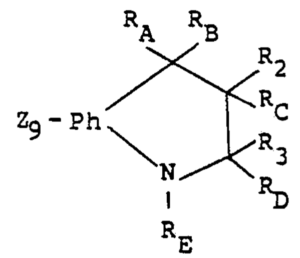

(XXIII),

worin $Z_9$ eine von der Gruppe $R_1—X—C(=O)—$ verschiedene funktionell abgewandelte Carboxygruppe bedeutet, $Z_9$ zu einer entsprechenden Gruppe $R_1—O—C(=O)—$ solvolysiert und gewünschtenfalls die so erhältliche Verbindung in eine andere Verbindung der Formel I und/oder eine erhaltene salzbildende Verbindung in ein Salz oder ein erhaltenes Salz in die freie Verbindung oder in ein anderes Salz überführt.

Derartige Gruppen sind beispielsweise Cyano, anhydridisierte Carboxygruppen, Iminoäthergruppen, Iminoestergruppen sowie verätherte und/oder veresterte Trihydroxymethylgruppen. Anhydridisierte Carboxygruppen sind beispielsweise mit einer Mineralsäure, wie einer Halogenwasserstoffsäure, mit einer Halogensulfonsäure, wie Fluorosulfonsäure, oder einer organischen Sulfon- oder Carbonsäure, wie einer aliphatischen oder aromatischen Sulfonoder Carbonsäure, anhydridisierte Carboxygruppe. Iminoäthergruppen sind beispielsweise von veresterten Carboxygruppen sind beispielsweise von veresterten Carboxygruppen $R_1—O—C(=O)—$ abgeleitete Iminoäthergruppen, wie O-Niederalkylcarbamoyl, oder cyclische Iminoäthergruppen, wie 4,4- oder 5,5-Diniederalkyl-, z.B. 4,4- oder 5,5-Dimethyl-4,5-dihydro-oxazolyl-(2), oder 4,4,4-Triniederalkyl-, z.B. 4,4,6-Trimethyl-5,6-dihydro-oxazinyl-(2). Iminoestergruppen sind beispielsweise von amidierten Carboxygruppen $R_4$ abgeleitete, mit Halogenwasserstoffsäuren oder organischen Carbonsäuren, wie Niederalkansäuren, veresterte Iminoestergruppen, wie z.B. Chlorcarbimino oder O-Niederalkanoylcarbamoyl. Verätherte und/oder veresterte Trihydroxymethylgruppen sind beispielsweise Triniederalkoxymethyl oder Trihalogenmethylgruppen.

Die genannten Gruppen können durch übliche Hydrolyse in Carboxygruppen, Iminoäthergruppen sowie veratherte Hydroxy-dihalogenmethyl-, wie Niederalkoxy-dihalogenmethylgruppen, ferner in veresterte Carboxygruppen, $R_1—O—C(=O)—$ überführt werden. Die Hydrolyse erfolgt in üblicher Weise, beispielsweise in Gegenwart eines sauren oder alkalischen Hydrolysemittels, üblicherweise in Gegenwart eines Lösungs- under/oder Verdünnungsmittels oder eines Gemisches davon, und, wenn notwending, unter Kühlen oder Erwärmen z.B. in einem Temperaturbereich won etwa 0°C bis etwa 120°C, erfoderlichenfalls unter Inertgas, wie Stickstoff, und/oder in geschlossenem Gefäss. Saure Hydrolysemittel sind beispielsweise Mineralsäuren, wie Halogenwasserstoffsäuren, z.B. Salzsäure, oder Sauerstoffsäuren des Schwefels oder Phosphors, wie Schwefel- oder Phosphorsäure, ferner organische Sulfonsäuren, z.B. p-Toluolsulfonsäure oder Mesitylensulfonsäure, oder organische Carbonsäuren, wie Niederalkancarbonsäuren, z.B. Ameisen- oder Essigsäure. Basische Hydrolysemittel sind beispielsweise Alkalimetallhydroxyde, z.B. Natrium- oder Kaliumhydroxyd, ferner Alkalimetallcarbonate, z.B. Natrium- oder Kaliumcarbonat. Geeignete Lösungs- oder Verdünnungsmittel sind vorzugsweise mit Wasser mischbare Lösungsmittell, wie Niederalkanole, z.B. Aethanol oder Methanol, niedere Ketone, z.B. Aceton, oder tertiärer Alkansäureamide, z.B. Dimethylformamid oder N-Methylpyrrolidon.

Anhydridiserte Carboxygruppen sowie cyclische Iminoäthergruppen können ferner durch Alkoholyse, d.h. Umsetzung mit einem entsprechenden Alkohol, in veresterte Carboxygruppen $R_1—O—C(=)—$ überführt werden. Dabei arbeitet man in üblicher Weise, ausgehend von anhydridisierten Carboxygruppen beispielsweise in Gegenwart eines basischen Kondensationsmittels, wie eines Alkalimetallhydroxydes oder -carbonates, z.B. von Natrium- oder Kaliumhydroxyd bzw. entsprechender Carbonate, oder von organischen Stickstoffbasen, wie Pyridin oder Triäthylamin, und ausgehend von cyclischen Iminoäthergruppen vorzugweise unter wasserfreien Bedingungen, z.B. in Gegenwart von Cheorwasserstoff, Phosphorsäure, Schwefelsäure oder p-Toluolsulfonsäure, erforderlichenfalls unter Erwärmen, beispielsweise im Temperaturbereich von etwa 0°C bis etwa 150°C, unter Inertgas, wie Stickstoff, und/oder in einem geschlossenen Gefäss.

Die Ausgangsstoffe der Formel XXIII können, soweit sie neu sind, nach an sich bekannten Methodfen hergestellt werden, beispielsweise durch Umsetzung einer Verbindung der Formel $Z_9$—Ph($NH_2$)—COOH (XXIV) mit Phosgen zum Isatosäureanhydrid, N-Substitution desselben und Kondensation mit Malonsäure oder einem Ester oder Anhydrid davon insbesondere in Analogie zu der für die intramolekulare Kondensation von Verbindungen der Formel VI beschriebenen Weise.

Die Verbindungen der Formel I, worin X eine direkte Bindung bedeutet, können weiterhin hergestellt werden, indem man eine Verbindung der Formel XXV

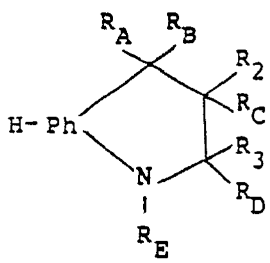

(XXV),

in Gegenwart einer Lewissäure mit einer Verbindung der Formel $R_1$-COOH (XXVI) oder einem geeigneten funktionellen Derivat davon umsetzt, und gewünschtenfalls die so erhältliche Verbindung in eine andere Verbindung der Formel I und/oder eine erhaltene salzbildende Verbindung in ein Salz oder ein erhaltenes Salz in die freie Verbindung oder in ein anderes Salz überführt.

Für obige Umsetzung geeignete funktionelle Derivate von Säuren oder Formel XXVI sind insbesondere Ester oder Anhydride derselben, wie Niederalkyl- oder gegebenenfalls substituierte Phenylester, deren symmetrische Anhydride oder deren gemischte Anhydride mit anderen Carbonsäuren, wie Ameisensäure, oder insbesondere mit Mineralsäuren, wie Halogenwasserstoffsäuren z.B. mit Chlorwasserstoffsäure. Lewissäuren sind beispielsweise koordinativ-ungesättigte Halogenide des Zinks, Bors, Aluminiums, Galliums, Zinns, Antimons oder Eisens, wie Zinkchlorid, Bortrichlorid bzw. Bortrifluorid, Aluminiumchlorid oder Aluminiumbromid, Galliumbromid, Zinnchlorid, Antimontrichlorid bzw. Antimonpentachlorid oder Eisentrichlorid.

Die Umsetzung erfolgt in für analoge Reaktionen bekannter Weise, beispielsweise in einem gegenüber den Reaktionsteilnehmern inerten, weitgehend wasserfreien Lösungsmittel, erforderlichenfalls unter Kühlen oder Erwärmen, z.B. im Temperaturbereich von -30 bis 100°C, unter Inertgas, wie Stickstoff, und/oder im geschlossenen System.

Die Ausgangsstoffe der Formel XXV können, soweit sie neu sind, nach an sich bekannten Methoden hergestellt werden, beispielsweise durch üblichen, beispielsweise der Herstellung und intramolekularen Cyclisierung von Verbindungen der Formel VI entsprechenden, Ringschluss von Verbindungen der Formel H-Ph(H)—N($R_E$)—C(=O)—CH($R_2$)—C(=O)—Z (XXVI), worin Z eine verätherte oder reaktionsfähig veresterte Hydroxygruppe, z.B. Niederalkoxy oder Halogen, bedeutet.

Die Verbindungen der Formel I, worin X direkte Bindung ist, können ferner hergestellt werden, indem man eine Verbindungen der Formel XXVII

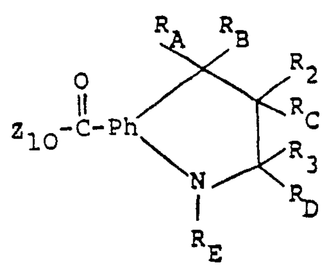

(XXVII),

worin $Z_{10}$ eine freie, verätherte oder reaktionsfähig veresterte Hydroxygruppe oder eine geeignete, über ein Ringstickstoffatom gebundene Heteroarylgruppe bedeutet, oder ein Salz davon mit einer Verbindung der Formel $R_1$—M (XXVIII), worin M einen Metallrest darstellt, zu einer entsprechenden Verbindung der Formel (I), worin $R_1$ von Wasserstoff verschieden ist, oder eine Verbindung der Formel (XXVII), worin $Z_{10}$ reaktionsfähig verestertes Hydroxy ist, mit Bis-(triphenylphin)-kupferboranat zu einer Verbindung der Formel I, worin $R_1$ Wasserstoff ist, unsetzt, und gewünschtenfalls die so erhältliche Verbindung in eine andere Verbindung der Formel I und/oder eine erhaltene salzbildende Verbindung in ein Salz oder ein erhaltenes Salz in die freie Verbindung oder in ein anderes Salz überführt. Verätherte Hydroxygruppen sind

beispielsweise mit einem aliphatischen Alkohol, z.B. einem Niederalkanol, oder einem gegebenenfalls substituierten Phenol verätherte Hydroxygruppen. Reaktionsfähig veresterte Hydroxygruppen sind insbesondere mit einer Halogenwasserstoffsäure, insbesondere mit Chlorwasserstoffsäure, veresterte Hydroxygruppen. Ein über ein Ringstickstoffatom gebundener Heteroarylrest ist beispielsweise 1-Imidazol. Metallradikale M sind beispielsweise Alkalimetallatome, z.B. Lithium, oder Magnesium- oder Cadmiumradikale, insbesondere der Formel —Mg/2, —Cd/2, —MgHal oder CdHal, worin Hal Halogen, vor allem Chlor, Brom oder Iod darstellt, oder Kupferradiakale. Salze von Verbindungen der Formel (XXVII) sind beispielsweise Alkalimetall-, wie Natrium-, Kalium oder Lithiumsalze, oder Ammoniumsalze derselben.

Die Umsetzung erfolgt in Üblicher, insbesondere in der für analoge Reaktionen bekannten, Weise, vorzugsweise in einem gegenüber den Reaktionsteilnehmern inerten, weitgehend wasserfreien Lösungsmittel, erforderlichenfalls unter Kühlen oder gelindem Erwärmen, z.B. im Temperaturbereich von -100 — +80°C, unter Inertgas, wie Stickstoff, und/oder im geschlossenen Gefäss. So kann man beispielsweise eine Verbindung der Formel XXVII, in der $Z_{10}$ Halogen bedeutet, bei Temperaturen bis etwa 40°C mit einer Cadmium- organischen Verbindung der Formel XXVIII, oder bei Temperaturen unter -25°C, vorzugsweise unterhalb von -50°C mit einer Halogenmagnesium-, Lithium- oder insbesondere Kupferverbindung der Formel XXVIII umsetzen.

Eine erfindungsgemäss erhältliche Verbindung der Formel I kann in an sich bekannter Weise in eine andere Verbindung der Formel I umgewandelt werden.

So kann man eine Verbindung der Formel I, worin $R_1$ einen nicht-aromatischen Rest und X eine direkte Bindung bedeutet, in üblicher Weise zu einer Verbindung der Formel I oxidieren, worin $R_1X$ Hydroxy ist, z.B. mit einem Alkalimetall-hyphohalogenit.

Weiterhin kann man in einer Verbindung der Formel I bzw. XIXa Carboxy $R_2$ oder $R_1$—X—C(=O)— nach bekannten Veresterungsverfahren in eine veresterte Carboxygruppe $R_2$ bzw. $R_1$—X—C(=O)— umwandeln. So kann man z.B. durch Behaldeln mit einem Alkohol in Gegenwart eines geeigneten Kondensationsmittels, wie eines dehydratisierenden Mittels, z.B. von Dicyclohexylcarbodiimid, oder einer Mineralsäure, z.B. von Schwefelsäure oder Chlowasserstoffsäure oder, zur Bildung einer Hydroxyniederalkylgruppe, mit einem entsprechenden Epoxyniederalkan umsetzen. Die Veresterung kann man ferner durch Behandeln mit einem geeigneten N,N-Diniederalkylformamidacetal, z.B. N,N-Dimethylformamiddiäthylacetal, oder N,N,O-Trimethylformamidiniummethosulfat, einem Carbonat oder Pyrocarbonat, z.B. mit Diäthyl(pyro)carbonat, oder mit einem organischen Sulfit oder Phosphit, wie einem Diniederalkylsulfit oder Triniederalkylphosphit, im letzten Fall in Gegenwart eines geeigneten sauren Mittels, z.B. von p-Toluolsulfonsäure, durchführen. Ferner kann man eine zu veresternde Säure der Formel I, worin die freie Carboxygruppe in Salzform z.B. in einer Alkalimetallsalz-, wie der Natriumsalzform, vorliegt, mit einem reaktionsfähigen Ester eines Alkohols, wie einem entsprechenden Halogenid, z.B. Chlorid, Bromid oder Iodid, oder mit Schwefelsäureester umsetzen. Die Veresterung kann man jedoch auch durch Umsetzung mit einem der einzuführenden Niederalkoxygruppe entsprechenden Niederalken, vorzugsweise in Gegenwart eines sauren Kondensationsmittels, wie einer starken Protonensäure, z.B. von Schwefelsäure oder einer Lewissäure, z.B. von Bortrifluorid-ätherat, durchführen. In einem der genannten Veresterungsreagentien können gegebenenfalls vorhandene Substituenten in funktionell abgewandelter Form vorliegen und dann in einer Verbindung der Formel I, worin $R_2$ bzw. $R_3$ beispielsweise für substituiertes Niederalkoxycarbonyl steht, in welcher Substituenten in funktionell abgewandelter Form vorliegen, freigesetzt werden. So kann man als Veresterungsreagenz z.B. das 2,3-Epoxy-propylchlorid verwenden und im erhaltenen Ester die 2,3-Epoxy-propyloxygruppierung nachträglich zur gewünschten 2,3-Dihydroxy-proplyoxygruppierung hydrolisieren.

In Verbindungen der Formel I bzw. XIXa kann verestertes Carboxy $R_2$ und/oder $R_1$—X—C(=O)— durch Umesterung, z.B. durch Behandeln mit einem Alkohol, erforderlichenfalls in Gegenwart eines geeigneten Umesterungskatlysators, wie eines betreffenden Aklalimetall-, z.B. des betreffenden Natrium- oder Kaliumalkoholates, oder einer Mineralsäure, z.B. von Schwefelsäure oder Chlorwasserstoffsäure in eine andere veresterte Carboxygruppe $R_2$ bzw. $R_1$—X—C(=O)— umgewandelt werden.

Die Verbindungen der Formel I bzw. XIXa kann freies oder verestertes Carboxy $R_2$ in an sich bekannter Weise in gegebenenfalls substituiertes Carbamyl umgewandelt werden. So kann man einen Ester der Formel I bzw. XIXa mit Ammoniak, Hydroxylamin oder einem entsprechenden primären oder sekundären Amin behandeln und so zu erfindungsgemässen Amiden gelangen. Ferner kann man das Ammoniumsalz oder ein Aminsalz einer Säure der Formel I durch Dehydratisierung, z.B. unter Erhitzen oder durch Einwirkung eines geeigneten Dehydratisierungsmittels, wie Schwefelsäure, in ein Amid der Formel I überführen.

Die Ueberführung von Carboxy in verestertes oder amidiertes Carboxy kann aber auch so erfolgen, dass man die Carboxyverbindung zunächst in ein reaktionsfähiges Carboxyderivat, z.B. durch Umsetzung mit N,N'-Bis-(Imidazolyl-1)-harnstoff in das 1-Imidazolid, mit einem reaktionsfähigen Alkohol, z.B. mit 4-Nitrophenol oder Cyano-methanol in einem reaktionsfähigen Ester oder mit einem Halogenierungsmittel, wie Thionylchlorid in das Säurechlorid überführt und anschliessend mit dem entsprechenden Alkohol bzw. mit Ammoniak oder einem mindestens ein Wasserstoffatom aufweisenden Amin zur Reduktion bringt.

In Verbindungen der Formel I bzw. XIXa kann man verestertes oder amidiertes Carboxy $R_2$ bzw. $R_1$—X—C(=O)— in üblicher Weise in die freie Carboxygruppe überführen, beispielsweise durch Hydrolyse, üblicherweise in Gegenwart eines sauren oder basischen Hydrolysemittels, wie einer Mineral- oder

Carbonsäure, z.B. von Chlorwasserstoffsäure, Schwefelsäure oder Essigsäure, oder eines Alkalimetallhydroxydes oder -carbonates, z.B. von Natriumhydroxyd bzw. -carbonat oder von Kaliumhydroxyd bzw. -carbonat.

Ferner kann man Verbindungen der Formel I bzw. XIXa worin $R_A$ und $R_B$ gemeinsam Oxo, $R_C$ und $R_D$ gemeinsam eine zusätzliche Bindung und $R_E$ Wasserstoff bedeuten, und ihre Tautomeren in üblicher Weise am Stickstoffatom in 1-Stellung durch einen von Wasserstoff verschiedenen Rest $R_E$ substituieren und/oder am 4-ständigen Sauerstoffatom zu einer veräthertenHydroxygruppe $R_A$ veräthern. Die Substitution des Ringstickstoffatoms bzw. die Verätherung der ringgebundenen Hydroxygruppe erfolgt in üblicher Weise, beispielsweise durch Umsetzung mit einem reaktionsfähigen Ester eines entsprechenden Alkohols, erforderlichenfalls in Gegenwart eines basischen Kondensationsmittels, wie eines Alkalimetallhydrides, -amides, -alkoholates oder -hydroxydes, z.B. von Natriumhydrid, Natriumamid, Lithium-diisopropylamid, Natriummethanolat, Kaliumtertiärbutanolat oder Kaliumhydroxyd, unter Kühlen oder Erwärmen, z.B. im Temperaturbereich von etwa 0—120°C, unter Inertgas, wie Stickstoff und/oder im geschlossenen Gefäss. Reaktionsfähige Ester entsprechender Alkohole sind insbesondere Mineralsäureester, wie Halogenwasserstoffsäure- oder Schwefelsäureester, oder organische Sulfonsäureester, z.B. Methan, Aethan,- Benzol-, p-Toluol- oder Fluorsulfonsäureester. Bei der Behandlung eines Esters der Formel I mit einem Alkylhalogenid in Gegenwart von Natriumhydrid in Dimethylformamid werden dabei ausgehend von Verbindungen, in denen $R_2$ eine wie angegebene veresterte Carboxygruppe ist, praktisch ausschliesslich N-Alkylderivate erhalten.

Ferner kann man in Verbindungen der Formel I bzw. XIXa zusätzliche Substituenten in den Rest Ph einführen, vorzugsweise Niederalkylgruppe und/oder Halogen. So kann man beispielsweise durch Umsetzung mit eiem Niederalkylhalogenid, einem Niederalkanol oder einem Niederalken in Gegenwart einer Lewissäure, z.B. von Aluminiumtrichlorid, Niederalkyl einführen, insbesondere in 8-Stellung. Ferner kann man in üblicher Weise, z.B. durch Umsetzung mit dem betreffenden Halogen in Gegenwart einer Lewissäure, wie des entsprechenden Eisenhalogenides, das durch *in situ* aus fein verteiltem Eisen und dem betreffenden Halogen gebildet werden kann, oder durch Behandlung mit N-Chlorsuxinimid, Halogen einführen, vorzugsweise ebenfalls in 8-Stellung.

Salzbildende Verbindungen der Formel I bzw. XIXa können in an sich bekannter Weise in Salze überführt werden, beispielsweise durch Behandeln mit einer Base oder einer Säure, üblicherweise in Gegenwart eines Lösungs- oder eines Verdunnungsmittels. Erhaltene Salze können in an sich bekannter Weise in die freien Verbindungen umgewandelt werden, z.B. durch Behandeln mit einem sauren Reagenz, wie einer Mineralsäure. bzw. einem basischen Reagenz, wie einem Alkalimetallhydroxyd. Die Verbindungen der Formel I, einschliesslich ihrer Salze, können auch in Form ihrer Hydrate erhalten werden oder das zur Kristallisation verwendete Lösungsmittel einschliessen. Infolge der engen Beziehung der Verbindungen der Formel I in der freier Form und in Form ihrer Salze, sind vor- und nachstehend unter den freien Verbindungen oder ihrer Salze sinn- und zweckgemäss, gegebenenfalls auch die entsprechenden Salze bzw. freien Verbindungen zu verstehen.

Die Erfindung betrifft auch diejenigen Ausführungsformen des Verfahrens, bei denen man von einer auf einer beliebigen Verfahrensstufe als Zwischenprodukte erhältlichen Verbindung ausgeht und die fehlenden Verfahrensschritte ausführt oder einen Ausgangsstoff unter den Reaktionsbedingungen bildet oder in Form eines Derivates oder gegebenenfalls eines Salzes verwendet. Beim Verfahren der vorliegenden Erfindung werden vorzugsweise diejenigen Ausgangsstoffe verwendet, welche zu den eingangs als besonders wertvoll hervorgehobenen Verbindungen führen. Neue Ausgangsstoffe, Analogieverfahren zu ihrer Herstellung und ihre Verwendung bilden ebenfalls einen Gegenstand der vorliegenden Erfindung.

Die Verbindungen der Formel I bzw. XIXa und ihre Salze zeigen wertvolle pharmakologische Eigenschaften. Insbesondere weisen sie antiallergische Wirkungen auf, die z.B. an der Ratte in Dosen ab etwa 1 mg/kg bei intravenöser und in Dosen ab etwa 1 mg/kg bei oraler Verabreichung in passiven kutanen Anaphylaxietest (PCA-Reaktion), der analog der von Goose und Blair, Immunology, Vol. 16, S.794 (1969) beschriebenen Methode durchgeführt wird, nachgewiesen werden können. Die passive kutane Anaphylaxie wird dabei nach dem von Ovary Progr. Allergy, Vol. 5, S.459 (1958) beschreibenen Verfahren erzeugt. Die antiallergische, insbesondere degranulationshemmende Wirkung der Verbindung der Formel I und ihrer Salze kann auch *in vitro* anhand der Histaminfreisetzung aus Peritonealzellen der Ratte im Dosisbereich von etwa 1 bis etwa 100 mg/1 bei immunologisch induzierter Freisetzung, wobei z.B. mit Nippostrongilus brasiliensis infestierte Ratten verwendet werden, und von etwa 1,0 bis etwa 100 mg/1 bei chemisch induzierter Freisetzung, wobei diese z.B. mit einem Polymeren von N-4-Methoxy-phenylethyl-N-methyl-amin bewirkt wird, festgestellt werden.

Aus GB—A—1,334,705 sowie aus Journal of Medicinal Chemistry *17*, Seiten 685—690 (1974) und *21*, Seiten 984—988 (1978) waren bereits antiallergische wirksame 4-Oxo-1,4-dihydro-chinolin-2-carbonsäureverbindungen bekannt. Ferner wurden in GB—A—830 832 und GB—A—1,502,405, 1-Alkyl- bzw. 1-Alkenyl-4-oxo-1,4-dihydro-chinolin-3-carbonsäuren als antibakterielle Arzneimittelwirkstoffe vorgeschlagen. Nach Journal of Medicinal Chemistry 17, loc.cit. weisen einige 4-Oxo-1,4-dihydro-chinolin-3-carbonsäureverbindungen zudem antiallergische Eigenschaften auf, die indes als gering und nur in einem Fall interessantes Ausmass erreichend angesehen wurden. Die die antiallergische Aktivität betreffenden Versuchsdaten beziehen sich jedoch durchwegs auf intravenöse bzw. intraperitoneale Applikationsweise. Anhaltspunkte für eine antiallergische Wirkung bei enteraler, z.B. oraler, Applikation fehlen völlig. Im Gegenteil, im

Journal of Medicinal Chemistry *21*, loc.cit. wird festgestellt, dass keine der wirksamen Verbindungen bei oraler Applikation aktiv war. Der Vorteil der erfindungsgemässen Verbindungen wird demgegenüber in ihrer guten antiallergischen Wirksamkeit bei oraler Applikationsweise gesehen.

Die Verbindungen der vorliegenden Erfindung sind, dementsprechend als Hemmer allergischer Reaktionen, z.B. in der Behandlung und Prophylaxe von allergischen Erkrankungen, wie des Asthma bronchale, sowohl der "extrinsic" als auch der "intrinsic" Form, oder anderer allergischer Erkrankungen, wie der allergischen Rhinitis, z.B. des Heufiebers, der Konjunktivitis oder allergischer Dermatiden z.B. der Urticaria oder von Ekzemem, verwendbar.

Wie bereits erwähnt, betrifft die vorliegende Erfindung auch pharmazeutische Präparate, welche Verbindungen der Formel I bzw. XIXa oder pharmazeutisch verwendbare Salze derselben enthalten. Bei den erfindungsgemässen pharmazeutischen Präparaten handelt es sich um solche zur enteralen, wie oralen, nasalen oder rektalen, sowie parenteralen oder buccalen Verabreichung an Warmblüter, welcher den pharmakologischen Wirkstoff alleine oder zusammen mit einem pharmazeutisch verwendbaren Trägermaterial enthalten. Die Dosierung des Wirskstoffs hängt von der Warmblüterspezies, dem Alter und dem individuellen Zustand und von der Applikationsweise ab.

Die erfindungsgemässen pharmazeutischen Präparate enthalten z.B. bis etwa 95%, vorzugsweise von etwa 5% bis etwa 90% des Wirkstoffs. Erfindungsgemässe pharmazeutische Präparate sind z.B. solche in Dosiseinheitsformen, wie Dragées, Tabletten, Kapseln, Suppositorien oder Ampullen, ferner Inhalationspräparate bzw. topisch oder lokal, z.B. zur Insufflation, verwendbare pharmazeutische Zubereitungen.

Die pharmazeutischen Präparata der vorliegenden Erfindung werden in an sich bekannter Weise, z.B. mittels konventioneller Misch-, Granulier-, Lösungs- oder Lyophilisierungsverfahren hergestellt. So kann man pharmazeutische Präparate zur oralen Anwendung erhalten, indem man den Wirkstoff mit festen Trägerstoffen kombiniert, das so erhaltene Gemisch gegebenenfalls granuliert und das Gemisch bzw. Granulat, wenn erwünscht oder notwendig, nach Zugabe von geeigneten Hilfstoffen, zur Tabletten oder Dragée-Kernen verarbeiten. Geeignete Trägerstoffe sind insbesondere Füllstoffe, wie Zucker z.B. Laktose, Saccharose, Mannit oder Sorbit, Cellulosepräparate und/oder Calciumphosphate z.B. Tricalciumphosphat oder Calciumhydrogenphosphat, ferner Bindemittel, wie Stärkekleister, z.B. Mais-, Weizen-, Reis- oder Kartoffelstärkekleister, Gelatine, Tragakant, Methylcellulose und/oder Polyvinylpyrrolidon, und/oder, wenn erwünscht, Sprengmittel, wie die oben genannten Stärken, ferner Carboxymethylstärker, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglycol. Dragée-Kerne werden mit geeigneten, gegebenenfalls Magensaft-resistenten Ueberzügen versehen, wozu man z.B. konzentrierte Zuckerlösungsn, welche gegebenenfalls arabischen Gummi, Talk, Polyvinylpyrrolidol, Polyäthylenglycol und/oder Titandioxyd enthalten, Lacklösung in geeigneten organischen Lösungsmitteln oder Lösungsmittelgemischen oder, zur Herstellung von Magensaft-resistenten Ueberzügen, Lösung von geeigneten Cellulosepräparaten, wie acetylcellulosephthalat oder Hydroxypropylmethylcellulosephthalat, verwendet. Den Tabletten- oder Dragéeüberzügen können Farbstoffe oder Pigmente z.B. zur Identifizierung oder zur Kennzeichnung verschiedener Wirkstoffdosen beigefügt werden. Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Laktose, Bindemittel, wie Stärken und/oder Gleitmitteln, wie Talk oder Magnesiumstearat, und gegebenenfalls von Stabilisatoren enthalten. In weichen Kapseln ist der Wirkstoff vorzugsweise in geeigneten Flüssigkeiten, wie fetten Oelen, paraffinoel oder flüssigen Polyäthylenglycolen, gelöst oder suspendiert, wobei ebenfalls Stabilisatoren hinzugefügt sein können.

Als rektal anwendbare pharmazeutische Präparate kommen z.B. Suppositorien in Betracht, welche aus einer Kombination des Wirkstoffes mit einer Suppositoriengrundmasse bestehen. Als Suppositoriengrundmasse eignen sich z.B. natürliche oder synthetische Triglyceride, Paraffinkohlenwasserstoffe, Polyäthylenglycole oder höhere Alkanole. Ferner können auch Gelatine-Rektalkapseln verwendet werden, die eine Kombination des Wirkstoffs mit einer Grundmasse enthalten. Als Grundmasse kommen z.B. flüssige Triglyceride, Polyethylenglycole oder Parafinkohlenwasserstoffe in Frage.

Zur parenteralen Verabreichung eignen sich in erster Linie wässrige Lösungen des Wirkstoffs in wasserlöslicher Form, z.B. eines wasserlöslichen Salzes, ferner Suspensionen des Wirkstoffs, wie entsprechende oelige Injektionssuspensionen, wobei man geeignete lipophile Lösungsmittel oder Vehikel, wie fette Oele, z.B. Sesamoel oder synthetische Fettsäureester, z.B. Aethyloleat oder Triglyceride, verwendet, oder wässrige Injektionssuspensionen, welche viskositätserhöhende Stoffe, z.B. Natriumcarboxy-methylcellulose, Sorbit und/oder Dextran und gegebenenfalls Stabilisatoren enthalten.

Inhalationspräparate für die Behandlung der Atemwege durch nasale oder buccale Verabreichung sind z.B. Aerosole oder Sprays, welche den pharmakologischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise ein flüssiges Treibgas mit einem Siedepunkt unter der Raumtemperatur, sowie, wenn erwünscht, Trägerstoffe, wie flüssige oder feste, nicht-ionische oder anionische oberflächenaktive Mittel und/oder feste Verdünnungsmittel. Präparate, in welchen der pharmakologische Wirkstoff in Lösung vorliegt, enthalten ausser diesen ein geeignetes Treibmittel, ferner,

falls notwendig, ein zusätzliches Lösungsmittel und/oder einen Stabilisator. Anstelle des Treibgases kann auch Druckluft verwendet werden, wobei diese mittels einer geeigneten Verdichtungs- und Entspannungsvorrichtung nach Bedarf erzeugt werden kann.

Pharmazeutische Präparate für topische und lokale Verwendung sind z.B. für die Hautbehandlung Lotionen und Crème, die eine flüssige oder halbfeste Oel-in-wasser- oder Wasser-in-Oel-Emulsion enthalten, und Salben, gegebenenfalls ein Konservierungsmittel enthaltend, für die Augen Augentropfen, welche die aktive Verbindung in wässriger oder oeliger Lösung enthalten und Augensalben, die vorzugsweise in steriler Form hergestellt werden, für die Behandlung der Nase Puder, Aerosole und Sprays (ähnlich den oben beschriebenen für die Behandlung der Atemwege), sowie grobe Puder, die durch schnelles Inhalieren durch die Nase verabreicht werden und Nasentropfen, welche die aktive Verbindung in wässriger oder oeliger Lösung enthalten, oder für die lokale Mundbehandlung Lutschbonbons, welche die aktive Verbindung in einem allgemeinen aus Zucker un arabischem Gummi oder Tragakant gebildeten Masse enthalten, welcher Geschmacksstoffe beigegeben sein können, sowie Pastillen, die den Aktivstoff in einer inerten Masse, z.B. aus Gelatine und Glycerin oder Zucker und arabischem Gummi enthalten.

Die Erfindung betrifft schliesslich die Verwendung von Verbindungen der Formel I bzw. XIXa oder ihrer Salze als pharmakologisch aktive Verbindung, insbesondere als Antiallergika, vorzugsweise in Form von pharmazeutischen Präparaten Die tägliche Dosis, die einem Warmblüter von etwa 70 kg verabreicht wird, beträgt, bei oraler Applikationsform von etwa 100 mg bis etwa 1000 mg, vorzugsweise von etwa 250 mg bis etwa 750 mg.

Die nachfolgenden Beispiele illustrieren die vorstehend beschriebene Erfindung; sie sollen jedoch diese in ihrem Umfang in keiner Weise einschränken. Temperaturen sind in Celsiusgraden angegeben.

## BEISPIELE

### Beispiel 1

1,681 g Malonsäurediäthylester in 25 ml Dimethylacetamid werden auf 5° abgekühlt und portionsweise mit Natriumhydrid (0,47 g der 50%-igen Suspension in Mineralöl, entölt mit Hexan) versetzt. Dabei soll die Innentemperatur 20° nicht übersteigen. Nach Abklingen der exothermen Reaktion lässt man 15 Minuten bei Raumtemperatur nachrühren, erwärmt dann auf etwa 100° und tropft innerhalb von 60 Minuten 2,628 g N-Aethyl-5-butyryl-4-methyl-isatosäureanhydrid, gelöst in 25 ml Dimethylacetamid hinzu. Dann erhitzt man 5 Stunden auf 115°, dampft unter vermindertem Druck zur Trockne ein, giesst auf ein Gemisch 50 ml Eiswasser und 10 ml 2n-Salzsäure und lässt bis zur Kristallisation rühren. Man saugt ab, wäscht mit wenig Diäthyläther nach, kristallisiert aus Methanol um und erhält den 1-Aethyl-6-butyryl-4-hydroxy-7-methyl-carbostiryl-3-carbonsäureäthylester vom Smp. 124—126°C.

Das Ausgangsmaterial kann z.B. folgendermassen hergestellt werden.

14,6 g 4-Amino-2-methyl-butyrophenon werden in 85 ml Essigsäure gelöst, mit 15,07 g Mesoxalsäurediäthylester versetzt und 60 Minuten zum Rückfluss erhitzt. Man dampft unter vermindertem Druck zur Trockne ein, verrührt den Eindampfrückstand mit 25 ml Diäthyläther, saugt ab, wäscht zweimal mit eiskaltem Diäthyläther nach und trocknet unter vermindertem Druck. Man erhält den 5-Butyl-3-hydroxy-6-methyloxindol-3-carbonsäureäthylester. Smp. 180—181°.

13,0 g 5-Butyryl-3-hydroxy-6-methyl-oxindol-3-carbonsäureäthylester werden in 200 ml Methanol suspendiert und mit 85 ml 2n-Natronlauge versetzt. Man lässt 90 Minuten bei Raumtemperatur rühren, zieht unter vermindertem Druck das Methanol ab, säuert mit 2n-Salzsäure auf pH = 3—4 an, saugt ab, wäscht mit Wasser nach, löst in Essigsäureäthylester, trocknet über Magnesiumsulfat, dampft unter vermindertem Druck zur Trockne ein, degeriert mit wenig Methylenchlorid und saugt ab. Man erhält die 5-Butyryl-4-methyl-anthranilsäure von Smp. 184—186°.

8,0 g 5-Butyryl-4-methyl-anthranilsäure werden unter Zugabe von 20 ml 2n-Salzsäure in 100 ml Dioxan gelöst. Darauf wird unter leichter Kühlung bei 40—50° 30 Minuten lang ein schwacher Phosgenstrom eingeleitet. Dann wird unter vermindertem Druck auf 30 ml eingeengt, abgesaugt, zweimal mit wenig Diäthyläther gewaschen und unter vermindertem Druck getrocknet. Man erhält das 5-Butyryl-4-methyl-isatosäureanhydrid von Smp. 224—226°.

3,5 g 5-Butyryl-4-methyl-isatosäureanhydrid werden unter gelindem Erwärmen in 30 ml Hexamethylphosphorsäuretriamid gelöst, auf 20° abgekühlt und mit Natriumhydrid (0,68 g der 50%-igen Suspension in Mineralöl, mit Hexan entölt) versetzt. Man lässt bis zur klaren Auflösung nachrühren, kühlt auf 10° ab, fügt 2,21 g Aethyljodid hinzu, lässt 30 Minuten bei Raumtemperatur nachrühren, giesst auf ein Gemisch von 300 ml Eiswasser und 20 ml 2n-Salzsäure, saugt ab, wäscht zweimal mit Eiswasser nach, nimmt in Methylenchlorid auf, trocknet über Magnesiumsulfat und dampft zur Trockne ein. Man erhält N-Aethyl-5-butyryl-4-methyl-isatosäureanhydrid von Smp. 141—143°.

### Beispiel 2

In analoger Weise wie in Beispiel 1 beschrieben oder nach einer der in der Beschreibung aufgeführten Verfahrensweisen kann man ferner herstellen:

6-Butyryl-4-hydroxy-1,7-dimethyl-carbostiryl-3-carbonsäureäthylester, Smp. 130—131°,

6-Butyryl-4-hydroxy-7-methyl-1-propyl-carbostiryl-3-carbonsäureäthylester, Smp. 116—117° und

1-Allyl-6-butyryl-4-hydroxy-7-methyl-carbostiryl-3-carbonsäureäthylester, Smp. 112—114°.

Beispiel 3

2,65 g Malonsäurediäthylester werden in 40 ml Dimethylacetamid gelöst, auf 10° abgekühlt und unter Stickstoff mit Natriumhydrid (0,768 g der 50%-igen Suspension in Mineralöl, mit Hexan entölt) in das Natriumsalz überführt. Man erwärmt auf 105°, tropft innerhalb 1 Stunde 3,80 g N-Aethyl-5-butyryl-isatosäureanhydrid in 20 ml Dimethylacetamid hinzu und lässt 10 Stunden bei 120° und über Nacht bei Raumtemperatur rühren. Dann filtriert man über Diatomeenerde, destilliert des Dimethylacetamid unter vermindertem Druck ab, versetzt mit 250 ml Eiswasser und 20 ml 2n-Salzsäure, lässt einige Zeit rühren, saugt ab und lässt an der Luft trocknen. Das Rohprodukt wird aus Methanol/Wasser (90 ml + 45 ml) umkristallisiert, abgesaugt, mit 50%-igem Methanol gewaschen und unter vermindertem Druck getrocknet. Man erhält den 1-Aethyl-6-butyryl-4-hydroxy-carbostiryl-3-carbonsäureäthylester von Smp. 163—165°.

Das Ausgangsmaterial kann beispielsweise folgendermassen hergestellt werden:

81,3 g Acetanilid werden in 600 ml Schwefelkohlenstoff suspendiert und unter Rühren mit 76,7 g Butyrylchlorid und portionsweise mit 198 g Aluminiumtrichlorid versetzt. Man lässt einige Zeit bei 30—35° rühren und erhitzt 18 Stunden zum Sieden. Dann lässt man abkühlen, rührt in 200 ml Eiswasser ein und extrahiert zweimal mit je 500 ml Essigester. Die organischen Phasen werden vereinigt, mit 500 ml Zusatzsäure und zweimal mit je 500 ml 15%-iger Natriumcarbonatlösung gewaschen, über Magnesiumsulfat getrocknet und unter vermindertem Druck auf 150 ml eingeengt. Man lässt abkühlen, saugt ab, wäscht zweimal mit Diäthyläther und trocknet unter vermindertem Druck. Man erhält das p-Acetamino-butyrophenon vom Smp. 141—143°.

20 g p-Acetamino-butyrophenon werden in 40 ml halbkonzentrierter Salzsäure suspendiert, 25 Minuten zum Rückfluss erhitzt und auf ein Gemisch von 100 ml Eis und 30 ml konzentrierter Natronlauge gegossen. Man saugt ab, wäscht zweimal mit eiskaltem Wasser nach, trocknet an der Luft und kristallisiert aus Methanol/Wasser (20 ml + 100 ml) um. Man erhält das p-Aminobutyrophenon vom Smp. 93—94°.

8,15 g p-Aminobutyrophenon werden in 300 ml Methylenchlorid gelöst und unter Stickstoff auf −30° abgekühlt. Dann tropft man 5,96 g tert.-Butylhypochlorit hinzu und kühlt dabei langsam auf −65° ab. Man lässt 10 Minuten bei −65° nachrühren, tropft 7,37 g Methylthio-essigsäureäthylester hinzu, belässt 1 Stunde bei −65°, fügt 5,55 g Triäthylamin hinzu und lässt langsam auf Raumtemperatur erwärmen. Dann fügt man 100 ml Wasser hinzu, trennt die organische Schicht ab, wäscht mit 100 ml Wasser nach, extrahiert die Wasserphasen zweimal mit Methylenchlorid, vereinigt die organischen Phasen, trocknet über Magnesiumsulfat, filtriert und dampft unter vermindertem Druck zur Trockne ein. Der Eindampfrückstand wird in 200 ml Diäthyläther gelöst, mit 25 ml 2n-Salzsäure versetzt und über Nacht bei Raumtemperatur gerührt. Dann verdünnt man mit 50 ml Wasser, trennt die organische Phase ab, wäscht die Wasserphase mit 100 ml Diäthyläther, vereinigt die organischen Phasen, wäscht mit einem Gemisch von 50 ml Wasser und 10 ml 2n-Salzsäure, trocknet über Magnesiumsulfat, dampft zur Trockne ein, schlämmt mit Diäthyläther an, saugt ab und trocknet unter vermindertem Druck. Man erhält 5-Butyryl-3-methylthio-oxindol von Smp. 133—136°.

1,50 g 5-Butyryl-3-methylthio-oxindol in 50 ml Tetrahydrofuran werden mit 1,10 g Kaliumhydroxid versetzt und 3 Stunden unter Luftzutritt intensiv gerührt. Man säuert mit 2n-Salzsäure auf pH = 1 an, destilliert das Tetrahydrofuran unter vermindertem Druck ab, versetzt mit 10 ml 2n-Natronlauge, fügt unter Rühren 0,5 ml 30%-iges Wasserstoffperoxid hinzu, lässt 15 Minuten bei Raumtemperatur rühren, säuert mit 2n-Salzsäure auf pH = 1 an, saugt ab und lässt an der Luft trocknen. Das Rohprodukt wird in wässriger Kaliumcarbonatlösung gelöst, mit Aktivkohle geklärt, abfiltriert, erneut mit 2n-Salzsäure auf pH = 1 angesäuert, abgesaugt und unter vermindertem Druck getrocknet. Man erhält die 5-Butyrylanthranilsäure (2-Amino-5-butyryl-benzoesäure) vom Smp. 195—197°.

In eine Lösung von 4,4 g derselben in 60 ml Dioxan und 20 ml 2n-Salzsäure wird unter Aussenkühlung (stark exotherme Reaktion!) gelinde Phosgen eingeleitet, bis die exotherme Reaktion abgeklungen ist (ca. 90 Minuten). Man saugt ab, wäscht je zweimal mit Wasser und Diäthyläther und trocknet unter vermindertem Druck. Man erhält 5-Butyryl-isatosäureanhydrid vom Smp. 218—220°.

4,00 g desselben werden in 35 ml Hexamethylphosphorsäuretriamid gelöst, auf 5° gekühlt und unter Stickstoff mit Natriumhydrid (0,864 g der 50%-igen Suspension in Mineralöl, mit Hexan entölt) versetzt. Man lässt auf Raumtemperatur erwärmen, fügt 2,8 g Aethyljodid hinzu, lässt 1 Stunde zunächst bei Raumtemperatur, dann bei 40° nachrühren, giesst auf ein Gemisch von 300 ml Eiswasser und 20 ml 2n-Salzsäure, saugt ab, wäscht mit Wasser und lässt an der Luft trocknen. Man erhält das N-Aethyl-5-butyryl-isatosäureanhydrid vom Smp. 130—132°.

Beispiel 4

3,8 g Malonsäure-di-tert.-butylester werden in 40 ml Dimethylacetamid gelöst und durch Behandeln mit Natriumhydrid (0,843 g der 50%-igen Suspension in Mineralöl, mit Hexan entölt), in das Natriumsalz überführt. Man erwärmt auf 105° und lässt innerhalb 1 Stunde eine Lösung von N-Aethyl-5-butyryl-4-methyl-isatosäureanhydrid in 30 ml Dimethylacetamid versetzt. Man lässt 5 Stunden bei 120° rühren, destilliert das Dimethylacetamid unter vermindertem Druck weitgehend ab, versetzt mit 100 ml Eiswasser und giesst auf ein Gemisch von 10 ml 2n-Salzsäure und 200 ml Eiswasser. Der kristalline Niederschlag wird abgesaugt, zweimal mit kaltem Wasser gewaschen und in Methylenchlorid aufgenommen. Die organische Phase wird abgetrennt, über Magnesiumsulfat getrocknet, filtriert und eingedampft. Das Rohprodukt wird in 20 ml eines Gemisches von 3 Teilen Toluol und 1 Teil Essigester gelöst und an 60 g Kieselgel unter

Verwendung des obigen Lösungsmittelgemisches als Laufmittel chromatographiert. Man fängt Fraktionen von je 30 ml auf. Die nach Ausweis des Dünnschichtchromatogramms das gewünschte Produkt enthaltenden Fraktionen 3—9 werden vereinigt, eingedampft und aus 15 ml Methanol umkristallisiert. Man erhält den 1-Aethyl-6-butyryl-4-hydroxy-7-methyl-carbostiryl-3-carbonsäure-tert.-butylester vom Smp. 124—126°.

Beispiel 5

7,13 g 1-Aethyl-6-butyryl-4-hydroxy-7-methyl-carbostiryl-3-carbonsäure-tert.-butylester werden in 100 ml Diäthyläther suspendiert und unter Rühren vorsichtig mit 2,0 ml 70%-iger Perchlorsäure versetzt. Man lässt 10 Minuten bei 30° rühren, kühlt mittels eines Eisbades ab, saugt ab und wäscht zweimal mit wenig Diäthyläther und anschliessend mit Hexan nach und lässt bei Raumtemperatur trocknen. Man erhält so die 1-Aethyl-6-butyryl-4-hydroxy-7-methylcarbostiryl-3-carbonsäure von Smp. 157—158°.

Beispiel 6

3,64 g Malonsäurediäthylester werden in 50 ml Dimethylacetamid gelöst und in der Kälte unter Stickstoff mit Natriumhydrid (1,09 g der 50%-igen Suspension in Mineralöl mit Hexan entölt) in das Natriumsalz überführt. Dann erwärmt man auf 105°, tropft innerhalb 1 Stunde 5,95 g N-Allyl-5-butyryl-4-methyl-isatosäureanhydrid in 30 ml Dimethylacetamid hinzu, lässt 9 Stunden bei 120° und über Nacht bei Raumtemperatur rühren. Dann wird das Dimethylacetamid unter vermindertem Druck weitgehend abdestilliert. Man fügt unter Rphren etwa 100 g Eis und 20 ml 2n-Salzsäure hinzu, saugt die ausgefallenen Kristalle ab, wäscht mit kaltem Diäthyläther nach, trocknet unter vermindertem Druck und kristallisiert das etwa 30 ml Methanol um. Man erhält den 1-Allyl-6-butyryl-4-hydroxy-7-methyl-carbostiryl-3-carbonsäureäthylester vom Smp. 112—114°.

Das Ausgangsprodukt kann durch Umsetzung von 5-Butyryl-4-methyl-isatosäureanhydrid mit Allylbromid in analoger Weise wie in Beispiel 1 beschrieben hergestellt werden.

Beispiel 7

3,95 g Malonsäurediäthylester werden in 50 ml Dimethylacetamid gelöst und mit Natriumhydrid (1,07 g der 50%-igen Suspension in Mineralöl, mit Hexan entölt) in das Natriumsalz überführt. Man erwärmt auf 105°, tropft innerhalb von 90 Minuten 6,20 g N-butyl-5-butyryl-4-methyl-isatosäureanhydrid in 30 ml Dimethylacetamid hinzu und lässt 10 Stunden bei 120° und über Nacht bei Raumtemperatur rühren. Dann destilliert man das Dimethylacetamid ab, versetzt mit 200 ml Eiswasser und 20 ml 2n-Salzsäure, lässt einige Zeit intensiv rühren, saugt ab, wäscht zweimal mit Eiswasser nach und trocknet an der Luft. Das Rohprodukt wird an 60 g Kieselgel mit Toluol/Essigester (9:1) als Elutionsmittel chromatographisch gereinigt. Man dampft ein, digeriert mit 30 ml Diäthyläther und anschliessend mit 50 ml Hexan, saugt ab, wäscht mit Hexan nach und trocknet unter vermindertem Druck. Man erhält den 1-Butyl-6-butyryl-4-hydroxy-7-methylcarbostiryl-3-carbonsäureäthylester von Smp. 104—105°.

Das Ausgangsmaterial kann durch Umsetzung von 5-Butyryl-4-methyl-isatosäureanhydrid mit Butyljodid, wie in Beispiel 1 beschrieben, hergestellt werden.

Beispiel 8

2,98 g Malonsäurediäthylester werden in 50 ml Dimethylacetamid gelöst und mit Natriumhydrid (0,895 g der 50%-igen Suspension in Mineralöl, mit Hexan entölt) in das Natriumsalz überführt. Man erwärmt auf 105°, tropft innerhalb 1 Stunde 4,9 g 5-Butyryl-4-methyl-N-propyl-isatosäureanhydrid in 30 ml Dimethylacetamid hinzu und erwärmt 10 Stunden auf 120° und über Nacht bei Raumtemperatur rühren. Dann destilliert man das Dimethylacetamid unter vermindertem Druck ab, versetzt mit 200 g Eis und 20 ml 2n-Salzsäure und lässt kurze Zeit intensiv rühren. Der kristalline Niederschlag wird abgesaugt, zweimal mit Eiswasser gewaschen, getrocknet mit Diäthyläther angeschlämmt, erneut abgesaugt und mit Diäthyläther gewaschen. Das Rohprodukt wird an 60 g Kieselgel mit Toluol als Laufmittel chromatographisch gereinigt. Das Eluat wird unter vermindertem Druck eingedampft, mit Diäthyläther angeschlämmt, abgesaugt, mit kaltem Diäthyläther gewaschen und getrocknet. Man erhält den 6-Butyryl-4-hydroxy-7-methyl-1-propyl-carbostiryl-3-carbonsäureäthylester von Smp. 116—117°.

Das Ausgangsmaterial erhält man durch N-Propylierung von 5-Butyryl-4-methyl-isatosäureanhydrid analog in Beispiel 1 beschrieben.

Beispiel 9

Eine Lösung von 6,1 g Malonsäurediäthylester in 210 ml wasserfreiem Dimethylformamid wird unter Rühren und Einleiten von Stickstoffgas bei Raumtemperatur portionsweise mit 2,6 g Natriumhydrid-Mineralöl-Dispersion (60%-ig) versetzt. Die Mischung wird 30 Minuten bei Raumtemperatur und 30 Minuten bei 120° gerührt, auf Raumtemperatur abgekühlt und mit einer Lösung von 14,0 g N-Allyl-5-methoxycarbonyl-isatosäureanhydrid in 140 ml wasserfreiem Dimethylformamid versetzt. Anschliessend wird das Reaktionsgemisch 4 Stunden bei 120° gerührt, abgekühlt und unter 0,13 mbar bei 60° zur Trockene eingedampft. Der Rückstand wird mit 350 ml Wasser versetzt und zweimal mit je 120 ml Methylenchlorid extrahiert. Die wässrige Phase wird bei 0° mit 2n-Salzsäure angesäuert. Das ausgeschiedene Oel wird dreimal mit je 170 ml Methylenchlorid extrahiert. Die vereinigten organischen Phasen werden mit 50 ml

Wasser gewaschen, über Magnesiumsulfat getrocknet und unter vermindertem Druck zur Trockene eingedampft. Der Rückstand wird alsdann aus Methylenchlorid-Petroläther kristallisiert. Man erhält den 1-Allyl-6-methoxycarbonyl-4-hydroxy-carbostiryl-3-carbonsäureäthylester von Smp. 123—125°.

Analog kann man ausgehend von N-Aethyl- bzw. N-Propargyl-5-methoxycarbonyl-isatosäureanhydrid und Malonsäurediäthylester auch den 1-Aethyl-6-methoxycarbonyl-4-hydroxycarbostiryl-3-carbonsäureäthylester, Smp. 178—180° (aus Methylenchlorid/Petroläther) sowie den 4-Hydroxy-6-methoxycarbonyl-1-propargyl-carbostiryl-3-carbonsäureäthylester herstellen.

Das Ausgangsmaterial kann z.B. folgendermassen hergestellt werden:

Eine Lösung von 34,5 g 5-Methoxycarbonyl-anthranilsäure in 200 ml Dioxan wird unter Rühren mit 100 ml 2n-Salzsäure versetzt. In die Suspension wird bei 40° unter Rühren Phosgengas eingeleitet, wobei zuerst eine Lösung entsteht und sich anschliessend Kristalle ausscheiden. Nach zweistündigem Einleiten von Phosgen wird die Mischung auf 5° abgekühlt und filtriert. Die Kristalle werden mit 50 ml Wasser gewaschen, unter vermindertem Druck getrocknet, in 200 ml Aether aufgeschlämmt, filtriert und bei 50° unter 0,13 mbar 15 Minuten lang getrocknet. Man erhält das 5-Methoxycarbonyl-isatosäureanhydrid von Smp. 256—257°.

Eine Suspension von 4,33 g Natriumhydrid-Mineralöldispersion (60%-ig) in 180 ml wasserfreiem Dimethylformamid wird unter Rühren und Einleiten von Stickstoff tropfenweise mit einer Lösung von 20,0 g 5-Methoxycarbonyl-isatosäureanhydrid in 630 ml wasserfreiem Dimethylformamid versetzt. Die Mischung wird 15 Minuten bei Raumtemperatur gerührt und mit einer Lösung von 23,2 g Allylbromid in 130 ml wasserfreiem Dimethylformamid versetzt. Man rührt das Reaktionsgemisch 20 Stunden bei Raumtemperatur, engt unter 0,13 mbar bei 50° auf 300 ml ein und giesst den Rückstand auf 2000 ml Eiswasser. Die ausgeschiedenen Kristalle werden dreimal mit je 500 ml Diäthyläther extrahiert. Die vereinigten Aetherextrakte werden mit 200 ml Wasser gewaschen, über Magnesiumsulfat getrocknet und unter vermindertem Druck zur Trockene eingedampft. Den Rückstand kristallisiert man zunächst aus Diäthyläther/Petroläther und dann aus Tetrahydrofuran/n-Hexan. Das N-Allyl-5-methoxycarbonyl-isatosäureanhydrid schmilzt bei 116—118°.

Analog kann hergestellt werden: N-Aethyl-5-methoxycarbonyl-isatosäureanhydrid, Smp. 145—147° (aus Tetrahydrofuran/n-Hexan) bzw. 5-Methoxycarbonyl-N-propargyl-isatosäureanhydrid (durch Umsetzung mit Aethylbromid bzw. Propargylbromid).

### Beispiel 10

Eine Lösung von 3,3 g 1-Allyl-6-methoxycarbonyl-4-hydroxy-carbostiryl-3-carbonsäureäthylester in 20,0 ml n-Natronlauge wird 15 Stunden bei Raumtemperatur gerührt, mit 10,0 ml Diäthyläther extrahiert und mit Salzsäure angesäuert. Die ausgeschiedenen Kristalle werden abfiltriert und aus Methanol umkristallisiert. Der erhaltene 1-Allyl-6-carboxy-4-hydroxy-carbostiryl-3-carbonsäureäthylester schmilzt höher als 280°.

Analog kann man ausgehend von 1-Aethyl-6-methoxycarbonyl-4-hydroxy-carbostiryl-3-carbonsäureäthylester 1-Aethyl-6-carboxy-4-hydroxy-carbostiryl-3-carbonsäureäthylester, Smp. >280° (aus Methanol), herstellen.

### Beispiel 11

1,4 g 1-Aethyl-6-butyryl-4-hydroxy-7-methyl-carbostiryl-3-carbonsäurenitril werden in 50 ml 96%-igem Aethanol und 10 g wasserfreiem Ammonchlorid über Nacht zum Rückfluss erhitzt. Man filtriert ab, dampft unter vermindertem Druck zur Trockene ein und kristallisiert aus 50%-igem Methanol um. Man erhält 1-Aethyl-6-butyryl-4-hydroxy-7-methyl-carbostiryl-3-carbonsäureäthylester von Smp. 125—126°.

Das Ausgangsmaterial kann durch Umsetzung von Cyanessigsäureäthylester/Natriumhydrid mit N-Aethyl-5-butyryl-4-methyl-isatosäureanhydrid in analoger Weise wie in Beispiel 1 beschrieben hergestellt werden.

### Beispiel 12

In analoger Weise wie in den Beispielen 1—11 beschrieben, kann man ferner herstellen:

6-Aethoxycarbonyl-1-äthyl-4-hydroxy-carbostiryl-3-carbonsäureäthylester,

1-Aethyl-6-pivaloyloxymethoxycarbonyl-4-hydroxy-carbostiryl-3-carbonsäureäthylester,

1-Aethyl-6-[2-(2-hydroxyäthoxy)-äthoxycarbonyl]-4-hydroxy-carbostiryl-3-carbonsäureäthylester,

1-Aethyl-6-{2-[2-(2-methoxyäthoxy)-äthoxy]-äthoxycarbonyl}-4-hydroxy-carbostiryl-3-carbonsäureäthylester,

1-Aethyl-6-{2-[2-(2-hydroxyäthoxy)-äthoxy]-äthoxycarbonyl}-4-hydroxy-carbostiryl-3-carbonsäureäthylester und

6-Aethoxycarbonyl-1-äthyl-4-hydroxy-carbostiryl-3-carbonsäure-tert.-butylester.

Die als Ausgangsmaterialien zu verwendenden höheren Ester von N-Aethyl-5-carboxy-isatosäureanhydrid können ausgehend von dem Methylester durch Umesterung oder Verseifung mittels Natronlauge zur Säure sowie Veresterung derselben mit dem betreffenden Alkohol in Gegenwart von Dicyclohexylcarbodiimid und 4-Dimethylaminopyridin in Tetrahydrofuran als Lösungsmittel erhalten werden.

Beispiel 13

Zu einer Lösung von 1,15 g Natrium in 30 ml absolutem Aethanol werden bei Raumtemperatur 19,6 g N-Aethyl-5-butyryl-4-methyl-N-(1'-oxo-2'-carbäthoxy-äthyl)-anthranilsäure-äthylester in 60 ml absolutem Aethanol getropft. Die gelbe Lösung wird 3 Stunden am Rückfluss gekocht. Die erhaltene Suspension wird anschliessend im Vakuum vom Aethanol befreit. Der Rückstand wird in Eiswasser gelöst und mit Salzsäure angesäuert. Das ausgeschiedene Oel wird mit Methylenchlorid extrahiert und die Methylenchloridlösung mit Wasser gewaschen, über Natriumsulfat getrocknet, filtriert und im Vakuum zur Trockne eingedampft. Man erhält ein Oel, das man aus Methanol zur Kristallisation bringt. Man erhält so den 1-Aethyl-6-butyryl-4-hydroxy-7-methyl-carbostyril-3-carbonsäureäthylester von Smp. 124—126°.

Der als Ausgangsmaterial benötigte N-Aethyl-5-butyryl-4methyl-N-(1'-oxo-2'-carbäthoxy-äthyl)-anthranilsäureäthylester kann auf folgende Weise erhalten werden:

2,3 g Natrium werden in 250 ml Aethanol gelöst. Zu dieser Lösung werden 27,5 g N-Aethyl-5-butyryl-4-methylisatosäureanhydrid in 300 ml Aethanol suspendiert, zugegeben. Man kocht 1 Stunde am Rückfluss. Die Lösung wird zur Trockene eingedampft und mit Eiswasser versetzt und vorsichtigerweise mit 2N HCl angesäuert. Dann wird mit Methylenchlorid ausgezogen, über Magnesiumsulfat getrocknet und eingedampft. Der so erhaltene N-Aethyl-5-butyryl-4-methyl-anthranilsäureäthylester wird ohne Reinigung folgendermassen weiterumgesetzt:

13,86 g des obigen Antranilsäureäthylester werden in 140 ml absoluten Toluol gelöst und mit 6,45 g Aethyldisiopropylamin versetzt. Zu dieser Lösung wird innerhalb 30 Minuten eine Lösung von 7,6 g Chlorcarbonylessigsäureäthylester in 75 ml absoluten Toluol bei Raumtemperatur getropft. Nach 10-stündigem Rühren bei Raumtemperatur tropft man nochmals 7,6 g Chlorcarbonylessigsäureäthylester in Toluol innerhalb 15 Minuten hinzu und lässt weitere 15 Stunden bei Raumtemperatur rühren. Das Reaktionsgemisch wird mit Wasser, Natriumbicarbonatlösung und Wasser gewaschen, über wasserfreiem Natriumsulfat getrocknet, filtriert und im Vakuum zur Trockne eingedampft. Man erhält so N-Aethyl-5-butyryl-4-methyl-N-(1'-oxo-2'-carbäthoxyäthyl)-anthranilsäureäthylester als hellgelbes Oel, das ohne weitere Reinigung weiterverarbeitet wird.

Beispiel 14

10,1 g 1-Aethyl-4-hydroxy-6-carboxy-carbostiryl-3-carbonsäureäthylester werden in 100 ml Pyridin gelöst und bei Raumtemperatur mit 2,4 ml Thionylchlorid 2 Stunden gerührt. Dann wird die dunkle Lösung zur Trockene eingedampft, zweimal mit je 30 ml Toluol versetzt und nochmals eingedampft. Das so erhaltene Säurechlorid wird in 100 ml absolutem Tetrahydrofuran gelöst, die Lösung abfiltriert und bei −20° zu einer Lösung von Propyllithium in Diäthyläther zugetropft. Man rührt 30 Minuten bei −20° und lässt 2 Stunden bei Raumtemperatur nachrühren. Unter Aussenkühlung tropft man dann eine gesättigte wässrige Ammonchloridlösung hinzu, trennt die organische Schicht ab und trocknet diese über Natriumsulfat. Die abfiltrierte Lösung wird zur Trockene eingedampft. Das Rohmaterial wird an 100 g Kieselgel chromatographiert. Mit Toluol/Essigester 1:1 wird der 1-Aethyl-6-butyryl-4-hydroxy-carbostiryl-3-carbonsäureäthylester von Smp. 163—165° eluiert.

Beispiel 15

4,2 g 1-Aethyl-6-chlorcarbonyl-4-hydroxy-7-methylcarbostiryl-3-carbonsäureäthylester werden in 60 ml Aceton gelöst und unter Rühren mit einer Lösung von 9,9 g Bis(triphenylphosphin)-kupferboranat der Formel $[(C_6H_5)_3P)]_2CuBH_4$ in 90 ml Aceton versetzt. Man lässt 2 Stunden bei Raumtemperatur rühren, filtriert ab und dampft das Filtrat zur Trockne ein. Das Rohprodukt wird in Essigester gelöst, über Kieselgel filtriert und erneut zur Trockne eingedampft. Man erhält den 1-Aethyl-6-formyl-4-hydroxy-7-methyl-carbostiryl-3-carbonsäureäthylester in Form eines hellgelben, viskosen Oels, IR-Spektrum: $V_{c=o} = 1711\ cm^{-1}$ (Methylenchlorid).

Beispiel 16

2,22 g Chromtrioxid werden in 120 ml Methylenchlorid und 3,5 ml Pyridin gelöst. Man rührt 30 Minuten und gibt dann eine Lösung von 1,12 g 1-Aethyl-6-(1-hydroxybutyl)-4-hydroxy-7-methyl-carbostiryl-3-carbonsäureäthylester in 10 ml Methylenchlorid hinzu und lässt unter Stickstoff 30 Minuten bei Raumtemperatur rühren. Man giesst in 600 ml Diäthyläther, filtriert vom Unlöslichen ab, wäscht mit Diäthyläther nach, dampft zur Trockne ein und kristallisiert aus Methanol um. Man erhält den 1-Aethyl-6-butyryl-7-methyl-4-hydroxy-carbostiryl-3-carbonsäureäthylester von Smp. 124—126°.

Das Ausgangsmaterial kann beispielsweise folgendermassen hergestellt werden:

3,6 g 1-Aethyl-6-formyl-4-hydroxy-7-methylcarbostiryl-3-carbonsäureäthylester werden in 50 ml Tetrahydrofuran gelöst, auf −20° abgekühlt und zu einer auf −20° gekühlten Lösung von Propyllithium in Diäthyläther (bereitet aus 1,47 g Propylbromid und 0,18 g Lithium) zugetropft. Man rührt 1 Stunde bei −20° und 2 Stunden bei Raumtemperatur nach, giesst in eine eiskalte gesättigte Aminochloridlösung, trennt die organische Phase ab und schüttelt die Wasserphase mit Methylenchlorid aus. Die organischen Phasen werden über Magnesiumsulfat getrocknet und unter vermindertem Druck zur Trockne eingedampft. Das Rohprodukt wird aus Methanol/Diäthyläther umkristallisiert. Man erhält den 1-Aethyl-4-hydroxy-6-(1-hydroxybutyl)-7-methyl-carbostiryl-3-carbonsäureäthylester von Smp. 162—163°.

Beispiel 17

1,0 g 1-Aethyl-6-butyryl-4-hydroxy-7-methylcarbostiryl-3-carbonsäurenitril werden in 30 ml 90%-iger Schwefelsäure unter Rühren gelöst. Man erwärmt unter Rühren 30 Minuten auf 75°, gibt 200 g Eis hinzu, saugt ab, wäscht mit Wasser und lässt an der Luft trocknen. Das Rohprodukt wird in 100 ml Methanol suspendiert, zum Sieden erhitzt, langsam auf 0° abgekühlt, abgesaugt, mit Methanol gewaschen und unter vermindertem Druck getrocknet. Man erhält das 1-Aethyl-6-butyryl-4-hydroxy-7-methyl-carbostiryl-3-carboxamid vom Smp. 233—235°.

Das Ausgangsmaterial kann z.B. folgendermassen hergestellt werden:

Zu einer auf 10° gekühlten Lösung von 9,04 g Cyanessigsäureäthylester in 200 ml Dimethylacetamid gibt man unter Stickstoff portionsweise 3,84 50%-ige Natriumhydridsuspension (in Mineralöl, mit Hexan entölt), erwärmt nach vollständiger Auflösung auf 105°, tropft innerhalb von 90 Minuten 20 g N-Aethyl-5-butyryl-4-methyl-isatosäureanhydrid in 100 ml Dimethylacetamid hinzu, erhitzt 8 Stunden auf 120°, lässt über Nacht bei Raumtemperatur nachrühren, giesst auf ein Gemisch von 2000 ml Wasser und 200 ml 2n-Salzsäure und schüttelt zweimal mit je 500 ml Essigester aus. Die organischen Phasen werden vereinigt, zweimal mit Wasser gewaschen, über Magnesiumsulfat getrocknet und unter vermindertem Druck zur Trockne eingedampft. Der Rückstand wird durch Digerieren mit 300 ml Diäthyläther zur Kristallisation gebracht. Man saugt ab, wäscht mit Diäthyläther nach, trocknet unter vermindertem Druck, suspensiert in 200 ml Essigester, erhitzt zum Sieden, lässt unter Rühren langsam abkühlen, saugt ab, wäscht zweimal mit wenig Essigester nach und trocknet unter vermindertem Druck bis zur Gewichtskonstanz. Man erhält das 1-Aethyl-6-butyryl-4-hydroxy-7-methyl-carbostiryl-3-carbonsäurenitril von Smp. 244—245°.

Beispiel 18

Eine zur Inhalation geeignete, 2%-ige wässrige Lösung eines Salzes von 1-Aethyl-6-butyryl-4-hydroxy-7-methyl-carbostiryl-3-carbonsäure (Wirkstoff), kann wie folgt hergestellt werden.

Zusammensetzung (für 100 ml)

| | |
|---|---|
| Wirkstoff | 2,000 g |
| Dinatriumsalz der Aethylendiamintetraessigsäure (Stabilisator) | 0,010 g |
| Benzalkoniumchlorid (Konservierungsmittel) | 0,010 g |
| Wasser, destilliert | ad 100 ml |

Der Wirkstoff wird in frisch destilliertem Wasser gelöst und die Lösung mit dem Dinatriumsalz der Säure und dem Benzalkoniumchlorid, das ist ein Gemisch von Alkylmethyl-benzyl-ammoniumchloriden, worin Alkyl von 8—18 Kohlenstoffatome enthält, versetzt. Nach vollständiger Auflösung der Komponenten wird die erhaltene Lösung mit Wasser auf ein Volumen von 100 ml gebracht, abgefüllt und gasdicht verschlossen.

Beispiel 19

Zur Insufflation geeignete, 0,025 g 1-Aethyl-6-butyryl-4-hydroxy-7-methyl-carbostiryl-3-carbonsäure-äthylester (Wirkstoff) enthaltende Kapseln können wie folgt hergestellt werden:

Zusammensetzung (für 1000 Kapseln)

| | |
|---|---|
| 1-Aethyl-6-butyryl-4-hydroxy-7-methyl-carbostiryl-3-carbonsäureäthylester (Wirkstoff) | 25,00 g |
| Laktose, gemahlen | 25,00 g |

Der Wirkstoff und die Laktose (feinst gemahlen) werden gut miteinander vermischt. Das erhaltene Pulver wird gesiebt und in Portionen zu je 0,05 g in Gelatinekapseln abgefüllt.

Beispiel 20

Tabletten, enthalten 100 mg 1-Aethyl-6-butyryl-4-hydroxy-7-methyl-carbostiryl-3-carbonsäureäthylester als Wirkstoff, können beispielsweise in folgender Zusammensetzung hergestellt werden.

| Zusammensetzung | pro Tablette |
|---|---|
| Wirkstoff | 100 mg |
| Milchzucker | 50 mg |
| Weizenstärke | 73 mg |
| Koloidale Kieselsäure | 13 mg |
| Talk | 12 mg |
| Magnesiumstearat | 2 mg |
| | 250 mg |

Herstellung

Der Wirkstoff wird mit dem Milchzucker, einem Teil der Weizenstärke und mit koloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist. Diese Masse wird durch ein Sieb von etwa 1 mm Maschenweite getrieben, getrocknet, und das trockene Granulat nochmals durch ein Sieb getrieben. Dann werden die restliche Weizenstärke, der Talk und das Magnesiumstearat zugemischt. Die erhaltene Tablettiermischung wird zu Tabletten von je 250 mg mit Bruchkerbe(n) verpresst.

Beispiel 21

In analoger Weise wie in den Beispielen 18 bis 20 beschrieben, können auch pharmazeutische Präparate, enthaltend eine andere Verbindung gemäss einem der Beispiele 1—17, hergestellt werden.

**Patentansprüche für die Vertragsstaaten: BE, CH, DE, FR, IT, LI, LU, NL, SE**

1. Ein Chinolinonderivat der Formel

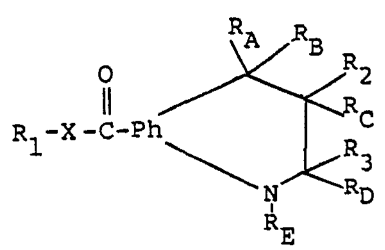

(I),

worin $R_1$ gegebenenfalls durch Hydroxy, Niederalkoxy, Hydroxyniederalkoxy, Niederalkoxyniederalkoxy, Hydroxyniederalkoxyniederalkoxy, Niederalkoxyniederalkoxyniederalkoxy, Niederalkylthio, Niederalkansulfinyl oder Niederalkansulfonyl substituiertes Niederalkyl, Niederalkanoyloxymethyl, Niederalkenyl oder Cycloalkyl, gegebenenfalls im Phenylteil durch Niederalkyl, Niederalkoxy und/oder Halogen substituiertes Phenyl oder Phenylniederalkyl, Furyl, Thienyl oder Pyridyl oder Furylniederalkyl, Thienylniederalkyl, Pyridylniederalkyl oder Wasserstoff bedeutet, X eine direkte Bindung oder Oxy darstellt, Ph gegebenenfalls zusätzlich durch Niederalkyl, Niederalkoxy, Hydroxy und/oder Halogen substituiertes 1,2-Phenylen bedeutet, $R_2$ Carboxy, Niederalkoxycarbonyl, Hydroxyniederalkoxycarbonyl, Niederalkoxyniederalkoxycarbonyl, Diniederalkylaminoniederalkoxy- oder 5- bis 7-gliedriges Niederalkylenamino- bzw. Aza-, Oxa- oder Thianiederalkylenaminoniederalkoxycarbonyl oder amidiertes Carboxy bedeutet, das als Aminogruppe Amino, Hydroxyamino, Niederalkylamino, Diniederalkylamino oder 5- bis 7-gliedriges Niederalkylen- bzw. Aza-, Oxa-, Thianiederalkylenamino aufweist, und worin entweder $R_A$ und $R_B$ gemeinsam Oxo darstellen, $R_C$ und $R_D$ gemeinsam eine zusätzliche Bindung bedeuten und $R_3$ Hydroxy ist bzw. $R_C$ Wasserstoff ist und $R_3$ und $R_D$ gemeinsam Oxo darstellen und $R_E$ Wasserstoff, Niederalkyl, Hydroxyniederalkyl, Niederalkoxyniederalkyl, Diniederalkylaminoniederalkyl, 5- bis 7-gliedriges Niederalkylenamino- bzw. Aza-, Oxa- oder Thianiederalkylenaminoniederalkyl, Niederalkenyl, Niederalkinyl, Cycloalkyl mit 3 bis 8, vor allem 5 bis 7, Ringgliedern oder gegebenenfalls durch Niederalkyl, Niederalkoxy und/oder Halogen substituiertes Phenylniederalkyl, Furylniederalkyl, Thienylniederalkyl oder Pyridylniederalkyl darstellt, oder $R_A$ Hydroxy oder Niederalkoxy bedeutet, $R_B$ gemeinsam mit $R_C$ sowie $R_D$ gemeinsam mit $R_E$ jeweils eine zusätzliche Bindung darstellen und $R_3$ Hydroxy ist bzw. $R_B$ und $R_C$ gemeinsam eine zusätzliche Bindung und $R_3$ und $R_D$ gemeinsam Oxo darstellen und $R_E$ Wasserstoff, Niederalkyl, Hydroxyniederalkyl,

Niederalkoxyniederalkyl, Diniederalkylaminoniederalkyl, 5- bis 7-gliedriges Niederalkylenamino- bzw. Aza-, Oxa- oder Thianiederalkylenaminoniederalkyl, Niederalkenyl, Niederalkinyl, Cycloalkyl mit 3—8 Ringgliedern oder gegebenenfalls durch Niederalkyl, Niederalkoxy und/oder Halogen substituiertes Phenylniederalkyl, Furylniederalkyl, Thienylniederalkyl oder Pyridylniederalkyl darstellt, wobei "niedere" Reste jeweils bis und mit 7 Kohlenstoffatome auf weisen, oder ein Salz davon.

2. Eine Verbindung gemäss Anspruch 1, worin X eine direkte Bindung darstellt und $R_1$, $R_2$, $R_3$, $R_A$, $R_B$, $R_C$, $R_D$ und $R_E$ die in Anspruch 1 angegebenen Bedeutungen haben, $R_1$ jedoch von Wasserstoff verschieden ist, oder X für Oxy steht, $R_1$ Niederalkyl oder gegebenenfalls durch Niederalkyl, Niederalkoxy, Hydroxy und/oder Halogen substituiertes Phenyl darstellt und $R_2$, $R_3$, $R_A$, $R_B$, $R_C$, $R_D$ und $R_E$ die in Anspruch 1 angegebene Bedeutungen haben.

3. Eine Verbindung gemäss Anspruch 1 der Formel

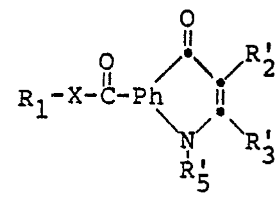

(Ia),

worin $R_1$ Niederalkylmit bis zu 7 Kohlenstoffatomen, Niederalkoxy-, Niederalkylthio-, Niederalkansulfinyl- oder Niederalkansulfonylniederalkyl mit jeweils bis zu 4 Kohlenstoffatomen in den Alkylteilen, Niederalkenyl mit bis zu 4 Kohlenstoffatomen, 3- bis 8-gliedriges Cycloalkyl, gegebenenfalls durch Niederalkyl mit bis zu 4 Kohlenstoffatomen, Niederalkoxy mit bis zu 4 Kohlenstoffatomen und/oder Halogen bis einschliesslich Atomnummer 35 substituiertes Phenyl bzw. Phenylniederalkyl mit bis zu 4 Kohlenstoffatomen im Alkylteil, Furyl, Tienyl oder Pyridyl oder Furyl-, Thienyl- oder Pyridyl-niederalkyl mit bis zu 4 Kohlenstoffatomen im Alkylteil und X eine direkte Bindung bedeutet oder $R_1$ Wasserstoff oder Niederalkyl mit bis zu 7 Kohlenstoffatomen und X Oxy darstellt, und worin Ph die Gruppe $R_1—X—C(=O)—$ aufweisendes, gegebenenfalls zusätzlich durch Niederalkyl mit bis zu 4 Kohlenstoffatomen, Niederalkoxy mit bis zu 4 Kohlenstoffatomen, Hydroxy oder Halogen bis einschliesslich Atomnummer 35 substituiertes 1,2-Phenylen darstellt, $R_2'$ für Carboxy, Niederalkoxycarbonyl mit bis zu 5 Kohlenstoffatomen, Hydroxyniederalkoxycarbony mit bis zu 5 Kohlenstoffatomen, Niederalkoxyniederalkoxycarbony mit bis zu 4 Kohlenstoffatomen in den Alkoxyteilen, Diniederalkylaminoniederalkoxycarbonyl mit jeweils bis zu 4 Kohlenstoffatomen im Alkyl- und Alkoxyteil, Carbamoyl, N-Hydroxycarbamoyl oder N-Niederalkyl- bzw. N,N-Diniederalkylcarbamoyl mit bis zu 4 Kohlenstoffatomen im Alkylteil steht, $R_3'$ Hydroxy darstellt und $R_5'$ Wasserstoff, Niederalkyl oder Niederalkenyl mit bis zu 4 Kohlenstoffatomen, gegebenenfalls durch Niederalkyl mit bis zu 4 Kohlenstoffatomen, Niederalkoxy mit bis zu 4 Kohlenstoffatomen und/oder Halogen bis und mit Atomnummer 35 substituiertes Phenyl-, Furyl-, Thienyl- oder Pyridylniederalkyl mit bis zu 4 Kohlenstoffatomen im Alkylteil, Niederalkoxyniederalkyl mit bis zu 4 Kohlenstoffatomen im Alkoxy- bzw. Alkylteil oder Diniederalkylaminoniederalkyl, worin Niederalkyl bis zu 4 Kohlenstoffatome aufweist, oder ein Tautomeres und/oder Salz davon.

4. Eine Verbindung gemäss Anspruch 1 der Formel

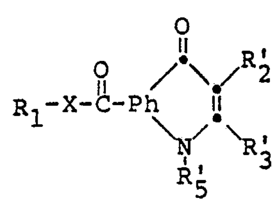

(Ia),

worin $R_1$ Wasserstoff, Niederalkyl mit bis zu 7 Kohlenstoffatomen, Niederalkoxy-, Niederalkylthio-, Niederalkansulfinyl- oder Niederalkanesufonylniederalkyl mit jeweils bis zu 4 Kohlenstoffatomen in den Alkylteilen, Niederalkenyl mit bis zu 4 Kohlenstoffatomen oder 3—8 gliedriges Cycloalkyl und X eine direkte Bindung oder $R_1$ Wasserstoff, Niederalkyl mit bis zu 7 Kohlenstoffatomen, Niederalkoxy-, Niederalkylthio-, Niederalkansulfonyl- oder Niederalkansulfinylniederalkyl mit jeweils bis zu 4 Kohlenstoffatomen in den Alkylteilen, Niederalkenyl mit bis zu 4 Kohlenstoffatomen oder 3- bis 8-gliedriges Cycloalkyl, Niederalkanoyloxymethyl mit bis zu 7 C-Atomen im Alkanoylteil oder ω-(ω-Hydroxyniederalkoxy)-niederalkyl, ω-(ω-Niederalkoxyniederalkoxy)-niederalkyl, ω-[ω-(ω-Hydroxyniederalkoxy)-niederalkoxy]-niederalkyl oder ω-[ω-(ω-Niederalkoxyniederalkoxy)-niederalkoxy]-niederalkyl mit jeweils 1 bis 4 Kohlenstoffatomen im Alkyl- und 2 bis 4 Kohlenstoffatomen im Alkylenteil und X Oxy bedeutet, Ph die Gruppe $R_1—X—C(=O)—$ aufweisendes, gegebenenfalls zusätzlich durch Niederalkyl mit bis zu 4 Kohlenstoffatomen, Niederalkoxy mit bis zu 4 Kohlenstoffatomen, Hydroxy oder Halogen bis einschliesslich Atomnummer 35 substituiertes 1,2-Phenylen darstellt, $R_2'$ für Carboxy, Niederalkoxycarbonyl mit bis zu 5 Kohlenstoffatomen, Niederalkoxyniederalkoxycarbonyl mit bis zu 4 Kohlenstoffatomen in den Alkoxyteilen, Carbamoyl, N-Hydroxycarbamoyl oder N-Niederalkyl- bzw. N,N-Diniederalkylcarbamoyl mit bis zu 4 Kohlenstoffatomen im Alkylteil steht, $R_3'$ Hydroxy darstellt und $R_5'$ Wasserstoff, Niederalkyl, Niederalkenyl oder Niederalkinyl mit bis zu 4 Kohlenstoffatomen bedeutet, Sowie Tautomere derselben, in denen die Hydroxygruppe $R_3'$ in der tautomeren Oxoform vorliegt, oder ein Salz einer salzbildenden Verbindungen der Formel Ia oder eines ihrer Tautomeren.

5. Eine Verbindung gemäss Anspruch 1 der Formel

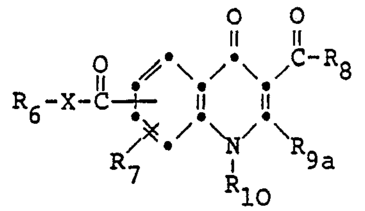

(II),

worin X eine direkte Bindung und $R_6$ Wasserstoff oder Niederalkyl mit bis und mit 7 Kohlenstoffatomen bedeutet oder X für Oxy und $R_6$ für Wasserstoff, Niederalkyl mit bis und mit 7 Kohlenstoffatomen, ω-Hydroxyniederalkyl mit 2 bis 4 Kohlenstoffatomen, Niederalkanoyloxymethyl mit bis und mit 7 C-Atomen im Alkanoylteil oder ω-(ω-Hydroxyniederalkoxy)-niederalkyl, ω-(ω-Niederalkoxyniederalkoxy)-niederalkyl, ω-[ω-(ω-Hydroxyniederalkoxy)-niederalkoxy]-niederalkyl oder ω-[ω-(ω-Niederalkoxyniederalkoxy)-niederalkoxy]-niederalkyl mit jeweils 1 bis 4 Kohlenstoffatomen im Alkyl- und 2 bis 4 Kohlenstoffatomen im Alkylenteil bedeutet, $R_7$ Wasserstoff, Niederalkyl mit bis und mit 4 Kohlenstoffatomen, Niederalkoxy mit bis und mit 4 Kohlenstoffatomen, Halogen bis Atomnummer 35 oder Hydroxy darstellt, $R_8$ Hydroxy oder Niederalkoxy mit bis zu 4 Kohlenstoffatomen bedeutet, $R_{9a}$ Hydroxy darstellt, und $R_{10}$ Niederalkyl, Niederalkenyl oder Niederalkinyl mit bis und mit zu 4 Kohlenstoffatomen ist, wobei eine 2-Hydroxy-4-oxo-1,4-dihydrochinolinverbindung der Formel II auch in der tautomeren 2-Oxo-4-hydroxy-1,2-dihydro- oder 2,4-Dioxo-1,2,3,4-tetrahydrochinolinform vorliegen kann, oder ein Salz davon.

6. Eine Verbindung gemäss Anspruch 5, worin X Oxy darstellt, die Gruppe $R_6$—O—C(=O)— in 6-Stellung gebunden ist, $R_6$ Wasserstoff, Niederalkyl mit bis zu 7 Kohlenstoffatomen, ω-Hydroxyniederalkyl mit 2 bis 4 Kohlenstoffatomen, Niederalkanoyloxymethyl mit bis und mit 7 C-Atomen im Alkylteil oder ω-(ω-Hydoxyniederalkoxy)-niederalkyl, ω-(ω-Niederalkoxy-niederalkoxy)-niederalkyl, ω-[ω-ω-[ω-(ω-Hydroxyniederalkoxy)-niederalkoxy]-niederalkyl oder ω-[ω-(ω-Niederalkoxyniederalkoxy)-niederalkoxy]-niederalkyl mit jeweils 1 bis 4 Kohlenstoffatomen im Alkyl- und 2 bis 4 Kohlenstoffatomen im Alkylenteil bedeutet, $R_7$ Wasserstoff darstellt und $R_{10}$ Niederalkenyl mit bis zu 4 Kohlenstoffatomen bedeutet, oder X eine direkte Bindung darstellt, $R_6$ Wasserstoff oder Niederalkyl mit bis zu 7 Kohlenstoffatomen bedeutet, $R_7$ Niederalkyl mit bis und mit 4 Kohlenstoffatomen darstellt, wobei die Gruppe $R_6$—C(=O)— die 6- und die Niederalkylgruppe $R_7$ die 7-Stellung einnimmt, und $R_{10}$ Niederalkyl mit bis und mit 4 Kohlenstoffatomen bedeutet, wobei $R_8$ und $R_{9a}$ jeweils in Anspruch 5 angegebenen Bedeutungen haben, bzw. das 2-Oxo-4-hydroxy-1,2,-dihydrochinolin- oder 2,4-Dioxo-1,2,3,4,-tetrahydrochinolin-Tautomere und/oder ein Salz davon.

7. Eine Verbindung gemäss Anspruch 5, worin X eine direkte Bindung darstellt, $R_6$ Niederalkyl mit bis zu 7 Kohlenstoffatomen bedeutet, $R_7$ Wasserstoff oder Niederalkyl mit bis und mit 4 Kohlenstoffatomen darstellt, wobei die Gruppe $R_6$—C(=O)— die 6- und eine Niederalkylgruppe $R_7$ die 7-Stellung einnimmt, $R_8$ und $R_{9a}$ Hydroxy darstellen und $R_{10}$ Niederalkyl oder Niederalkenyl mit bis und mit 4 Kohlenstoffatomen bedeutet, bzw. das 2-Oxo-4-hydroxy-1,2-dihydrochinolin- oder 2,4-Dioxo-1,2,3,4,-tetrahydrochinolin-Tautomere und/oder ein Salz davon.

8. Eine Verbindung gemäss Anspruch 5, worin X Oxy darstellt, $R_6$ Wasserstoff oder Niederalkyl mit bis zu 7 Kohlenstoffatomen bedeutet, $R_7$ Wasserstoff oder Niederalkyl mit bis und mit 4 Kohlenstoffatomen darstellt, wobei die Gruppe $R_6$—O—C(=O)— die 6- und eine Niederalkylgruppe $R_7$ die 7-Stellung einnimmt, $R_8$ und $R_{9a}$ die in Anspruch 5 angegebenen Bedeutungen hanen und $R_{10}$ Niederalkyl oder Niederalkenyl mit bis und mit 4 Kohlenstoffatomen bedeutet, bzw. das 2-Oxo-4-hydroxy-1,2-dihydrochinolin- oder 2,4-Dioxo-1,2,3,4,-tetrahydrochinolin-Tautomere und/oder ein Salz davon.

9. 6-Butyryl-4-hydroxy-1,7-dimethyl-carbostiryl-3-carbonsäureäthylester gemäss Anspruch 1.

10. 6-Butyryl-4-hydroxy-7-methyl-1-propyl-carbostiryl-3-carbonsäureäthylester gemäss Anspruch 1.

11. 1-Aethyl-6-butyryl-4-hydroxy-7-methyl-carbostiryl-3-carbonsäureäthylester gemäss Anspruch 1.

12. 1-Allyl-6-butyryl-4-hydroxy-7-methyl-carbostiryl-3-carbonsäureäthylester gemäss Anspruch 1.

13. 1-Aethyl-6-butyryl-4-hydroxy-carbostiryl-3-carbonsäureäthylester gemäss Anspruch 1.

14. 1-Butyl-6-butyryl-4-hydroxy-7-methyl-carbostiryl-3-carbonsäureäthylester gemäss Anspruch 1.

15. 1-Allyl-6-methoxycarbonyl-4-hydroxy-carbostiryl-3-carbonsäureäthylester gemäss Anspruch 1.

16. 1-Allyl-6-carboxy-4-hydroxy-carbostiryl-3-carbonsäureäthylester gemäss Anspruch 1 oder ein Salz davon.

17. 1-Aethyl-6-butyryl-4-hydroxy-7-methyl-carbostiryl-3-carbonsäure-tert.-butylester gemäss Anspruch 1.

18. 1-Aethyl-6-butyryl-4-hydroxy-7-methyl-carbostiryl-3-carbonsäure gemäss Anspruch 1 oder ein Salz davon.

19. 6-Methoxycarbonyl-1-propargyl-4-hydroxy-carbostiryl-3-carbonsäureäthylester gemäss Anspruch 1 oder ein Salz davon.

20. 1-Aethyl-6-butyryl-4-hydroxy-7-methyl-carbostiryl-3-carboxamid gemäß Anspruch 1.

21. 1-Aethyl-6-formyl-4-hydroxy-7-methyl-carbostiryl-3-carbonsäureäthylester gemäss Anspruch 1.

22. 1-Aethyl-6-methoxycarbonyl-4-hydroxy-carbostiryl-3-carbonsäureäthylester gemäss Anspruch 1.

23. 1-Aethyl-6-carboxy-4-hydoxy-carbostiryl-3-carbonsäureäthylester gemäss Anspruch 1 oder ein Salz davon.

24. Eine Verbindung gemäss einem der Ansprüche 1—23 zur Anwendung in einem Verfahren zur prophylaktischen und/oder therapeutischen Behandlung des tierischen oder menschlichen Körpers.

25. Verbindungen gemäss einem der Ansprüche 1—23 zur Anwendung gemäss Anspruch 24 als antiallergisches Mittel.

26. Pharmazeutische Präparate enthaltend eine Verbindung gemäss einem der Ansprüche 1, 4—6 und 13—23, gegebenenfalls in Form eines pharmazeutisch verwendbaren Salzes, neben üblichen pharmazeutischen Hilfs- und Trägerstoffen.

27. Pharmazeutische Präparate enthaltend eine Verbindung gemäss einem der Ansprüche 2, 3 und 7—12, gegebenenfalls in Form eines pharmazeutisch verwendbaren Salzes, neben üblichen pharmazeutischen Hilfds- und Trägerstoffen.

28. Verfahren zur Herstellung neuer Chinolinonderivate der Formel I gemäss Anspruch 1 und ihrer Salze, dadurch gekennzeichnet, dass man eine Verbindung der Formel

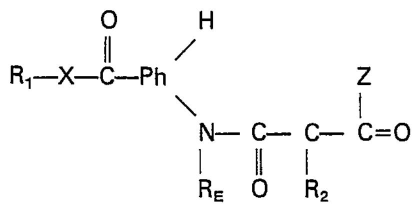

(IV),

worin Z einen abspaltbaren Rest bedeutet, oder ein Tautomeres und/oder Salz davon intramolekular cyclisiert oder eine Verbindung der Formel

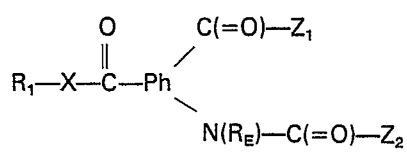

(VI),

worin einer der Reste $Z_1$ und $Z_2$ eine Gruppe der Formel $CH_2—R_2$ und der andere eine gegebenenfalls reaktionsfähig verätherte oder veresterte Hydroxygruppe bedeutet, oder eine Tautomeres und/oder Salz davon intramolekular cyclisiert oder eine Verbindung der Formel

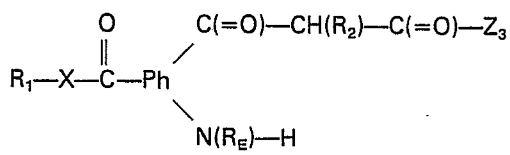

(IX),

worin $Z_3$ Hydroxy oder eine reaktionsfähige abgewandelte Hydroxygruppee bedeutet, oder ein Tautomeres und/oder Salz davon intramolekular kondensiert oder in einer Verbindung der Formel

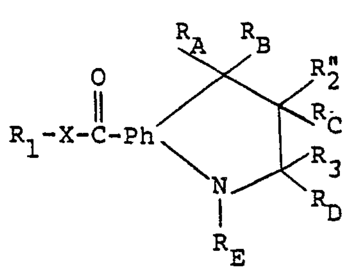

(XI),

worin $R_2''$ einen in die gegebenenfalls veresterte oder amidierte Carboxygruppe $R_2$ überführbaren Rest bedeutet, oder in einem Tautomeren und/oder Salze davon die Gruppe $R_2''$ in die gegebenenenfalls veresterte oder amidierte Carboxygruppe $R_2$ überführt oder in eine Verbindung der Formel

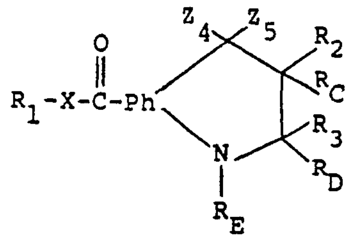

(XIII),

worin $Z_4$ einen in Hydroxy oder Niederalkoxy überführbaren Rest bedeutet und $Z_5$ gemeinsam mit $R_C$ sowie $R_D$ gemeinsam mit $R_E$ jeweils eine zusätzliche Bindung darstellt, oder worin $Z_4$ und $Z_5$ jeweils einen einwertigen oder gemeinsam einen zweiwertigen in Oxo überführbaren Rest bedeuten und $R_C$ gemeinsam mit $R_D$ eine zusätzliche Bindung darstellt, oder in einem Salz davon einen in Hydroxy bzw. Niederalkoxy überführbaren Rest $Z_4$ in Hydroxy oder Niederalkoxy bzw. in Oxo überführbare Reste $Z_4$ und $Z_5$ gemeinsam in Oxo überführt oder in einer Verbindung der Formel

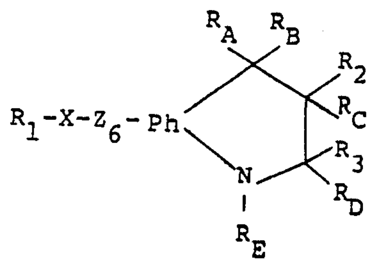

(XIX),

worin $Z_6$ einen in die Carbonylgruppe überführbaren Rest bedeutet, oder in einem Salz davon $Z_6$ in die Carbonylgruppe überführt oder in einer Verbindung der Formel

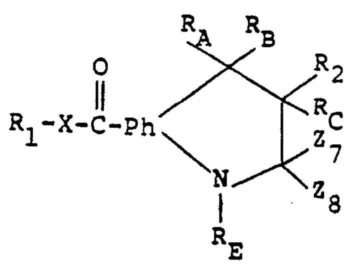

(XXI),

worin $Z_7$ einen in Hydroxy überführbaren Rest und $Z_8$ einen Rest $R_D$ bedeutet oder $Z_7$ und $Z_8$ jeweils einen einwertigen oder gemeinsam einen zweiwertigen in Oxo überführbaren Rest bedeuten, $Z_7$ in Hydroxy bzw. $Z_7 + Z_8$ in oxo überführt oder zur Herstellung von Verbindungen der Formel I, worin X Oxy bedeutet, in einer Verbindung der Formel

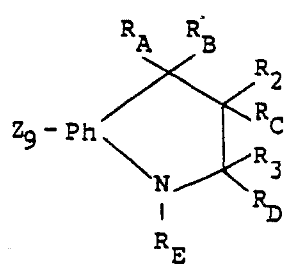

(XXIII),

worin $Z_9$ eine von der Gruppe $R_1$—X—(=O)— verschiedene funktionell abgewandelte Carboxygruppe bedeutet, $Z_9$ zu einer entsprechenden Gruppe $R_1$—O—C(=O)— solvolysiert oder zur Herstellung von Verbindungen der Formel I, worin X eine direkte Bindung bedeutet, eine Verbindung der Formel .

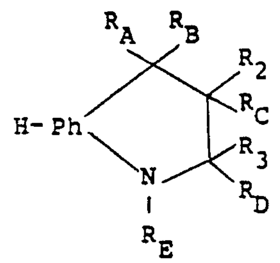

(XXV),

in Gegenwart einer Lewissäure mit einer Verbindung der Formel $R_1$—COOH (XXVI) oder einem geeigneten funktionellen Derivat davon umsetzt oder zur Herstellung von Verbindungen der Formel (I), worin X eine direkte Bindung ist, eine Verbindung der Formel XXVII

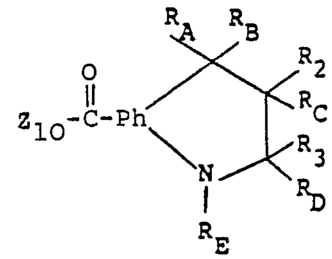

(XXVII),

worin $Z_{10}$ eine freie, verätherte oder reaktionsfähig veresterte Hydroxygruppe oder eine geeignete, über ein Ringstickstoffatom gebundene Heteroarylgruppe bedeutet, oder eine Salz davon mit einer Verbindung der Formel $R_1$—M (XXVIII), worin M einen Metallrest darstellt, zu einer entsprechenden Verbindung der Formel (I), worin $R_1$ von Wasserstoff verschieden ist, oder eine Verbindung der Formel (XXVII), worin $Z_{10}$ reaktionsfähig verestertes Hydroxy ist, mit Bis-(triphenylphin)-kupferboranat zu einer Verbindung der Formel I, worin $R_1$ Wasserstoff ist, umsetzt, und gewündschtenfalls die so erhältliche Verbindung in eine andere Verbindung der Formel I und/oder eine erhaltene salzbildende Verbindung in ein Salz oder ein erhaltenes Salz in die freie Verbindung oder in ein anderes Salz überführt.

29. Eine Verbindung der Formel

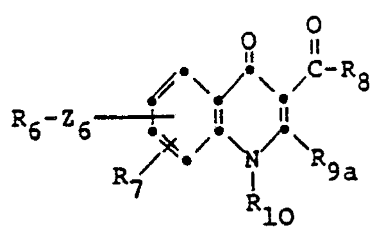

(XIXa),

worin $Z_6$ Hydroxymethylen und $R_6$ Niederalkyl mit bis zu 7 Kohlenstoffatomen bedeutet, $R_7$ Wasserstoff oder Niederalkyl mit bis und mit 4 Kohlenstoffatomen darstellt, wobei die Gruppe $R_6$—$Z_6$ die 6- und eine Niederalkylgruppe $R_7$ die 7-Stellung einnimmt, $R_8$ Hydroxy oder Niederalkoxy mit bis und mit 4 Kohlenstoffatomen bedeutet, $R_{9a}$ Hydroxy bedeutet und $R_{10}$ Niederalkyl oder Niederalkenyl mit bis und mit 4 Kohlenstoffatomen bedeutet, bzw. das 2-Oxo-4-hydroxy-1,2-dihydrochinolin- oder 2,4-Dioxo-1,2,3,4-tetrahydrochinolin-Tautomere und/oder ein Salz davon.

30. 1-Aethyl-4-hydroxy-6-(1-hydroxybutyl)-7-methyl-carbostiryl-3-carbonsäureäthylester gemäss Anspruch 29.

31. Eine Verbindung gemäss einem der Ansprüche 29 und 30 zur Anwendung in einem Verfahren zur prophylaktischen und/oder therapeutischen Behandlung des tierischen oder menschlichen Körpers.

32. Eine Verbindung gemäss einem der Ansprüche 29 und 30 zur Anwendung gemäss Anspruch 31 als antiallergisches Mittel.

33. Pharmazeutische Präparate enthaltend eine Verbindung gemäss einem der Ansprüche 29 und 30, gegebenenfalls in Form eines pharmazeutisch verwendbaren Salzes, neben üblichen pharmazeutischen Hilfs- und Trägerstoffen.

34. Verfahren zur Herstellung von Chinolinonderivaten der Formel XIXa gemäss Anspruch 29 und ihrer Salze, dadurch gekennzeichnet, dass man eine Verbindung der Formel

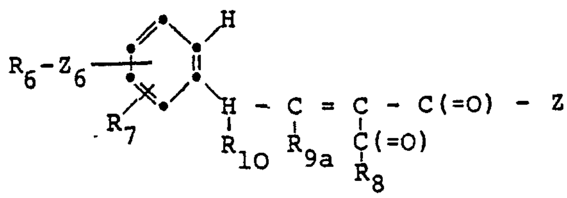

(IVa),

worin Z einen abspaltbaren Rest bedeutet, oder ein Tautomeres und/oder Salz davon intramolekular cyclisiert oder eine Verbindung der Formel

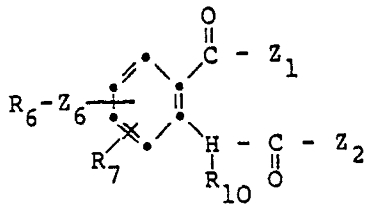

(VIa),

worin einer der Reste $Z_1$ und $Z_2$ eine Gruppe der Formel $CH_2—C(=O)—R_8$ und der andere eine gegebenenfalls reaktionsfähig verätherte oder veresterte Hydroxygruppe bedeutet, oder ein Tautomeres und/oder Salz davon intramolekular cyclisiert oder eine Verbindung der Formel

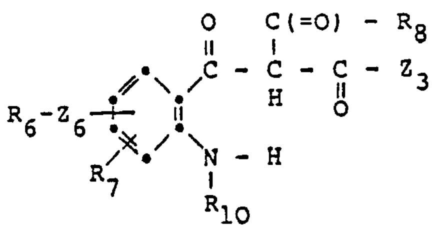

(IXa),

worin $Z_3$ Hydroxy oder eine reaktionsfähige abgewandelte Hydroxygruppe bedeutet, oder ein Tautomeres und/oder Salz davon intramolekular kondensiert oder in einer Verbindung der Formel

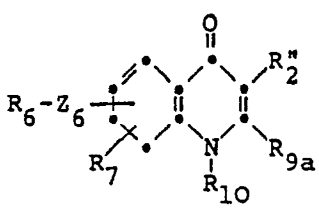

(XIa),

worin $R_2''$ einen in die gegebenenfalls veresterte oder amidierte Carboxygruppe $—C(=O)—R_8$ überführbaren Rest bedeutet, oder in einem Tautomeren und/oder Salz davon die Gruppe $R_2''$ in die gegebenenfalls veresterte oder amidierte Carboxygruppe $—C(=O)—R_8$ überführt oder in einer Verbindung der Formeln

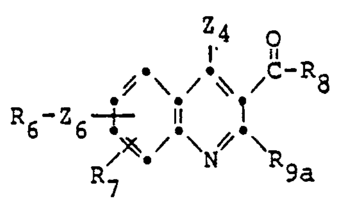

(XIIIa),

oder

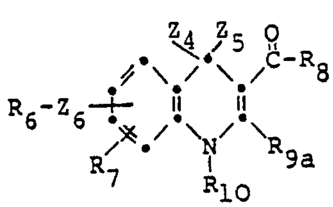

(XIIIb),

worin $Z_4$ in Formel XIIIa einen Hydroxy überführbaren Rest bedeutet oder worin in Formel XIIIb $Z_4$ und $Z_5$ jeweils einen einwertigen oder gemeinsam einen zweiwertigen in Oxo überführbaren Rest bedeuten, oder in einem Salz davon einen in Hydroxy überführbaren Rest $Z_4$ in Hydroxy bzw. in Oxo überführbare Reste $Z_4$ und $Z_5$ gemeinsam in Oxo überführt oder in einer Verbindung der Formeln

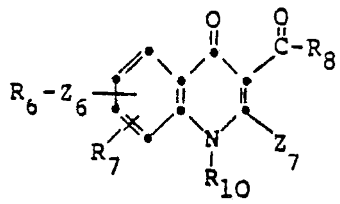

(XXIa),

oder

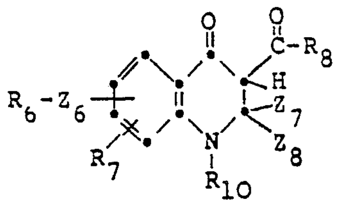

(XXIb),

worin in Formel XXIa $Z_7$ einen in Hydroxy überführbaren Rest oder in Formel XXIb $Z_7$ und $Z_8$ jeweils einen einwertigen oder gemeinsam einen zweiwertigen in Oxo überführbaren Rest bedeuten, $Z_7$ in Hydroxy bzw. $Z_7$ und $Z_8$ gemeinsam in Oxo überführt, oder eine Verbindung der Formel

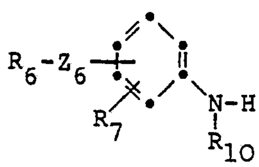

(Va)

mit einer Verbindung der Formel

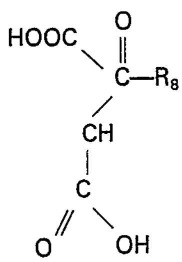

(XXa)

oder einen funktionellen Carboxyderivat bzw. Salz davon kondensiert oder in einer Verbindung der Formel

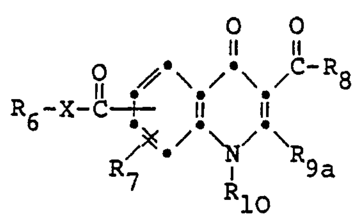

(II),

worin X eine direkte Bindung und $R_6$ Niederalkyl mit bis und mit 7 Kohlenstoffatomen ist, die Gruppe der Formel $R_6$—X—C(=O)— durch behandeln mit einem geeigneten Reduktionsmittel, wie einem Dileichtmetallhydrid, z.B. Natriumboranat oder Natriumcyanboranat, oder mit einem sekundären Alkohol, wie einem sekundären Niederalkanol oder einem Cycloalkanol, z.B. mit Isopropanol oder Cyclohexanol, in Gegenwart eines Aluminiumalkoholates, z.B. von Aluminiumisopropanolat oder -cyclohexanolat, zu einer Gruppe der Formel $R_6$—$Z_6$ reduziert oder einen Aldehyd der Formel

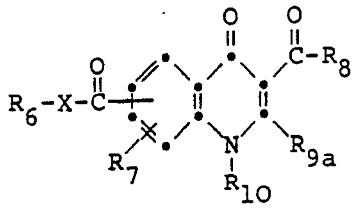

(II),

worin $R_6$ Wasserstoff und X eine direkte Bindung ist, mit einer Metallverbindung eines Niederalkans mit bis und mit 7 Kohlenstoffatomen der Formel $R_6$—H, wie einer Halogenmagnesium- oder Lithiumverbindung desselben, z.B. mit einem Niederalkyllithium, umsetzt, und gewünschtenfalls in einer verfahrensgemäss erhältlichen Verbindung der Formel XIXa Niederalkoxycarbonyl —C(=O)—$R_8$ zu Carboxy solvolysiert, Carboxy —C(=O)—$R_8$ zu Niederalkoxycarbonyl verestert und/oder ein verfahrensgemäss erhältliches Salz in die freie Verbindung oder eine verfahrensgemäss erhältliche freie Verbindung in ein Salz überführt.

35. Die nach dem Verfahren eines der Ansprüche 28, 29 und 34 erhältlichen Verbindungen.

36. Verwendung von Verbindungen gemäss einem der Ansprüche 1—25 und 29—32 als Arzneimittel oder zur Herstellung von Arzneimitteln auf nicht-chemischem Weg.

**Patentansprüche für den Vertragsstaat: AT**

1. Verfahren zur Herstellung von Chinolinderivaten der Formel

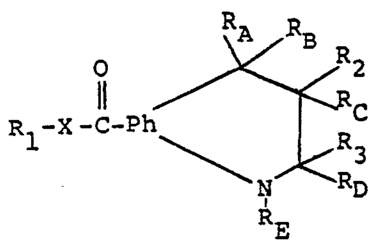

(I),

worin $R_1$ gegebenenfalls durch Hydroxy, Niederalkoxy, Hydroxyniederalkoxy, Niederalkoxyniederalkoxy, Hydroxyniederalkoxyniederalkoxy, Niederalkoxyniederalkoxyniederalkoxy, Niederalkylthio, Niederalkansulfinyl oder Niederalkansulfonyl substituiertes Niederalkyl, Niederalkanoyloxymethyl, Niederalkenyl oder Cycloalkyl, gegebenenfalls im Phenylteil durch Niederalkyl, Niederalkoxy und/oder Halogen substituiertes Phenyl oder Phenylniederalkyl, Furyl, Thienyl oder Pyridyl oder Furylniederalkyl, Thienylniederalkyl, Pyridylniederalkyl oder Wasserstoff bedeutet, X eine direkte Bindung oder Oxy darstellt, Ph gegebenenfalls zusätzlich durch Niederalkyl, Niederalkoxy, Hyroxy und/ oder Halogen substituiertes 1,2-Phenylen bedeutet, $R_2$ Carboxy, Niederalkoxycarbonyl, Hydroxyniederalkoxycarbonyl, Niederalkoxyniederalkoxycarbonyl, Diniederalkylaminoniederalkoxy- oder 5- bis 7-gliedriges Niederalkylenamino- bzw. Aza-, Oxa- oder Thianiederalkylenaminoniederalkoxycarbonyl oder amidiertes Carboxy bedeutet, das als Aminogruppe Amino, Hydroxyamino, Niederalkylamino, Diniederalkylamino oder 5- bis 7-gliedriges Niederalkylen- bzw. Aza-, Oxa-, Thianiederalkylenamino aufweist, und worin entweder $R_A$ und $R_B$ gemeinsam Oxo darstellen, $R_C$ und $R_D$ gemeinsam eine zusätzliche Bindung bedeuten und $R_3$ Hydroxy ist bzw. $R_C$ Wasserstoff ist und $R_3$ und $R_D$ gemeinsam Oxo darstellen und $R_E$ Wasserstoff, Niederalkyl, Hydroxyniederalkyl, Niederalkoxyniederalkyl, Diniederalkylaminoniederalkyl, 5- bis 7-gliedriges Niederalkylenamino- bzw. Aza-, Oxa- oder Thianiederalkylenaminoniederalkyl, Niederalkenyl, Niederalkinyl, Cycloalkyl mit 3 bis 8, vor allem 5 bis 7, Ringgliedern oder gegebenenfalls durch Niederalkyl, Niederalkoxy und/oder Halogen substituiertes Phenylniederalkyl, Furylniederalkyl, Thienylniederalkyl oder Pyridylniederalkyl darstellt, oder $R_A$ Hydroxy oder Niederalkoxy bedeutet, $R_B$ gemeinsam mit $R_C$ sowie $R_D$ gemeinsam mit $R_E$ jeweils eine zusätzliche Bindung darstellen und $R_3$ Hydroxy ist bzw. $R_B$ und $R_C$ gemeinsam eine zusätliche Bindung und $R_3$ und $R_D$ gemeinsam Oxo darstellen und $R_E$ Wasserstoff, Niederalkyl, Hydroxyniederalkyl, Niederalkoxyniederalkyl, Diniederalkylaminoniederalkyl, 5- bis 7-gliedriges Niederalkylenamino- bzw. Aza-, Oxa- oder Thianiederalkylenaminoniederalkyl, Niederalkenyl, Niederalkinyl, Cycloalkyl mit 3—8 Ringgliedern oder gegebenenfalls durch Niederalkyl, Niederalkoxy und/oder Halogen substituiertes Phenylniederalkyl, Furylniederalkyl, wobei "niedere" Reste jeweils bis und mit 7 Kohlenstoffatome aufweisen, Thienylniederalkyl oder Pyridylniederalkyl darstellt, und ihrer Salze, dadurch gekennzeichnet, dass man eine Verbindung der Formel

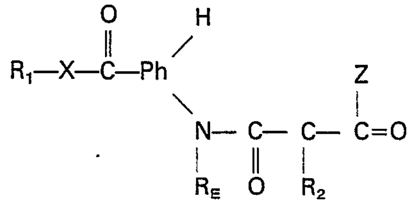

(IV),

worin Z einen abspaltbaren Rest bedeutet, oder ein Tautomeres und/oder Salz davon intramolekular cyclisiert oder eine Verbindung der Formel

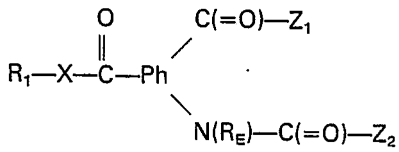

(VI),

worin einer der Reste $Z_1$ und $Z_2$ eine Gruppe der Formel $CH_2—R_2$ und der andere eine gegebenenfalls reaktionsfähig verätherte oder veresterte Hydroxygruppe bedeutet, oder eine Tautomeres und/oder Salz davon intramolekular cyclisiert oder eine Verbindung der Formel

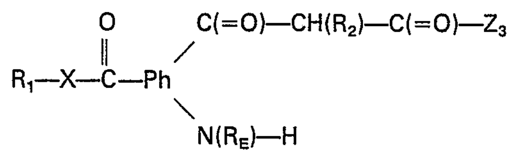

(IX),

worin $Z_3$ Hydroxy oder eine reaktionsfähige abgewandelte Hydroxygruppe bedeutet, oder ein Tautomeres und/oder Salz davon intramolekular kondensiert oder in einer Verbindung der Formel

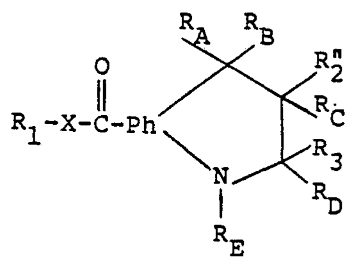

(XI),

worin $R_2''$ einen in die gegebenenfalls veresterte oder amidierte Carboxygruppe $R_2$ überführbaren Rest bedeutet, oder in einem Tautomeren und/oder Salze davon die Gruppe $R_2''$ in die gegebenenenfalls veresterte oder amidierte Carboxygruppe $R_2$ überführt oder in eine Verbindung der Formel

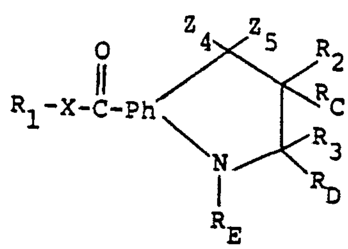

(XIII),

worin $Z_4$ einen in Hydroxy oder Niederalkoxy überführbaren Rest bedeutet und $Z_5$ gemeinsam mit $R_C$ sowie $R_D$ gemeinsam mit $R_E$ jeweils eine zusätzliche Bindung darstellt, oder worin $Z_4$ und $Z_5$ jeweils einen einwertigen oder gemeinsam einen zweiwertigen in Oxo überführbaren Rest bedeuten und $R_C$ gemeinsam mit $R_D$ eine zusätzliche Bindung darstellt, oder in einem Salz davon einen in Hydroxy bzw. Niederalkoxy überführbaren Rest $Z_4$ in Hydroxy oder Niederalkoxy bzw. in Oxo überführbare Reste $Z_4$ und $Z_5$ gemeinsam in Oxo überführt oder in einer Verbindung der Formel

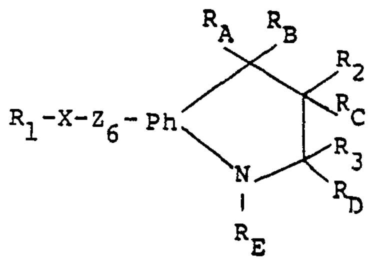

(XIX),

worin $Z_6$ einen in die Carbonylgruppe überführbaren Rest bedeutet, oder in einem Salz davon $Z_6$ in die Carbonylgruppe überführt oder in einer Verbindung der Formel

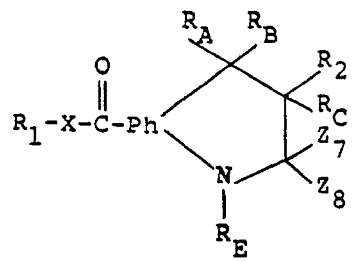

(XXI),

worin $Z_7$ einen in Hydroxy überführbaren Rest und $Z_8$ einen Rest $R_D$ bedeutet oder $Z_7$ und $Z_8$ jeweils einen einwertigen oder gemeinsam einen zweiwertigen in Oxo überführbaren Rest bedeuten, $Z_7$ in Hydroxy bzw. $Z_7 + Z_8$ in Oxo überführt oder zur Herstellung von Verbindungen der Formel I, worin X Oxy bedeutet, in einer Verbindung der Formel

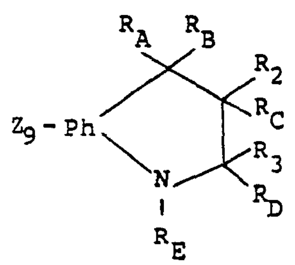

(XXIII),

worin $Z_9$ eine von der Gruppe $R_1$—X—(=O)— verschiedene funktionell abgewandelte Carboxygruppe bedeutet, $Z_9$ zu einer entsprechenden Gruppe $R_1$—O—C(=O)— solvolysiert oder zur Herstellung von Verbindungen der Formel I, worin X eine direkte Bindung bedeutet, eine Verbindung der Formel

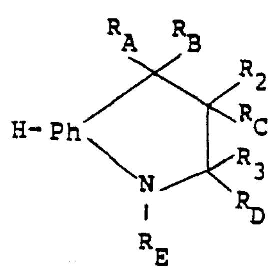

(XXV),

in Gegenwart einer Lewissäure mit einer Verbindung der Formel $R_1$—COOH (XXVI) oder einem geeigneten funktionellen Derivat davon umsetzt oder zur Herstellung von Verbindungen der Formel (I), worin X eine direkte Bindung ist, eine Verbindung der Formel XXVII

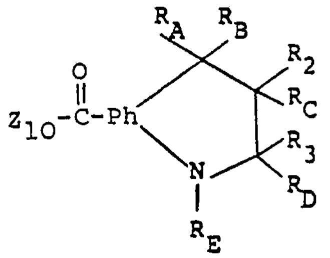

(XXVII),

worin $Z_{10}$ eine freie, verätherte oder reaktionsfähig veresterte Hydroxygruppe oder eine geeignete, über ein Ringstickstoffatom gebundene Heteroarylgruppe bedeutet, oder ein Salz davon mit einer Verbindung der Formel $R_1$—M (XXVIII), worin M einen Metallrest darstellt, zu einer entsprechenden Verbindung der Formel (I), worin $R_1$ von Wasserstoff verschieden ist, oder eine Verbindung der Formel (XXVII), worin $Z_{10}$ reaktionsfähig verestertes Hydroxy ist, mit Bis-(triphenylphin)-kupferboranat zu einer Verbindung dei Formel I, worin $R_1$ Wasserstoff ist, umsetzt, und gewünschtenfalls die so erhältliche Verbindung in eine andere Verbindung der Formel I und/oder eine erhaltene saltzbildende Verbindung in ein Salz oder ein erhaltenes Salz in die freie Verbindung oder in ein anderes Salz überführt.

2. Verfahren gemäss Anspruch 1, dadurch gekennzeichnet, dass man eine Verbindung der Formel

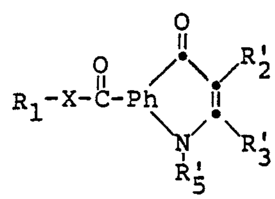

(Ia),

worin $R_1$ Niederalkyl mit bis zu 7 Kohlenstoffatomen, Niederalkoxy-, Niederalkylthio-, Niederalkansulfinyl- oder Niederalkansulfonylniederalkyl mit jeweils bis zu 4 Kohlenstoffatomen in den Alkylteilen, Niederalkenyl mit bis zu 4 Kohlenstoffatomen, 3- bis 8-gliedriges Cycloalkyl, gegebenenfalls durch Niederalkyl mit bis zu 4 Kohlenstoffatomen, Niederalkoxy mit bis zu 4 Kohlenstoffatomen und/oder Halogen bis einschliesslich Atomnummer 35 substituiertes Phenyl bzw. Phenylniederalkyl mit bis zu 4 Kohlenstoffatomen im Alkylteil, Furyl, Tienyl oder Pyridyl oder Furyl-, Thienyl- oder Pyridyl-niederalkyl mit bis zu 4 Kohlenstoffatomen im Alkylteil und X eine direkte Bindung bedeutet oder $R_1$ Wasserstoff oder Niederalkyl mit bis zu 7 Kohlenstoffatomen und X Oxy darstellt, und worin Ph die Gruppe $R_1$—X—C(=O)— aufweisendes, gegebenenfalls zusätzlich durch Niederalkyl mit bis zu 4 Kohlenstoffatomen, Niederalkoxy mit bis zu 4 Kohlenstoffatomen, Hydroxy oder Halogen bis einschliesslich Atomnummer 356 substituiertes 1,2-Phenylen darstellt, $R_2'$ für Carboxy, Niederalkoxycarbonyl mit bis zu 5 Kohlenstoffatomen, Hydroxyniederalkoxycarbonyl mit bis zu 5 Kohlenstoffatomen, Niederalkoxyniederalkoxycarbonyl mit bis zu 4 Kohlenstoffatomen in den Alkoxyteilen, Dieniederalkylaminoniederalkoxycarbonyl mit jeweils bis zu 4 Kohlenstoffatomen im Alkyl- und Alkoxyteil, Carbamoyl, N-Hydroxycarbamoyl oder N-Niederalkyl- bzw. N,N-Diniederalkylcarbamoyl mit bis zu 4 Kohlenstoffatomen im Alkylteil steht, $R_3'$ Hydroxy darstellt und $R_5'$ Wasserstoff, Niederalkyl oder Niederalkenyl mit bis zu 4 Kohlenstoffatomen, gegebenenfalls durch Niederalkyl mit bis zu 4 Kohlenstoffatomen, Niederalkoxy mit bis zu 4 Kohlenstoffatomen und/oder Halogen bis und mit Atomnummer 35 substituiertes Phenyl-, Furyl-, Thienyl- oder Pyridylniederalkyl mit bis zu 4 Kohlenstoffatomen im Alkylteil, Niederalkoxyniederalkyl mit bis zu 4 Kohlenstoffatomen im Alkoxy- bzw. Alkylteil oder Diniederalkylaminoniederalkyl, worin Niederalkyl bis zu 4 Kohlenstoffatome aufweist, oder ein Tautomeres und/oder Salz davon herstellt.

3. Verfahren gemäss Anspruch 1, dadurch gekennzeichnet, dass man eine Verbindung der Formel

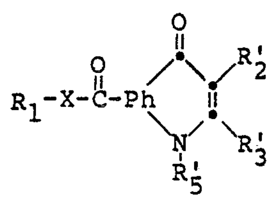

(Ia),

worin $R_1$ Wasserstoff, Niederalkyl mit bis zu 7 Kohlenstoffatomen, Niederalkoxy-, Niederalkylthio-, Niederalkanesulfinyl- oder Niederalkanesufonylniederalkyl mit jeweils bis zu 4 Kohlenstoffatomen in den Alkylteilen, Niederalkenyl mit bis zu 4 Kohlenstoffatomen oder 3—8 gliedriges Cycloalkyl und X eine direkte Bindung oder $R_1$ Wasserstoff, Niederalkyl mit bis zu 7 Kohlenstoffatomen, Niederalkoxy-, Niederalkylthio-, Niederalkansulfonyl- oder Niederalkansulfinylniederalkyl mit jeweils bis zu 4 Kohlenstoffatomen in den

Alkylteinen, Niederalkenyl mit bis zu 4 Kohlenstoffatomen oder 3- bis 8-gliedriges Cycloalkyl, Niederalkanoyloxymethyl mit bis zu 7 C-Atomen im Alkanoylteil oder ω-(ω-Hydroxyniederalkoxy)-niederalkyl, ω-(ω-Niederalkoxyniederalkoxy)-niederalkyl, ω-[ω-(ω-Hydroxyniederalkoxy)-niederalkoxy]-niederalkyl oder ω-[ω-(ω-Niederalkoxyniederalkoxy)-niederalkoxy]-niederalkyl mit jeweils 1 bis 4 Kohlenstoffatomen im Alkyl- und 2 bis 4 Kohlenstoffatomen im Alkylenteil und X Oxy bedeutet, Ph die Gruppe $R_1$—X—C(=O)— aufweisendes, gegebenenfalls zusätzlich durch Niederalkyl mit bis zu 4 Kohlenstoffatomen, Niederalkoxy mit bis zu 4 Kohlenstoffatomen, Hydroxy oder Halogen bis einschliesslich Atomnummer 35 substituiertes 1,2-Phenylen darstellt, $R_2'$ für Carboxy, Niederalkoxycarbonyl mit bis zu 5 Kohlenstoffatomen, Niederalkoxyniederalkoxycarbonyl mit bis zu 4 Kohlenstoffatomen in den Alkoxyteilen, Carbamoyl, N-Hydroxycarbamoyl oder N-Niederalkyl- bzw. N,N-Diniederalkylcarbamoyl mit bis zu 4 Kohlenstoffatomen im Alkylteil steht, $R_3'$ Hydroxy darstellt und $R_5'$ Wasserstoff, Niederalkyl, Niederalkenyl oder Niederalkinyl mit bis zu 4 Kohlenstoffatomen bedeutet, Sowie Tautomere derselben, in denen die Hydroxygruppe $R_3'$ in der tautomeren Oxoform vorliegt, oder eine Salz und/oder Tautomeres davon herstellt.

4. Verfahren gemäss Anspruch 1 dadurch gekennzeichnet, dass man eine Verbindung der Formel

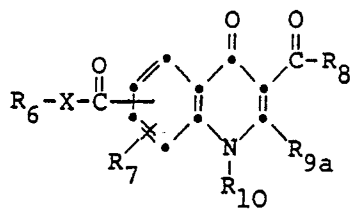

(II),

worin X eine direkte Bindung und $R_6$ Wasserstoff oder Niederalkyl mit bis und mit 7 Kohlenstoffatomen bedeutet oder X für Oxy und $R_6$ für Wasserstoff, Niederalkyl mit bis und mit 7 Kohlenstoffatomen, ω-Hydroxyniederalkyl mit 2 bis 4 Kohlenstoffatomen, Niederalkanoyloxymethyl mit bis und mit 7 C-Atomen im Alkanoylteil oder ω-(ω-Hydroxyniederalkoxy)-niederalkyl, ω-(ω-Niederalkoxyniederalkoxy)-niederalkyl, ω-[ω-(ω-Hydroxyniederalkoxy)-niederalkoxy]-niederalkyl oder ω-[ω-(ω-Niederalkoxyniederalkoxy)-niederalkoxy]-niederalkyl mit jeweils 1 bis 4 Kohlenstoffatomen im Alkyl- und 2 bis 4 Kohlenstoffatomen im Alkylenteil bedeutet, $R_7$ Wasserstoff, Niederalkyl mit bis und mit 4 Kohlenstoffatomen, Niederalkoxy mit bis und mit 4 Kohlenstoffatomen, Halogen bis Atomnummer 35 oder Hydroxy darstellt, $R_8$ Hydroxy oder Niederalkoxy mit bis zu 4 Kohlenstoffatomen bedeutet, $R_{9a}$ Hydroxy darstellt, und $R_{10}$ Niederalkyl, Niederalkenyl oder Niederalkinyl mit bis und mit zu 4 Kohlenstoffatomen ist, wobei eine 2-Hydroxy-4-oxo-1,4-dihydrochinolinverbindung der Formel II auch in der tautomeren 2-Oxo-4-hydroxy-1,2-dihydro- oder 2,4-Dioxo-1,2,3,4-tetrahydrochinolinform vorliegen kann, oder ein Salz davon herstellt.

5. Verfahren gemäss Anspruch 4, dadurch gekennzeichnet, dass man eine Verbindung der Formel II, worin X Oxy darstellt die Gruppe $R_6$—O—C(=O)— in 6-Stellung gebunden ist, $R_6$ Wasserstoff, Niederalkyl mit bis zu 7 Kohlenstoffatomen, ω-Hydroxyniederalkyl mit 2 bis 4 Kohlenstoffatomen, Niederalkanoyloxymethyl mit bis und mit 7 C-Atomen im Alkylteil oder ω-(ω-Hydoxyniederalkoxy)-niederalkyl, ω-(ω-Niederalkoxyniederalkoxy)-niederalkyl, ω-ω-[ω-[ω-(ω-Hydroxyniederalkoxy)-niederalkoxy]-niederalkyl oder ω-[ω-(ω-Niederalkoxyniederalkoxy)-niederalkoxy]-niederalkyl mit jeweils 1 bis 4 Kohlenstoffatomen im Alkyl- und 2 bis 4 Kohlenstoffatomen im Alkylenteil bedeutet, $R_7$ Wasserstoff darstellt und $R_{10}$ Niederalkenyl mit bis zu 4 Kohlenstoffatomen bedeutet, oder X eine direkte Bindung darstellt , $R_6$ Wasserstoff oder Niederalkyl mit bis zu 7 Kohlenstoffatomen bedeutet, $R_7$ Niederalkyl mit bis und mit 4 Kohlenstoffatomen darstellt, wobei die Gruppe $R_6$—C(=O)— die 6- und die Niederalkylgruppe $R_7$ die 7-Stellung einnimmt, und $R_{10}$ Niederalkyl mit bis und mit 4 Kohlenstoffatomen bedeutet, wobei $R_8$ und $R_{9a}$ jeweils in Anspruch 5 angegebenen Bedeutungen haben, bzw. das 2-Oxo-4-hydroxy-1,2,-dihydrochinolin- oder 2,4-Dioxo-1,2,3,4,-tetrahydrochinolin-Tautomere und/oder ein Salz davon herstellt.

6. Verfahren gemäss Anspruch 4, dadurch gekennzeichnet, dass man eine Verbindung der Formel II, worin X eine direkte Bindung darstellt, $R_6$ Niederalkyl mit bis zu 7 Kohlenstoffatomen bedeutet, $R_7$ Wasserstoff oder Niederalkyl mit bis und mit 4 Kohlenstoffatomen darstellt, wobei die Gruppe $R_6$—C(=O)— die 6- und eine Niederalkylgruppe $R_7$ die 7-Stellung einnimmt, $R_8$ und $R_{9a}$ Hydroxy darstellen und $R_{10}$ Niederalkyl oder Niederalkenyl mit bis und mit 4 Kohlenstoffatomen bedeutet, bzw. das 2-Oxo-4-hydroxy-1,2-dihydrochinolin- oder 2,4-Dioxo-1,2,3,4,-tetrahydrochinolin-Tautomere und/oder ein Salz davon herstellt.

7. Verfahren gemäss Anspruch 4, dadurch gekennzeichnet, das man eine Verbindung der Formel II, worin X Oxy darstellt, $R_6$ Wasserstoff oder Niederalkyl mit bis zu 7 Kohlenstoffatomen bedeutet, $R_7$ Wasserstoff oder Niederalkyl mit bis und mit 7 Kohlenstoffatomen darstellt, wobei die Gruppe $R_6$—O—C(=O)— die 6- und eine Niederalkylgruppe $R_7$ die 7-Stellung einnimmt, $R_8$ und $R_{9a}$ die in Anspruch 5 angegebenen Bedeutungen haben und $R_{10}$ Niederalkyl oder Niederalkenyl mit bis und mit 4 Kohlenstoffatomen bedeutet, bzw. das 2-Oxo-4-hydroxy-1,2-dihydrochinolin- oder 2,4-Dioxo-1,2,3,4,-tetrahydrochinolin-Tautomere und/oder ein Salz davon herstellt.

8. Verfahren gemäss Anspruch 1, dadurch gekennzeichnet, dam men den 6-Butyryl-4-hydroxy-1,7-dimethyl-carbostiryl-3-carbonsäureäthylester herstellt.

9. Verfahren gemäss Anspruch 1, dadurch gekennzeichnet, dass man den 6-Butyryl-4-hydroxy-7-methyl-1-propyl-carbostiryl-3-carbonsäureäthylester herstellt.

10. Verfahren gemäss Anspruch 1, dadurch gekennzeichnet, dass man den 1-Aethyl-6-butyryl-4-hydroxy-7-methyl-carbostiryl-3-carbonsäureäthylester herstellt.

11. Verfahren gemäss Anspruch 1, dadurch gekennzeichnet, dass man den 1-Allyl-6-butyryl-4-hydroxy-7-methyl-carbostiryl-3-carbonsäureäthylester herstellt.

12. Verfahren gemäss Anspruch 1, dadurch gekennzeichnet, dass man den 1-Aethyl-6-butyryl-4-hydroxy-carbostiryl-3-carbonsäureäthylester herstellt.

13. Verfahren gemäss Anspruch 1, dadurch gekennzeichnet, dass man den 1-Butyl-6-butyryl-4-hydroxy-7-methyl-carbostiryl-3-carbonsäureäthylester herstellt.

14. Verfahren gemäss Anspruch 1, dadurch gekennzeichnet, dass man den 1-Allyl-6-methoxycarbonyl-4-hydroxy-carbostiryl-3-carbonsäureäthylester herstellt.

15. Verfahren gemäss Anspruch 1, dadurch gekennzeichnet, dass man den 1-Allyl-6-carboxy-4-hydroxy-carbostiryl-3-carbonsäureäthylester oder ein Salz Davon herstellt.

16. Verfahren gemäss Anspruch 1, dadurch gekennzeichnet, dass man den 1-Aethyl-6-butyryl-4-hydroxy-7-methyl-carbostiryl-3-carbonsäure-tert.-butylester herstellt.

17. Verfahren gemäss Anspruch 1, dadurch gekennzeichnet, dass man die 1-Aethyl-6-butyryl-4-hydroxy-7-methyl-carbostiryl-3-carbonsäure oder ein Salz davon herstellt.

18. Verfahren gemäss Anspruch 1, dadurch gekennzeichnet, dass man den 6-Methoxycarbonyl-1-propargyl-4-hydroxy-carbostiryl-3-carbonsäureäthylester oder ein Salz davon herstellt.

19. Verfahren gemäss Anspruch 1, dadurch gekennzeichnet, dass man den 1-Aethyl-6-butyryl-4-hydroxy-7-methyl-carbostiryl-3-carboxamid herstellt.

20. Verfahren gemäss Anspruch 1, dadurch gekennzeichnet, dass man den 1-Aethyl-6-formyl-4-hydroxy-7-methyl-carbostiryl-3-carbonsäureäthylester herstellt.

21. Verfahren gemäss Anspruch 1, dadurch gekennzeichnet, dass man den 1-Aethyl-6-methoxycarbonyl-4-hydroxy-carbostiryl-3-carbonsäureäthylester herstellt.

22. Verfahren gemäss Anspruch 1, dadurch gekennzeichnet, dass man den 1-Aethyl-6-carboxy-4-hydroxy-carbostiryl-3-carbonsäureäthylester oder ein Salz davon herstellt.

23. Verfahren zur Herstellung pharmazeutischer Präparate, dadurch gekennzeichnet, dass man eine gemäss einer der Ansprüche 1—22 erhältliche Verbindung, gegebebenenfalls in Form eines pharmazeutisch verwendbaren Salzes, durch Vermischen mit üblichen pharmazeutischen Hilfs- und Trägerstoffen in eine für die pharmazeutische Verabreichung geeignete Form bring.

24. Verfahren gemäss Anspruch 23 zur Herstellung antiallergischer Mittel.

25. Verfahren zur Herstellung neuer Chinolinonderivate der Formel

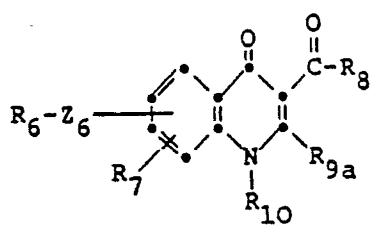

(XIXa),

worin $Z_6$ Hydroxymethylen und $R_6$ Niederalkyl mit bis zu 7 Kohlenstoffatomen bedeutet, $R_7$ Wasserstoff oder Niederalkyl mit bis und mit 4 Kohlenstoffatomen darstellt, wobei die Gruppe $R_6$—$Z_6$ die 6- und eine Niederalkylgruppe $R_7$ die 7-Stellung einnimmt, $R_8$ Hydroxy oder Niederalkoxy mit bis und mit 4 Kohlenstoffatomen bedeutet, $R_{9a}$ Hydroxy bedeutet und $R_{10}$ Niederalkyl oder Niederalkenyl mit bis und mit 4 Kohlenstoffatomen bedeutet, bzw. ihrer 2-Oxo-4-hydroxy-1,2-dihydrochinolin- oder 2,4-Dioxo-1,2,3,4-tetrahydrochinolin-Tautomeren und/oder Salze, dadurch gekennzeichnet, dass man eine Verbindung der Formel

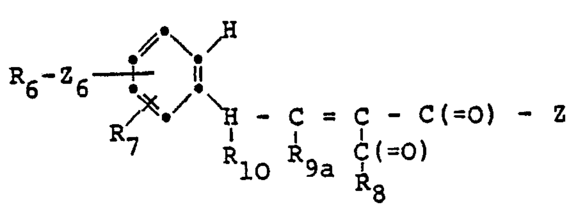

(IVa),

worin Z einen abspaltbaren Rest bedeutet, oder ein Tautomeres und/oder Salz davon intramolekular cyclisiert oder eine Verbindung der Formel

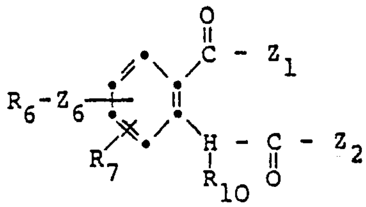

(VIa),

worin einer der Reste $Z_1$ und $Z_2$ eine Gruppe der Formel $CH_2—C(=O)—R_8$ und der andere eine gegebenenfalls reaktionsfähig verätherte oder veresterte Hydroxygruppe bedeutet, oder ein Tautomeres und/oder Salz davon intramolekular cyclisiert oder eine Verbindung der Formel

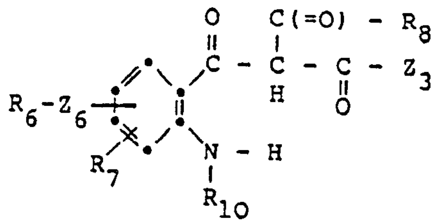

(IXa),

worin $Z_3$ Hydroxy oder eine reaktionsfähige abgewandelte Hydroxygruppe bedeutet, oder ein Tautomeres und/oder Salz davon intramolekular kondensiert oder in einer Verbindung der Formel

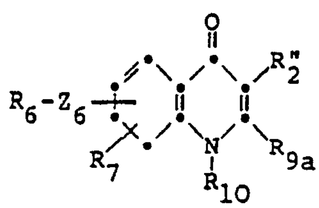

(XIa),

worin $R_2''$ einen in die gegebenenfalls veresterte oder amidierte Carboxygruppe $—C(=O)—R_8$ überführbaren Rest bedeutet, oder in einem Tautomeren und/oder Salz davon die Gruppe $R_2''$ in die gegebenenfalls veresterte oder amidierte Carboxygruppe $—C(=O)—R_8$ überführt oder in einer Verbindung der Formeln

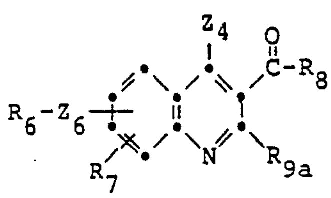

(XIIIa),

oder

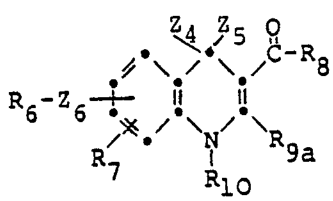

(XIIIb),

worin $Z_4$ in Formel XIIIa einen Hydroxy überführbaren Rest bedeutet oder worin in Formel XIIIb $Z_4$ und $Z_5$ jeweils einen einwertigen oder gemeinsam einen zweiwertigen in Oxo überführbaren Rest bedeuten, oder in einem Salz davon einen in Hydroxy überführbaren Rest $Z_4$ in Hydroxy bzw. in Oxo überführbare Reste $Z_4$ und $Z_5$ gemeinsam in Oxo überführt oder in einer Verbindung der Formeln

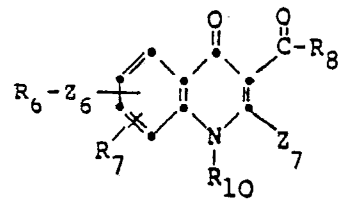

(XXIa),

oder

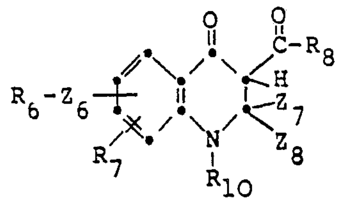

(XXIb),

worin in Formel XXIa $Z_7$ einen in Hydroxy überführbaren Rest oder in Formel XXIb $Z_7$ und $Z_8$ jeweils einen einwertigen oder gemeinsam einen zweiwertigen in Oxo überführbaren Rest bedeuten, $Z_7$ in Hydroxy bzw. $Z_7$ und $Z_8$ gemeinsam in Oxo überführt, oder eine Verbindung der Formel

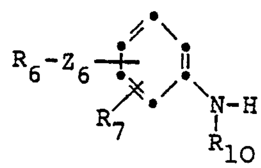

(Va)

mit einer Verbindung der Formel

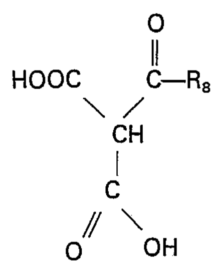

(XXa)

oder einen funktionellen Carboxyderivat bzw. Salz davon kondensiert oder in einer Verbindung der Formel

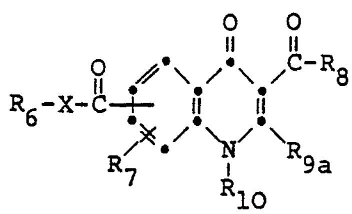

(II),

worin X eine direkte Bindung und $R_6$ Niederalkyl mit bis und mit 7 Kohlenstoffatomen ist, die Gruppe der Formel $R_6$—X—C(=O)— durch Behandeln mit einem geeigneten Reduktionsmittel, wie einem Dileichtmetallhydrid, z.B. Natriumboranat oder Natriumcyanboranat, oder mit einem sekundären Alkohol, wie einem sekundären Niederalkanol oder einem Cycloalkanol, z.B. mit Isopropanol oder Cyclohexanol, in Gegenwart eines Aluminumalkoholates, z.B. von Aluminiumisopropanolat oder -cyclohexanolat, zu einer Gruppe der Formel $R_6$—$Z_6$ reduziert oder einen Aldehyd der Formel

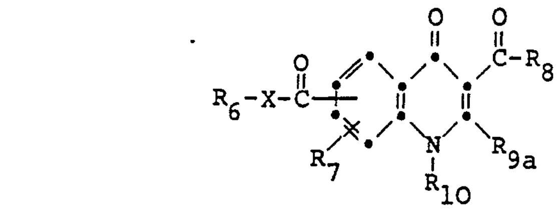

(II),

worin $R_6$ Wasserstoff und X eine direkte Bindung ist, mit einer Metallverbindung eines Niederalkans mit bis und mit 7 Kohlenstoffatomen der Formel $R_6$—H, wie einer Halogenmagnesium- oder Lithiumverbindung desselben, z.B. mit einem Niederalkyllithium, umsetzt, und gewünschtenfalls in einer verfahrensgemäss erhältlichen Verbindung der Formel XIXa Niederalkoxycarbonyl —C(=O)—$R_8$ zu Carboxy solvolysiert, Carboxy —C(=O)—$R_8$ zu Niederalkoxycarbonyl verestert und/oder ein verfahrensgemäss erhältliches Salz in die freie Verbindung oder eine verfahrensgemäss erhältliche freie Verbindung in ein Salz überführt.

26. Verfahren gemäss Anspruch 25, dadurch gekennzeichnet, dass man den 1-Aethyl-4-hydroxy-6-(1-hydroxybutyl)-7-methylcarbostiryl-3-carbonsäureäthylester herstellt.

27. Verfahren zur Herstellung pharmazeutischer Präparate, dadurch gekennzeichnet, dass man eine gemäss einem der Ansprüche 25 und 26 erhältlichen Verbindung oder ein pharmazeutische verwendbares Salz davon durch Vermischen mit üblichen pharmazeutischen Hilfs- und Trägerstoffen in eine für die pharmazeutische Verabreichung geeigneter Form bringt.

**Claims for the Contracting States: DE FR CH LI IT NL BE SE LU**

1. A quinoline derivative of the formula

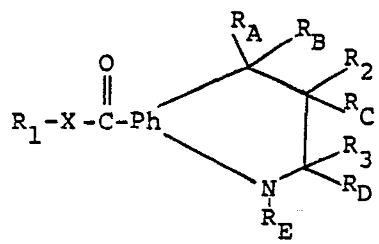

(I),

in which $R_1$ represents lower alkyl, lower alkanoyloxymethyl, lower alkenyl or cycloalkyl, each of which is unsubstituted or substituted by hydroxy, lower alkoxy, hydroxy-lower alkoxy, lower alkoxy-lower alkoxy, hydroxy-lower alkoxy-lower alkoxy, lower alkoxy-lower alkoxy-lower alkoxy, lower alkylthio, lower alkanesulphinyl or lower alkanesulphonyl; phenyl or phenyl-lower alkyl, each of which is unsubstituted or substituted in the phenyl moiety by lower alkyl, lower alkoxy and/or halogen; furyl, thienyl or pyridyl, or furyl-lower alkyl, thienyl-lower alkyl or pyridyl-lower alkyl or hydrogen, X represents a direct bond or oxy, Ph represents 1,2-phenylene which may be additionally substituted by lower alkyl, lower alkoxy, hydroxy and/or halogen, $R_2$ represents carboxy, lower alkoxycarbonyl, hydroxy-lower alkoxycarbonyl, lower alkoxy-lower alkoxycarbonyl, di-lower alkylamino-lower alkoxy- or 5- to 7-membered lower alkyleneamino- or aza-, oxa- or thia-lower alkylene-amino-lower alkoxycarbonyl or amidated carboxy containing, as amino group, amino, hydroxyamino, lower alkylamino, di-lower alkylamino or 5- to 7-membered lower alkylene- or aza-, oxa- or thia-lower alkyleneamino, and in which either $R_A$ and $R_B$ together represent oxo, $R_C$ and $R_D$ together represent an additional bond and $R_3$ represents hydroxy, or $R_C$ is hydrogen and $R_3$ and $R_D$ together represent oxo, and $R_E$ represents hdyrogen, lower alkyl, hydroxy-lower alkyl, lower alkoxy-lower alkyl, di-lower alkylamino-lower alkyl, 5- to 7-membered lower alkyleneamino- or aza-, oxa- or thia-lower alkyleneamino-lower alkyl, lower alkenyl, lower alkynyl, cycloalkyl having 3 to 8, especially 5 to 7, ring members; phenyl-lower alkyl, furyl-lower alkyl, thienyl-lower alkyl or pyridyl-lower alkyl, each of which is unsubstituted or substituted by lower alkyl, lower alkoxy and/or halogen, or $R_A$ represents hdyroxy or lower alkoxy, $R_B$ together with $R_C$ and also $R_D$ together with $R_E$ represent an additional bond in each case and $R_3$ represents hydroxy, or $R_B$ and $R_C$ together represent an additional bond and $R_3$ and $R_D$ together represent oxo, and $R_E$ represents hydrogen, lower alkyl, hydroxy-lower alkyl, lower alkoxy-lower alkyl, di-lower alkylamino-lower alkyl, 5- to 7-membered lower alkyleneamino- or aza-, oxa- or thia-lower alkyleneamino-lower alkyl, lower alkenyl, lower alkynyl, cycloalkyl having 3 to 8 ring members; phenyl-lower alkyl, furyl-lower alkyl, thienyl-lower alkyl or pyridyl-lower alkyl, each of which is substituted by lower alkyl, lower alkoxy and/or halogen, with radicals qualified by the term "lower" containing up to and including 7 carbon atoms, or a salt thereof.

2. A compound according to claim 1, in which X repesents a direct bond and $R_1$, $R_2$, $R_3$, $R_A$, $R_B$, $R_C$, $R_D$ and $R_E$ are as defined in claim 1, except that $R_1$ is other than hydrogen; or X represents oxy, $R_1$ represents lower alkyl, or phenyl which is unsubstituted or substituted by lower alkyl, lower alkoxy, hydroxy and/or

halogen, and $R_2$, $R_3$, $R_A$, $R_B$, $R_C$, $R_D$ and $R_E$ are as defined in claim 1.

3. A compound according to claim 1 of the formula

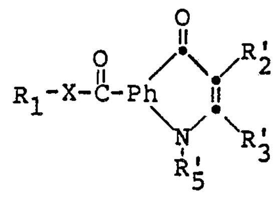

(Ia),

in which $R_1$ represents lower alkyl having up to 7 carbon atoms; lower alkoxy-, lower alkylthio-, lower alkanesulphinyl- or lower alkanesulphonyl-lower alkyl, each having up to 4 carbon atoms in the alkyl moiety; lower alkenyl having up to 4 carbon atoms; 3- to 8-membered cycloalkyl; phenyl or phenyl-lower alkyl having up to 4 carbon atoms in the alkyl moiety, each of which is unsubstituted or substituted by lower alkyl having up to 4 carbon atoms, lower alkoxy having up to 4 carbon atoms and/or halogen having an atomic number of up to and including 35; furyl, thienyl or pyridyl, or furyl-, thienyl- or pyridyl-lower alkyl having up to 4 carbon atoms in the alkyl moiety, and X represents a direct bond, or $R_1$ represents hydrogen or lower alkyl having up to 7 carbon atoms and X represents oxy, and in which Ph represents 1,2-phenylene which contains the group $R_1—X—C(=O)—$ and may be additionally substituted by lower alkyl having up to 4 carbon atoms, lower alkoxy having up to 4 carbon atoms, hydroxy or halogen having an atomic number of up to and including 35, $R_2'$ represents carboxy, lower alkoxycarbonyl having up to 5 carbon atoms, hydroxy-lower alkoxycarbonyl having up to 5 carbon atoms, lower alkoxy-lower alkoxycarbonyl having up to 4 carbon atoms in the alkoxy moieties, di-lower alkylamino-lower alkoxycarbonyl having up to 4 carbon atoms in the alkyl moiety and up to 4 carbon atoms in the alkoxy moiety, carbamoyl, N-hydroxy-carbamoyl or N-lower alkyl- or N,N-di-lower alkyl-carbamoyl having up to 4 carbon atoms in the alkyl moiety, $R_3'$ represents hydroxy and $R_5'$ represents hydrogen; lower alkyl or lower alkenyl having up to 4 carbon atoms; phenyl- furyl-, thienyl- or pyridyl-lower alkyl which have up to 4 carbon atoms in the alkyl moiety and each of which is unsubstituted or substituted by lower alkyl having up to 4 carbon atoms, lower alkoxy having up to 4 carbon atoms and/or halogen having an atomic number of up to and including 35; lower alkoxy-lower alkyl having up to 4 carbon atoms in the alkoxy moiety and up to 4 carbon atoms in the alkyl moiety; or di-lower alkylamino-lower alkyl in which lower alkyl has up to 4 carbon atoms, or a tautomer and/or a salt thereof.

4. A compound according to claim 1 of the formula

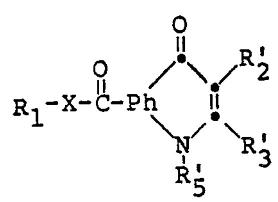

(Ia),

in which $R_1$ represents hydrogen; lower alkyl having up to 7 carbon atoms; lower alkoxy-, lower alkylthio-, lower alkanesulphinyl- or lower alkanesulphonyl-lower alkyl, each having up to 4 carbon atoms in the alkyl moiety; lower alkenyl having up to 4 carbon atoms; or 3- to 8-membered cycloalkyl, and X represents a direct bond, or $R_1$ represents hydrogen; lower alkyl having up to 7 carbon atoms; lower alkoxy-, lower alkylthio-, lower alkanesulphinyl- or lower alkanesulphonyl-lower alkyl, each having up to 4 carbon atoms in the alkyl moiety; lower alkenyl having up to 4 carbon atoms; or 3- to 8-membered cycloalkyl; lower alkanoyloxymethyl having up to and including 7 carbon atoms in the alkanoyl moiety; or ω-(ω-hydroxy-lower alkoxy)-lower alkyl, ω-(ω-lower alkoxy-lower alkoxy)-lower alkyl, ω-[ω-(ω-hydroxy-lower alkoxy)-lower alkoxy]-lower alkyl or ω-[ω-(ω-lower alkoxy-lower alkoxy)-lower alkoxy]-lower alkyl, each having from 1 to 4 carbon atoms in the alkyl moiety and from 2 to 4 carbon atoms in the alkylene moiety, and X represents oxy, Ph represents 1,2-phenylene which contains the group $R_1—X—C(=O)—$ and may be additionally substituted by lower alkyl having up to 4 carbon atoms, lower alkoxy having up to 4 carbon atoms, hydroxy or halogen having an atomic number of up to and including 35, $R_2'$ represents carboxy, lower alkoxycarbonyl having up to 5 carbon atoms, lower alkoxy-lower alkoxycarbonyl having up to 4 carbon atoms in the alkoxy moieties, carbamoyl, N-hydroxycarbamoyl or N-lower alkyl- or N,N-di-lower alkyl-carbamoyl having up to 4 carbon atoms in the alkyl moiety , $R_3'$ represents hydroxy, and $R_5'$ represents hydrogen, lower alkyl or lower alkenyl or lower alkynyl having up to 4 carbon atoms, or a tautomer thereof in which a hydroxyl group $R_3'$ is in the tautomeric oxo form, or a salt of a salt-forming compound of the formula (Ia) or a tautomer thereof.

5. A compound according to claim 1 of the formula

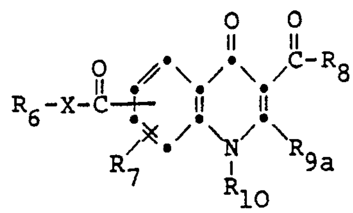

(II),

in which X represents a direct bond and $R_6$ represents hydrogen or lower alkyl having up to 7 carbon atoms, or X represents oxy and $R_6$ represents hydrogen, lower alkyl having up to and including 7 carbon atoms, ω-hydroxy-lower alkyl having from 2 to 4 carbon atoms, lower alkanoyloxymethyl having up to and including 7 carbon atoms in the alkanoyl moiety, or ω-(ω-hydroxy-lower alkoxy)-lower alkyl, ω-(ω-lower alkoxy-lower alkoxy)-lower alkyl, ω-[ω-(ω-hydroxy-lower alkoxy)-lower alkoxy]-lower alkyl or ω-[ω-(ω-lower alkoxy-lower alkoxy)-lower alkoxy]-lower alkyl, each having from 1 to 4 carbon atoms in the alkyl moiety and 2 to 4 carbon atoms in the alkylene moiety, $R_7$ represents hydrogen or lower alkyl having up to and including 4 carbon atoms, lower alkoxy having up to and including 4 carbon atoms, halogen having an atomic number of up to and including 35, or hydroxy, $R_8$ represents hydroxy or lower alkoxy having up to 4 carbon atoms, $R_{9a}$ represents hyrogen or hydroxy, and $R_{10}$ represents lower alkyl, lower alkenyl or lower alkynyl having up to 4 carbon atoms, it also being possible for a 2-hydroxy-4-oxo-1,4-dihydroquinoline compound of the formula II to be in the tautomeric 2-oxo-4-hydroxy-1,2-dihydro- or 2,4-dioxo-1,2,3,4-tetrahydroquinoline form, or a salt thereof.

6. A compound according to claim 5, in which X represents oxy, the group $R_6$—O—C(=O)— is bonded in the 6-position, $R_6$ represents hydrogen, lower alkyl having up to 7 carbon atoms, ω-hydroxy-lower alkyl having 2 to 4 carbon atoms, lower alkanoyloxymethyl having up to and including 7 carbon atoms in the alkyl moiety, or ω-(ω-hydroxy-lower alkoxy)-lower alkyl, ω-(ω-lower alkoxy-lower alkoxy)-lower alkyl, ω-[ω-ω-[ω-(ω-hydroxy-lower alkoxy)-lower alkoxy]-lower alkyl or ω-[ω-(ω-lower alkoxy-lower alkoxy)-lower alkoxy]-lower alkyl, each having from 1 to 4 carbon atoms in the alkyl moiety and 2 to 4 carbon atoms in the alkylene moiety, $R_7$ represents hydrogen, and $R_{10}$ represents lower alkenyl having up to 4 carbon atoms, or X represents a direct bond, $R_6$ represents hydrogen or lower alkyl having up to 7 carbon atoms, $R_7$ represents lower alkyl having up to and including 4 carbon atoms, the group $R_6$—C(=O)— being in the 6-position and the lower alkyl group $R_7$ in the 7-position, and $R_{10}$ represents lower alkyl having up to and including 4 carbon atoms, and each of $R_8$ and $R_{9a}$ is as defined in claim 5, or the 2-oxo-4-hydroxy-1,2-dihydroquinoline or 2,4-dioxo-1,2,3,4-tetrahydroquinoline tautomer and/or a salt thereof.

7. A compound according to claim 5, in which X represents a direct bond, $R_6$ represents lower alkyl having up to 7 carbon atoms, $R_7$ represents hydrogen or lower alkyl having up to and including 4 carbon atoms, the group $R_6$—C(=O)— being in the 6-position and the lower alkyl group $R_7$ in the 7-position, $R_8$ and $R_{9a}$ represent hydroxy, and $R_{10}$ represents lower alkyl or lower alkenyl having up to and including 4 carbon atoms, or the 2-oxo-4-hydroxy-1,2-dihydroquinoline or 2,4-dioxo-1,2,3,4-tetrahydroquinoline tautomer and/or a salt thereof.

8. A compound according to claim 5, in which X represents oxy, $R_6$ represents hydrogen or lower alkyl having up to 7 carbon atoms, $R_7$ represents hydrogen or lower alkyl having up to and including 4 carbon atoms, the group $R_6$—O—C(=O)— being in the 6-position and the lower alkyl group $R_7$ in the 7-position, $R_8$ and $R_{9a}$ are as defined in claim 5, and $R_{10}$ represents lower alkyl or lower alkenyl having up to and including 4 carbon atoms, or the 2-oxo-4-hydroxy-1,2-dihydroquinoline or 2,4-dioxo-1,2,3,4-tetrahydroquinoline tautomer and/or a salt thereof.

9. 6-Butyryl-4-hydroxy-1,7-dimethylcarbostyryl-3-carboxylic acid ethyl ester according to claim 1.

10. 6-Butyryl-4-hydroxy-7-methyl-1-propylcarbostyryl-3-carboxylic acid ethyl ester according to claim 1.

11. 1-Ethyl-6-butyryl-4-hydroxy-7-methylcarbostyryl-3-carboxylic acid ethyl ester according to claim 1.

12. 1-Allyl-6-butyryl-4-hydroxy-7-methylcarbostyryl-3-carboxylic acid ethyl ester according to claim 1.

13. 1-Ethyl-6-butyryl-4-hydroxycarbostyryl-3-carboxylic acid ethyl ester according to claim 1.

14. 1-Butyl-6-butyryl-4-hydroxy-7-methylcarbostyryl-3-carboxylic acid ethyl ester according to claim 1.

15. 1-Allyl-6-methoxycarbonyl-4-hydroxycarbostyryl-3-carboxylic acid ethyl ester according to claim 1.

16. 1-Allyl-6-carboxy-4-hydroxycarbostyryl-3-carboxylic acid ethyl ester according to claim 1 or a salt thereof.

17. 1-Ethyl-6-butyryl-4-hyroxy-7-methylcarbostyryl-3-carboxylic acid tert-butyl ester according to claim 1.

18. 1-Ethyl-6-butyryl-4-hydroxy-7-methylcarbostyryl-3-carboxylic acid according to claim 1 or salt thereof.

19. 6-Methoxycarbonyl-1-propargyl-4-hydroxycarbostyryl-3-carboxylic acid ethyl ester according to claim 1 or a salt thereof.

20. 1-Ethyl-6-butyryl-4-hydroxy-7-methylcarbostyryl-3-carboxamide according to claim 1.

21. 1-Ethyl-6-formyl-4-hydroxy-7-methylcarbostyryl-3-carboxylic acid ethyl ester according to claim 1.

22. 1-Ethyl-6-methoxycarbonyl-4-hydroxycarbostyryl-3-carboxylic acid ethyl ester according to claim 1.

23. 1-Ethyl-6-carboxy-4-hydroxycarbostyryl-3-carboxylic acid ethyl ester according to claim 1 or a salt thereof.

24. A compound according to any one of claims 1 to 23 for use in a prophylactic and/or therapeutic method of treating the animal or human body.

25. A compound according to any one of claims 1 to 23 for use according to claim 24 as antiallergic agent.

26. A pharmaceutical composition containing a compound according to any one of claims 1, 4 to 6 and 13 to 23, which compound may be in the form of a pharmaceutically acceptable salt, together with customary pharmaceutical adjuvants and carriers.

27. A pharmaceutical composition containing a compound according to any one of claims 2, 3 and 7 to 12, which compound may be in the form of a pharmaceutically acceptable salt, together with customary pharmaceutical adjuvants and carriers.

28. A process for the preparation of a novel quinoline derivative of the formula I according to claim 1, or a salt thereof, characterised in that a compound of the formula

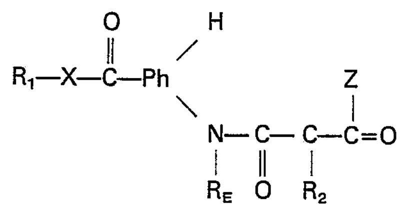

(IV)

in which Z is a removable radical, or a tautomer and/or a salt thereof, is subjected to intramolecular cyclisation, or, a compound of the formula

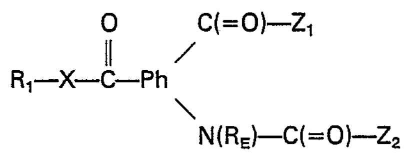

(VI)

in which one of the radicals $Z_1$ and $Z_2$ represents a group of the formula $CH_2$—$R_2$ and the other represents a free or reactively etherified or esterified hydroxy group, or a tautomer and/or salt thereof, is subjected to intramolecular cyclisation, or a compound of the formula

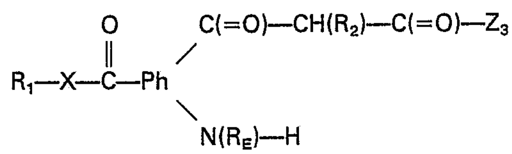

(IX)

in which $Z_3$ represents hydroxy or a reactive modified hydroxy group, or a tautomer and/or salt thereof, is subjected to intramolecular condensation, or, in a compound of the formula

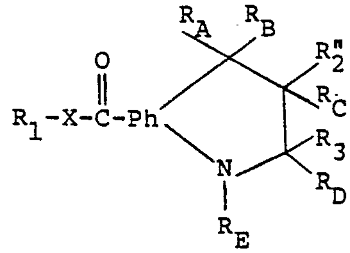

(XI),

in which $R_2''$ represents a radical which can be converted into the free, esterified or amidated carboxy group $R_2$, or in a tautomer and/or salt thereof, the group $R_2''$ is converted into the free, esterified or amidated carboxy group $R_2$, or, in a compound of the formula

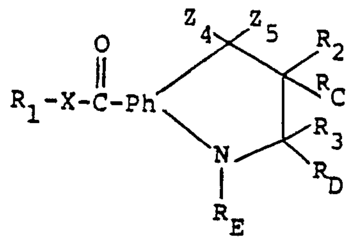

(XIII),

in which $Z_4$ represents a radical which can be converted into hydroxy or lower alkyl, and $Z_5$ together with $R_C$ and $R_D$ together with $R_E$ represent an additional bond in each case, or in which $Z_4$ and $Z_5$ each represent a monovalent, or together represent a divalent, radical which can be converted into oxo and $R_C$ together with $R_D$ represents an additional bond, or in a salt thereof, a radical $Z_4$ which can be converted into hydroxy or lower alkyl is converted into hydroxy or lower alkyl, or radicals $Z_4$ and $Z_5$ which can be converted into oxo are together converted into oxo, or in a compound of the formula

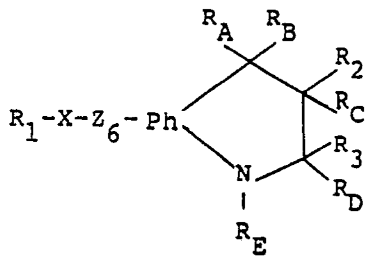

(XIX),

in which $Z_6$ represents a radical which can be converted into the carbonyl group, or in a salt thereof, $Z_6$ is converted into the carbonyl group, or, in a compound of the formula

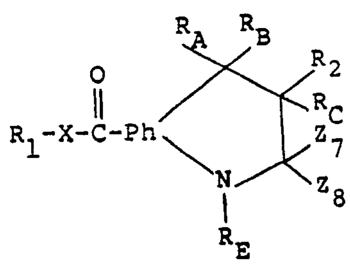

(XXI),

in which $Z_7$ represents a radical which can be converted into hydroxy and $Z_8$ represents a radical $R_D$, or $Z_7$ and $Z_8$ each represent a monovalent, or together represent a divalent, radical which can be converted into oxo, $Z_7$ is converted into hydroxy or $Z_7 + Z_8$ are converted into oxo, or for the preparation of a compound of the formula I, wherein X represents oxy, in a compound of the formula

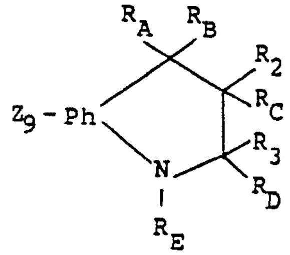

(XXIII),

in which $Z_9$ represents a functionally modified carboxy group other than the group $R_1$—X—C(=O)—, $Z_9$ is solvolysed to a corresponding group $R_1$—O—C(=O)—, or for the preparation of a compound of the formula I, wherein X represents a direct bond, a compound of the formula

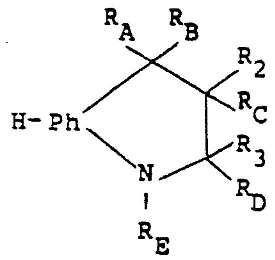

(XXV),

is reacted in the presence of a Lewis acid with a compound of the formula $R_1$—COOH (XXVI) or a suitable functional derivative thereof, or for the preparation of a compound of the formula I, wherein X represents a direct bond, a compound of the formula XXVII

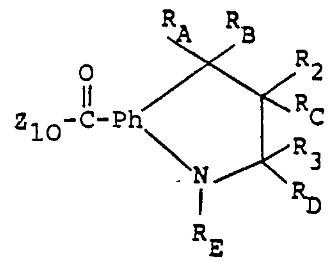

(XXVII),

in which $Z_{10}$ represents a free, etherified or reactively esterified hydroxyl group or a suitable heteroaryl group bonded via a ring nitrogen atom, or a salt thereof, is reacted with a compound of the formula $R_1$—M (XXVIII) in which M represents a metal radical, to form a corresponding compound of the formula I, wherein $R_1$ differs from hydrogen, or a compound of the formula XXVII, wherein $Z_{10}$ represents reactively esterified hydroxy, is reacted with bis-(triphenylphosphino)-copper boranate to form a compound of the formula I, wherein $R_1$ represents hydrogen, and, if desired, the resultant compound is converted into a different compound of the formula I and/or a resultant salt-forming compound is converted into a salt or a resultant salt is converted into the free compound or into a different salt.

29. A compound of the formula

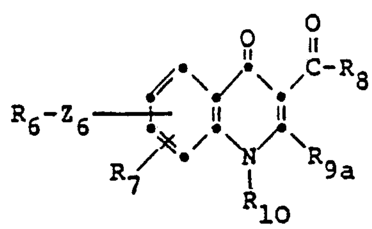

(XIXa),

in which $Z_6$ represents hydroxymethylene and the group $R_6$ represents lower alkyl having up to 7 carbon atoms, $R_7$ represents hydrogen or lower alkyl having up to and including 4 carbon atoms, the group $R_6$—$Z_6$ being in the 6- and a lower alkyl group $R_7$ in the 7-position, $R_8$ represents hydroxy or lower alkoxy having up to and including 4 carbon atoms, $R_{9a}$ represents hydroxy, and $R_{10}$ represents lower alkyl or lower alkenyl having up to and including 4 carbon atoms, or the tautomeric 2-oxo-4-hydroxy-1,2-dihydro- or 2,4-dioxo-1,2,3,4-tetrahydroquinoline form and/or a salt thereof.

30. 1-Ethyl-4-hydroxy-6-(1-hydroxybutyl)-7-methylcarbostyryl-3-carboxylic acid ethyl ester according to claim 29.

31. A compound according to either of claims 29 or 30 for use in a prophylactic and/or therapeutic method of treating the animal or human body.

32. A compound according to either of claims 29 or 30 for use according to claim 31 as antiallergic agent.

33. A pharmaceutical composition containing a compound according to either of claims 29 or 30, which compound may be in the form of a pharmaceutically acceptable salt, together with conventional pharmaceutical adjuvants or carriers.

34. A process for the preparation of a quinoline derivative of formula XIXa according to claim 29, or of a salt thereof, characterised in that a compound of the formula

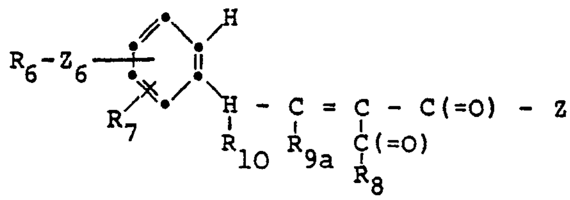

(IVa),

in which Z is a removable radical, or a tautomer and/or salt thereof, is subjected to intramolecular cyclisation, or a compound of the formula

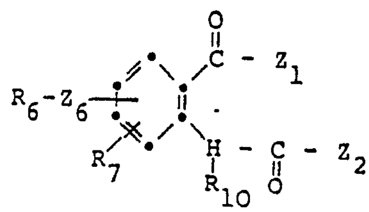

(VIa),

in which one of the radicals $Z_1$ and $Z_2$ represents a group of the formula $CH_2—C(=O)—R_8$ and the other represents a free or reactively etherified or esterified hydroxy group, or a tautomer and/or salt thereof, is subjected to intramolecular cyclisation, or a compound of the formula

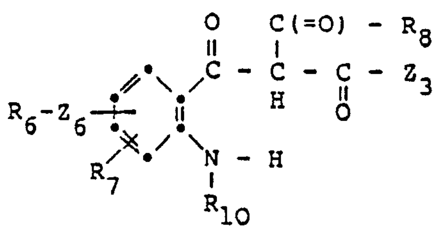

(IXa),

in which $Z_3$ represents hydroxy or a reactive modified hydroxy group, or a tautomer and/or salt thereof, is subjected to intramolecular condensation, or, in a compound of the formula

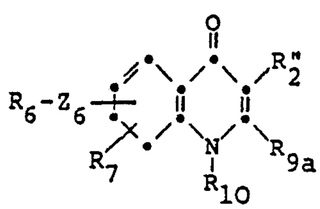

(XIa),

in which $R_1''$ represents a radical which can be converted into the free, esterified or amidated carboxy group $—C(=O)R_8$, or, in a tautomer and/or salt thereof, the group $R_2''$ is converted into the free, esterified or amidated carboxy group $—C(=O)—R_8$, or, in a compound of the formula

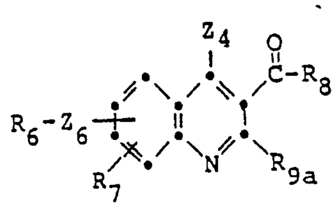

(XIIIa),

or

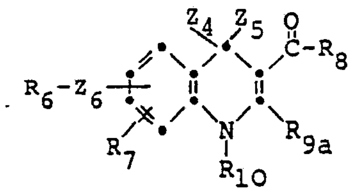

(XIIIb),

in which formula XIIIa $Z_4$ represents a radical which can be converted into hydroxy, or in which formula XIIIb $Z_4$ and $Z_5$ each represent a monovalent, or together represent a divalent, radical which can be converted into oxo, or in a salt thereof, a radical $Z_4$ which can be converted into hydroxy is converted into hydroxy, or radicals $Z_4$ and $Z_5$ which can be converted into oxo are together converted into oxo, or, in a compound of the formula

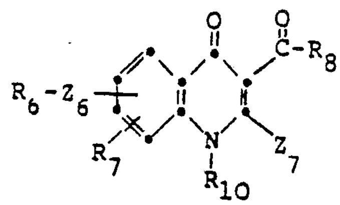

(XXIa),

or

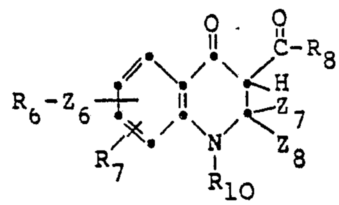

(XXIb),

in which formula XXIa $Z_7$ represents a radical which can be converted into hydroxy, or in which formula XXIb $Z_7$ and $Z_8$ each represent a monovalent, or together represent a divalent, radical which can be converted into oxo, $Z_7$ is converted into hydroxy or $Z_7 + Z_8$ are converted into oxo, or a compound of the formula

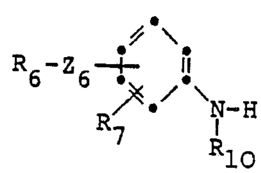

(Va)

is condensed with a compound of the formula

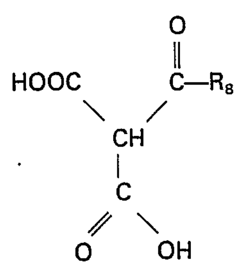

(XXa)

or a functional carboxy derivative or salt theroef, or, in a compound of the formula

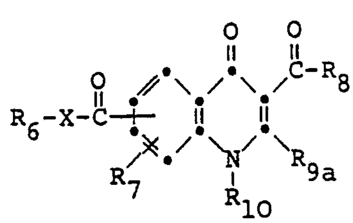

(II),

in which X represents a direct bond and $R_6$ represents lower alkyl having up to and including 7 carbon atoms, the group of the formula $R_6$—X—C(=O) is, by reaction with a suitable reducing agent such as a di-light metal hydride, for example sodium boronate or sodium cyanoboranate, or with a secondary alcohol such as a secondary lower alkanol or a cycloalkanol, for example with isopropanol or cyclohexanol, in the presence of an aluminium alcoholate, for example aluminium isopropanolate or cyclohexanolate, reduced to a group of the formula $R_6$—$Z_6$, or an aldehyde of the formula

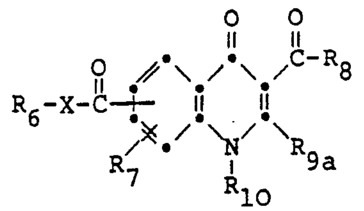

(II),

in which $R_6$ represents hydrogen and X represents a direct bond, is reacted with a metal derivative of a lower alkane having up to and including 7 carbon atoms of the formula $R_6$—H such as halomagnesium or lithium compound, e.g. with a lower alkyl lithium, and, if desired, in a compound of the formula XIXa which is obtainable by the process of this invention, lower alkoxycarbonyl —C(=O)—$R_8$ is solvolysed to carboxy, carboxy —C(=O)—$R_8$ is esterified to lower alkoxycarbonyl and/or a salt which is obtainable by the process of the invention is converted into the free compound or a free compound which is obtainable by the process of this invention is converted into a salt.

35. A compound which is obtainable by a process according to any one of claims 28, 29 and 34.

36. Use of a compound according to any one of claims 1 to 25 and 29 to 32 as medicament or for the preparation of medicaments by non-chemical methods.

**Claims for the Contracting State: AT**

1. A process for the preparation of a quinoline derivative of the formula

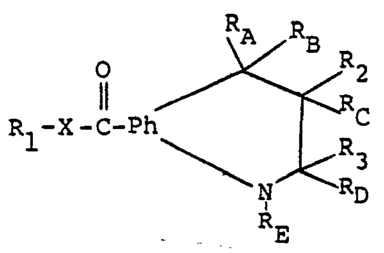

(I),

in which $R_1$ represents lower alkyl, lower alkanoyloxymethyl, lower alkenyl or cycloalkyl, each of which is unsubstituted or substituted by hydroxy, lower alkoxy, hydroxy-lower alkoxy, lower alkoxy-lower alkoxy, hydroxy-lower alkoxy-lower alkoxy, lower alkoxy-lower alkoxy-lower alkoxy, lower alkylthio, lower alkanesulphinyl or lower alkanesulphonyl; phenyl or phenyl-lower alkyl, each of which is unsubstituted or substituted in the phenyl moiety by lower alkyl, lower alkoxy and/or halogen; furyl, thienyl or pyridyl, or furyl-lower alkyl, thienyl-lower alkyl or pyridyl-lower alkyl or hydrogen, X represents a direct bond or oxy, Ph represents 1,2-phenylene which may be additionally substituted by lower alkyl, lower alkoxy, hydroxy and/or halogen, $R_2$ represents carboxy, lower alkoxycarbonyl, hydroxy-lower alkoxycarbonyl, lower alkoxy-lower alkoxycarbonyl, di-lower alkylamino-lower alkoxy- or 5- to 7-membered lower alkyleneamino- or aza-, oxa- or thia-lower alkylene-amino-lower alkoxycarbonyl or amidated carboxy containing, as amino group, amino, hydroxyamino, lower alkylamino, di-lower alkylamino or 5- to 7-membered lower alkylene- or aza-, oxa- or thia-lower alkyleneamino, and in which either $R_A$ and $R_B$ together represent oxo, $R_C$ and $R_D$ together represent an additional bond and $R_3$ represents hydroxy, or $R_C$ is hydrogen and $R_3$ and $R_D$ together represent oxo, and $R_E$ represents hydrogen, lower alkyl, hydroxy-lower alkyl, lower alkoxy-lower alkyl, di-lower alkylamino-lower alkyl, 5- to 7-membered lower alkyleneamino- or aza-, oxa- or thia-lower alkyleneamino-lower alkyl, lower alkenyl, lower alkynyl, cycloalkyl having 3 to 8, especially 5 to 7, ring members; phenyl-lower alkyl, furyl-lower alkyl, thienyl-lower alkyl or pyridyl-lower alkyl, each of which is unsubstituted or substituted by lower alkyl, lower alkoxy and/or halogen, or $R_A$ represents hydroxy or lower alkoxy, $R_B$ together with $R_C$ and also $R_D$ together with $R_E$ represent an additional bond in each case and $R_3$ represents hydroxy, or $R_B$ and $R_C$ together represent an additional bond and $R_3$ and $R_D$ together represent oxo, and $R_E$ represents hydrogen, lower alkyl, hydroxy-lower alkyl, lower alkoxy-lower alkyl, di-lower alkylamino-lower alkyl, 5- to 7-membered lower alkyleneamino- or aza-, oxa- or thia-lower alkyleneamino-lower alkyl, lower alkenyl, lower alkynyl, cycloalkyl having 3 to 8 ring members; phenyl-lower alkyl, furyl-lower alkyl, thienyl-lower alkyl or pyridyl-lower alkyl, each of which is substituted by lower alkyl, lower

alkoxy and/or halogen, with radicals qualified by the term "lower" containing up to and including 7 carbon atoms, or a salt thereof, characterised in that a compound of the formula

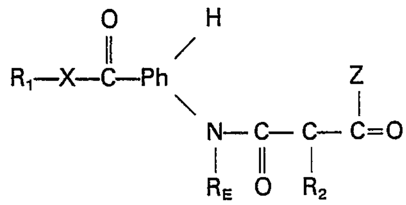

(IV)

in which Z is a removable radical, or a tautomer and/or a salt thereof, is subjected to intramolecular cyclisation, or, a compound of the formula

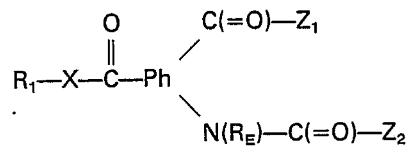

(VI)

in which one of the radicals $Z_1$ and $Z_2$ represents a group of the formula $CH_2—R_2$ and the other represents a free or reactively etherified or esterified hydroxy group, or a tautomer and/or salt thereof, is subjected to intramolecular cyclisation, or a compound of the formula

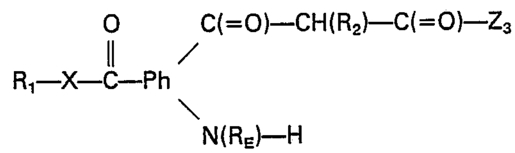

(IX)

in which $Z_3$ represents hydroxy or a reactive modified hydroxy group, or a tautomer and/or salt thereof, is subjected to intramolecular condensation, or, in a compound of the formula

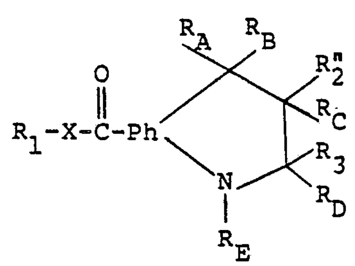

(XI),

in which $R_2''$ represents a radical which can be converted into the free, esterified or amidated carboxy group $R_2$, or in a tautomer and/or salt thereof, the group $R_2''$ is converted into the free, esterified or amidated carboxy group $R_2$, or, in a compound of the formula

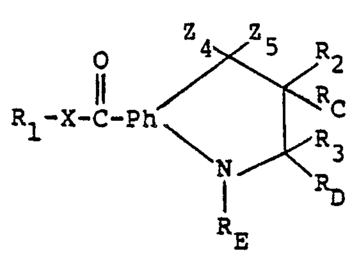

(XIII),

in which $Z_4$ represents a radical which can be converted into hydroxy or lower alkyl, and $Z_5$ together with $R_C$ and $R_D$ together with $R_E$ represent an additional bond in each case, or in which $Z_4$ and $Z_5$ each represent a monovalent, or together represent a divalent, radical which can be converted into oxo and $R_C$ together with $R_D$ represents an additional bond, or in a salt thereof, a radical $Z_4$ which can be converted into hydroxy or lower alkyl is converted into hydroxy or lower alkyl, or radicals $Z_4$ and $Z_5$ which can be converted into oxo are together converted into oxo, or in a compound of the formula

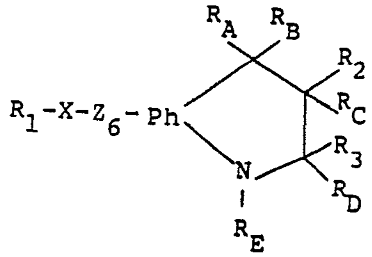

(XIX),

in which $Z_6$ represents a radical which can be converted into the carbonyl group, or in a salt thereof, $Z_6$ is converted into the carbonyl group, or, in a compound of the formula

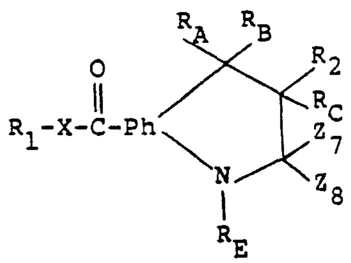

(XXI),

in which $Z_7$ represents a radical which can be converted into hydroxy and $Z_8$ represents a radical $R_D$, or $Z_7$ and $Z_8$ each represent a monovalent, or together represent a divalent, radical which can be converted into oxo, $Z_7$ is converted into hydroxy or $Z_7 + Z_8$ are converted into oxo, or for the preparation of a compound of the formula I, wherein X represents oxy, in a compound of the formula

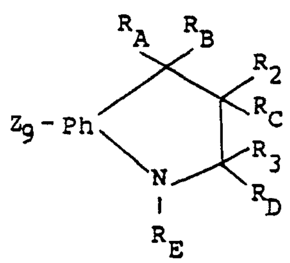

(XXIII),

in which $Z_9$ represents a functionally modified carboxy group other than the group $R_1—X—C(=O)—$, $Z_9$ is solvolysed to a corresponding group $R_1—O—C(=O)—$, or for the preparation of a compound of the formula I, wherein X represents a direct bond, a compound of the formula

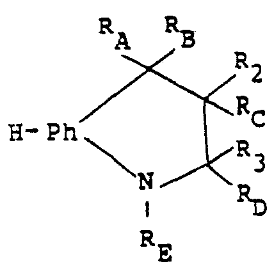

(XXV),

is reacted in the presence of a Lewis acid with a compound of the formula $R_1—COOH$ (XXVI) or a suitable functional derivative thereof, or for the preparation of a compound of the formula I, wherein X represents a direct bond, a compound of the formula XXVII

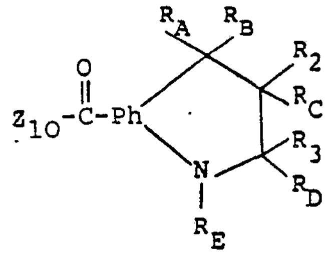

(XXVII),

in which $Z_{10}$ represents a free, etherified or reactively esterified hydroxyl group or a suitable heteroaryl group bonded via a ring nitrogen atom, or a salt thereof, is reacted with a compound of the formula $R_1$—M (XXVIII) in which M represents a metal radical, to form a corresponding compound of the formula I, wherein $R_1$ differs from hydrogen, or a compound of the formula XXVII, wherein $Z_{10}$ represents reactively esterified hydroxy, is reacted with bis-(triphenylphosphino)-copper boranate to form a compound of the formula I, wherein $R_1$ represents hydrogen, and, if desired, the resultant compound is converted into a different compound of the formula I and/or a resultant salt-forming compound is converted into a salt or a resultant salt is converted into the free compound or into a different salt.

2. A process according to claim 1, characterised by preparing a compound of the formula

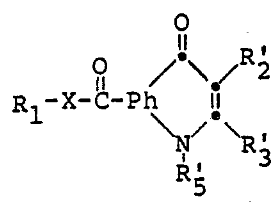

(Ia),

in which $R_1$ represents lower alkyl having up to 7 carbon atoms; lower alkoxy-, lower alkylthio-, lower alkanesulphinyl- or lower alkanesulphonyl-lower alkyl, each having up to 4 carbon atoms in the alkyl moiety; lower alkenyl having up to 4 carbon atoms; 3- to 8-membered cycloalkyl; phenyl or phenyl-lower alkyl having up to 4 carbon atoms in the alkyl moiety, each of which is unsubstituted or substituted by lower alkyl having up to 4 carbon atoms, lower alkoxy having up to 4 carbon atoms and/or halogen having an atomic number of up to and including 35; furyl, thienyl or pyridyl, or furyl-, thienyl- or pyridyl-lower alkyl having up to 4 carbon atoms in the alkyl moiety, and X represents a direct bond, or $R_1$ represents hydrogen or lower alkyl having up to 7 carbon atoms and X represents oxy, and in which Ph represents 1,2-phenylene which contains the group $R_1$—X—C(=O)— and may be additionally substituted by lower alkyl having up to 4 carbon atoms, lower alkoxy having up to 4 carbon atoms, hydroxy or halogen having an atomic number of up to and including 35, $R_2'$ represents carboxy, lower alkoxycarbonyl having up to 5 carbon atoms, hydroxy-lower alkoxycarbonyl having up to 5 carbon atoms, lower alkoxy-lower alkoxycarbonyl having up to 4 carbon atoms in the alkoxy moieties, di-lower alkylamino-lower alkoxycarbonyl having up to 4 carbon atoms in the alkyl moiety and up to 4 carbon atoms in the alkoxy moiety, carbamoyl, N-hydroxy-carbamoyl or N-lower alkyl- or N,N-di-lower alkyl-carbamoyl having up to 4 carbon atoms in the alkyl moiety, $R_3'$ represents hydroxy and $R_5'$ represents hydrogen; lower alkyl or lower alkenyl having up to 4 carbon atoms; phenyl- furyl-, thienyl- or pyridyl-lower alkyl which have up to 4 carbon atoms in the alkyl moiety and each of which is unsubstituted or substituted by lower alkyl having up to 4 carbon atoms, lower alkoxy having up to 4 carbon atoms and/or halogen having an atomic number of up to and including 35; lower alkoxy-lower alkyl having up to 4 carbon atoms in the alkoxy moiety and up to 4 carbon atoms in the alkyl moiety; or di-lower alkylamino-lower alkyl in which lower alkyl has up to 4 carbon atoms, or a tautomer and/or a salt thereof.

3. A process according to claim 1, characterised by preparing a compound of the formula

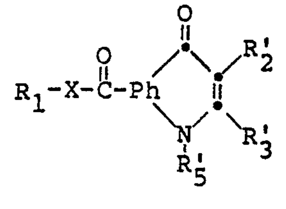

(Ia),

in which $R_1$ represents hydrogen; lower alkyl having up to 7 carbon atoms; lower alkoxy-, lower alkylthio-, lower alkanesulphinyl- or lower alkanesulphonyl-lower alkyl, each having up to 4 carbon atoms in the alkyl moiety; lower alkenyl having up to 4 carbon atoms; or 3- to 8-membered cycloalkyl, and X represents a direct bond, or $R_1$ represents hydrogen; lower alkyl having up to 7 carbon atoms; lower

alkoxy-, lower alkylthio-, lower alkanesulphinyl- or lower alkanesulphonyl-lower alkyl, each having up to 4 carbon atoms in the alkyl moiety; lower alkenyl having up to 4 carbon atoms; or 3- to 8-membered cycloalkyl; lower alkanoyloxymethyl having up to and including 7 carbon atoms in the alkanoyl moiety; or ω-(ω-hydroxy-lower alkoxy)-lower alkyl, ω-(ω-lower alkoxy-lower alkoxy)-lower alkyl, ω-[ω-(ω-hydroxy-lower alkoxy)-lower alkoxy]-lower alkyl or ω-[ω-(ω-lower alkoxy-lower alkoxy)-lower alkoxy]-lower alkyl, each having from 1 to 4 carbon atoms in the alkyl moiety and from 2 to 4 carbon atoms in the alkylene moiety, and X represents oxy, Ph represents 1,2-phenylene which contains the group $R_1$—X—C(=O)— and may be additionally substituted by lower alkyl having up to 4 carbon atoms, lower alkoxy having up to 4 carbon atoms, hydroxy or halogen having an atomic number of up to and including 35, $R_2'$ represents carboxy, lower alkoxycarbonyl having up to 5 carbon atoms, lower alkoxy-lower alkoxycarbonyl having up to 4 carbon atoms in the alkoxy moieties, carbamoyl, N-hydroxycarbamoyl or N-lower alkyl- or N,N-di-lower alkyl-carbamoyl having up to 4 carbon atoms in the alkyl moiety , $R_3'$ represents hydroxy, and $R_5'$ represents hydrogen, lower alkyl or lower alkenyl or lower alkynyl having up to 4 carbon atoms, or a tautomer thereof in which a hydroxyl group $R_3'$ is in the tautomeric oxo form, or a salt and/or a tautomer thereof.

4. A process according to claim 1, characterised by preparing a compound of the formula

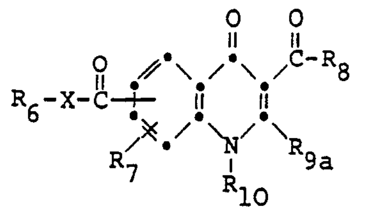

(II),

in which X represents a direct bond and $R_6$ represents hydrogen or lower alkyl having up to 7 carbon atoms, or X represents oxy and $R_6$ represents hydrogen, lower alkyl having up to and including 7 carbon atoms, ω-hydroxy-lower alkyl having from 2 to 4 carbon atoms, lower alkanoyloxymethyl having up to and including 7 carbon atoms in the alkanoyl moiety, or ω-(ω-hydroxy-lower alkoxy)-lower alkyl, ω-(ω-lower alkoxy-lower alkoxy)-lower alkyl, ω-[ω-(ω-hydroxy-lower alkoxy)-lower alkoxy.]-lower alkyl or ω-[ω-(ω-lower alkoxy-lower alkoxy)-lower alkoxy]-lower alkyl, each having from 1 to 4 carbon atoms in the alkyl moiety and 2 to 4 carbon atoms in the alkylene moiety, $R_7$ represents hydrogen or lower alkyl having up to and including 4 carbon atoms, lower alkoxy having up to and including 4 carbon atoms, halogen having an atomic number of up to and including 35, or hydroxy, $R_8$ represents hydroxy or lower alkoxy having up to 4 carbon atoms, $R_{9a}$ represents hydrogen or hydroxy, and $R_{10}$ represents lower alkyl, lower alkenyl or lower alkynyl having up to 4 carbon atoms, it also being possible for a 2-hydroxy-4-oxo-1,4-dihydroquinoline compound of the formula II to be in the tautomeric 2-oxo-4-hydroxy-1,2-dihydro- or 2,4-dioxo-1,2,3,4-tetrahydroquinoline form, or a salt thereof.

5. A process according to claim 4, characterised by preparing a compound of formula II, in which X represents oxy, the group $R_6$—O—C(=O)— is bonded in the 6-position, $R_6$ represents hydrogen, lower alkyl having up to 7 carbon atoms, ω-hydroxy-lower alkyl having 2 to 4 carbon atoms, lower alkanoyloxymethyl having up to and including 7 carbon atoms in the alkyl moiety, or ω-(ω-hydroxy-lower alkoxy)-lower alkyl, ω-(ω-lower alkoxy-lower alkoxy)-lower alkyl, ω-[ω-ω-[ω-(ω-hydroxy-lower alkoxy)-lower alkoxy]-lower alkyl or ω-[ω-(ω-lower alkoxy-lower alkoxy)-lower alkoxy]-lower alkyl, each having 1 to 4 carbon atoms in the alkyl moiety and 2 to 4 carbon atoms in the alkylene moiety, $R_7$ represents hydrogen, and $R_{10}$ represents lower alkenyl having up to 4 carbon atoms, or X represents a direct bond, $R_6$ represents hydrogen or lower alkyl having up to 7 carbon atoms, $R_7$ represents lower alkyl having up to and including 4 carbon atoms, the group $R_6$—C(=O)— being in the 6-position and the lower alkyl group $R_7$ in the 7-position, and $R_{10}$ represents lower alkyl having up to and including 4 carbon atoms, and each of $R_8$ and $R_{9a}$ is as defined in claim 5, or the 2-oxo-4-hydroxy-1,2-dihydroquinoline or 2,4-dioxo-1,2,3,4-tetrahydroquinoline tautomer and/or a salt thereof.

6. A process according to claim 4, characterised by preparing a compound of formula II, in which X represents a direct bond, $R_6$ represents lower alkyl having up to 7 carbon atoms, $R_7$ represents hydrogen or lower alkyl having up to and including 4 carbon atoms, the group $R_6$—C(=O)— being in the 6-position and the lower alkyl group $R_7$ in the 7-position, $R_8$ and $R_{9a}$ represent hydroxy, and $R_{10}$ represents lower alkyl or lower alkenyl having up to and including 4 carbon atoms, or the 2-oxo-4-hydroxy-1,2-dihydroquinoline or 2,4-dioxo-1,2,3,4-tetrahydroquinoline tautomer and/or a salt thereof.

7. A process according to claim 4, characterised by preparing a compound of formula II, in which X represents oxy, $R_6$ represents hydrogen or lower alkyl having up to 7 carbon atoms, $R_7$ represents hydrogen or lower alkyl having up to and including 4 carbon atoms, the group $R_6$—O—C(=O)— being in the 6-position and the lower alkyl group $R_7$ in the 7-position, $R_8$ and $R_{9a}$ are as defined in claim 5, and $R_{10}$ represents a lower alkyl or lower alkenyl having up to and including 4 carbon atoms, or the 2-oxo-4-hydroxy-1,2-dihydroquinoline or 2,4-dioxo-1,2,3,4-tetrahydroquinoline tautomer and/or a salt thereof.

8. A process according to claim 1, characterised by preparing 6-butyryl-4-hydroxy-1,7-dimethylcarbostyril-3-carboxylic acid ethyl ester.

9. A process according to claim 1, characterised by preparing 6-butyryl-4-hyroxy-7-methyl-1-propylcarbostyryl-3-carboxylic acid ethyl ester.

10. A process according to claim 1, characterised by preparing 1-ethyl-6-butyryl-4-hydroxy-7-methylcarbostyryl-3-carboxylic acid ethyl ester.

11. A process according to claim 1, characterised by preparing 1-allyl-6-butyryl-4-hydroxy-7-methylcarbostyryl-3-carboxylic acid ethyl ester.

12. A process according to claim 1, characterised by preparing 1-ethyl-6-butyryl-4-hydroxycarbostyryl-3-carboxylic acid ethyl ester.

13. A process according to claim 1, characterised by preparing 1-butyl-6-butyryl-4-hydroxy-7-methylcarbostyryl-3-carboxylic acid ethyl ester.

14. A process according to claim 1, characterised by preparing 1-allyl-6-methoxycarbonyl-4-hydroxycarbostyryl-3-carboxylic acid ethyl ester.

15. A process according to claim 1, characterised by preparing 1-allyl-6-carboxy-4-hydroxycarbostyryl-3-carboxylic acid ethyl ester or a salt thereof.

16. A process according to claim 1, characterised by preparing 1-ethyl-6-butyryl-4-hydroxy-7-methylcarbostyryl-3-carboxylic acid tert-butyl ester.

17. A process according to claim 1, characterised by preparing 1-ethyl-6-butyryl-4-hydroxy-7-methylcarbostyryl-3-carboxylic acid or a salt thereof.

18. A process according to claim 1, characterised by preparing 6-methoxycarbonyl-1-propargyl-4-hydroxycarbostyryl-3-carboxylic acid ethyl ester or a salt thereof.

19. A process according to claim 1, characterised by preparing 1-ethyl-6-butyryl-4-hydroxy-7-methylcarbostyryl-3-carboxamide.

20. A process according to claim 1, characterised by preparing 1-ethyl-6-formyl-4-hydroxy-7-methylcarbostyryl-3-carboxylic acid ethyl ester.

21. A process according to claim 1, characterised by preparing 1-ethyl-6-methoxycarbonyl-4-hydroxycarbostyryl-3-carboxylic acid ethyl ester.

22. A process according to claim 1, characterised by preparing 1-ethyl-6-carboxy-4-hydroxycarbostyryl-3-carboxylic acid ethyl ester or a salt thereof.

23. A process for the preparation of a pharmaceutical composition, characterised in that a compound which is obtainable by a process according to any one of claims 1 to 22, which compound may be in the form of a pharmaceutically acceptable salt, is brought, by mixing with customary pharmaceutical adjuvants and carriers, into a form suitable for pharmaceutical administration.

24. A process according to claim 23 for the preparation of anti-allergic agents.

25. A process for the preparation of a novel quinoline derivative of the formula

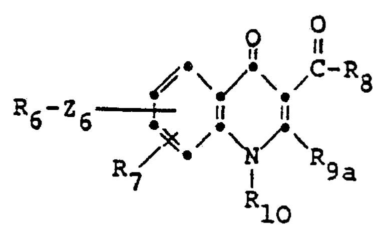

(XIXa),

in which $Z_6$ represents hydroxymethylene and the group $R_6$ represents lower alkyl having up to 7 carbon atoms, $R_7$ represents hydrogen or lower alkyl having up to and including 4 carbon atoms, the group $R_6$—$Z_6$ being in the 6- and a lower alkyl group $R_7$ in the 7-position, $R_8$ represents hydroxy or lower alkoxy having up to and including 4 carbon atoms, $R_{9a}$ represents hydroxy, and $R_{10}$ represents lower alkyl or lower alkenyl having up to and including 4 carbon atoms, or of the tautomeric 2-oxo-4-hydroxy-1,2-dihydro- or 2,4-dioxo-1,2,3,4-tetrahydroquinoline form and/or of a salt thereof, characterised in that a compound of the formula

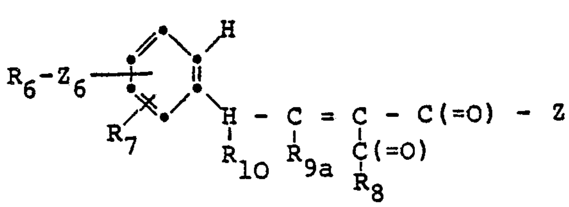

(IVa),

in which Z is a removable radical, or a tautomer and/or salt thereof, is subjected to intramolecular cyclisation, or a compound of the formula

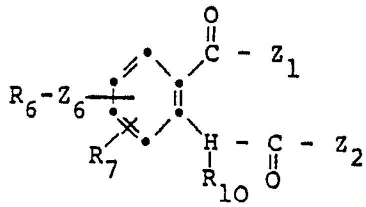

(VIa),

in which one of the radicals $Z_1$ and $Z_2$ represents a group of the formula $CH_2—C(=O)—R_8$ and the other represents a free or reactively etherified or esterified hydroxy group, or a tautomer and/or salt thereof, is subjected to intramolecular cyclisation, or a compound of the formula

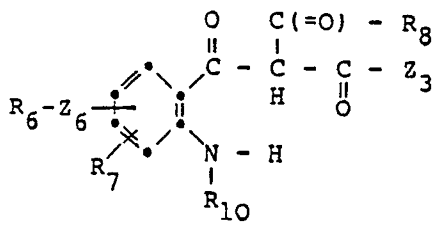

(IXa),

in which $Z_3$ represents hydroxy or a reactive modified hydroxy group, or a tautomer and/or salt thereof, is subjected to intramolecular condensation, or, in a compound of the formula

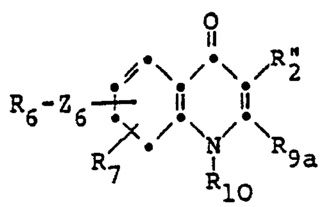

(XIa),

in which $R_2''$ represents a radical which can be converted into the free, esterified or amidated carboxy group $—C(=O)R_8$, or, in a tautomer and/or salt thereof, the group $R_2''$ is converted into the free, esterified or amidated carboxy group $—C(=O)—R_8$, or, in a compound of the formula

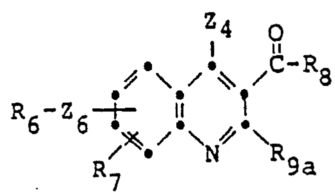

(XIIIa),

or

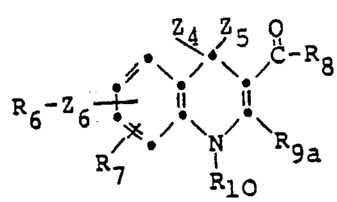

(XIIIb),

in which formula XIIIa $Z_4$ represents a radical which can be converted into hydroxy, or in which formula XIIIb $Z_4$ and $Z_5$ each represent a monovalent, or together represent a divalent, radical which can be converted into oxo, or in a salt thereof, a radical $Z_4$ which can be converted into hydroxy is converted into hydroxy, or radicals $Z_4$ and $Z_5$ which can be converted into oxo are together converted into oxo, or, in a compound of the formula

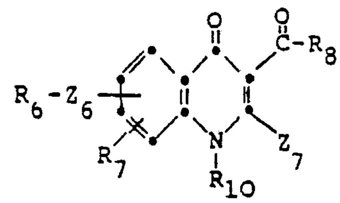

(XXIa),

or

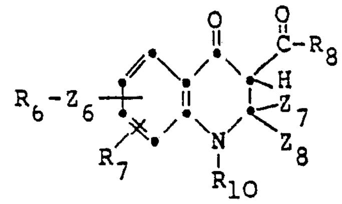

(XXIb),

in which formula XXIa $Z_7$ represents a radical which can be converted into hydroxy, or in which formula XXIb $Z_7$ and $Z_8$ each represent a monovalent, or together represent a divalent, radical which can be converted into oxo, $Z_7$ is converted into hydroxy or $Z_7 + Z_8$ are converted into oxo, or a compound of the formula

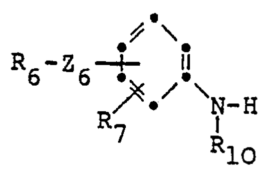

(Va)

is condensed with a compound of the formula

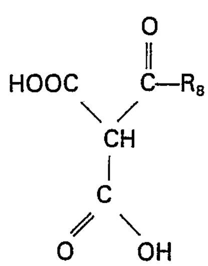

(XXa)

or a functional carboxy derivative or salt theroef, or, in a compound of the formula

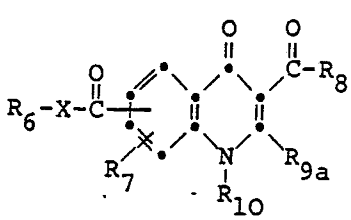

(II),

in which X represents a direct bond and $R_6$ represents lower alkyl having up to and including 7 carbon atoms, the group of the formula $R_6—X—C(=O)$ is, by reaction with a suitable reducing agent such as a di-light metal hydride, for example sodium boronate or sodium cyanoboranate, or with a secondary alcohol such as a secondary lower alkanol or a cycloalkanol, for example with isopropanol or cyclohexanol, in the presence of an aluminium alcoholate, for example aluminium isopropanolate or cyclohexanolate, reduced to a group of the formula $R_6—Z_6$, or an aldehyde of the formula

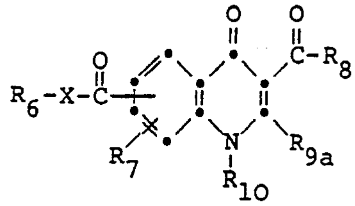

(II),

in which $R_6$ represents hydrogen and X represents a direct bond, is reacted with a metal derivative of a lower alkane having up to and including 7 carbon atoms of the formula $R_6$—H such as halomagnesium or lithium compound, e.g. with a lower alkyl lithium, and, if desired, in a compound of the formula XIXa which is obtainable by the process of this invention, lower alkoxycarbonyl —C(=O)—$R_8$ is solvolysed to carboxy, carboxy —C(=O)—$R_8$ is esterified to lower alkoxycarbonyl and/or a salt which is obtainable by the process of the invention is converted into the free compound or a free compound which is obtainable by the process of this invention is converted into a salt.

26. A process according to claim 25, characterised by preparing 1-ethyl-4-hydroxy-6-(1-hydroxybutyl)-7-methylcarbostyryl-3-carboxylic acid ester.

27. A process for the preparation of a pharmaceutical composition, characterised in that a compound which is obtainable by a process according to either of claims 25 and 26, which compound may be in the form of a pharmaceutically acceptable salt, is brought, by mixing with customary pharmaceutical adjuvants and carriers, into a form suitable for pharmaceutical administration.

**Revendications pour les Etats contractants: DE FR CH LI IT NL BE SE LU**

1. Dérivé de quinolinone de formule:

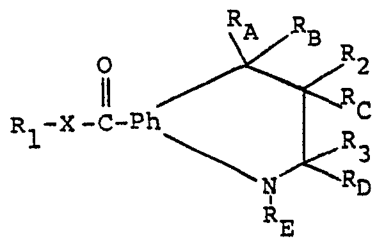

(I),

où $R_1$ représente un alcoyle inférieur éventuellement substitué par un hydroxy, alcoxy inférieur, hydroxyalcoxy inférieur, alcoxy inférieur-alcoxy inférieur, hydroxyalcoxy inférieur-alcoxy inférieur, alcoxy inférieur-alcoxy inférieur, alcoyle inférieur-thio, alcane inférieur sulfinyle ou alcane inférieur-sulfonyle, un alcanoyloxyméthyle inférieur, un alcényle inférieur ou cycloalcoyle, un phényle ou phénylalcoyle inférieur éventuellement substitué dans la fraction phényle par un alcoyle inférieur, un alcoxy inférieur, et/ou un halogène, un furyle, thiényle ou pyridyle ou furylalcoyle inférieur, thiénylalcoyle inférieur, pyridylalcoyle inférieur ou hydrogène, X représente une liaison directe ou un oxy, Ph représente un 1,2-phénylène éventuellement substitué en outre par un alcoyle inférieur, alcoxy inférieur, hydroxy et/ou halogène, $R_2$ représente un carboxy, alcoxycarbonyle inférieur, hydroxycarbonyle inférieur, alcoxy inférieur-alcoxy inférieur-carbonyle, dialcoyle inférieur-aminoalcoxy inférieur, ou alcoylène-mino inférieur à 5 ou 6 chaînons ou, selon les cas, asa- oxa- ou thiaalcoylène inférieur-aminoalcoxy inférieur-carbonyle ou un carboxy amidé, qui présente comme groupe amino un amino, hydroxy amino, alcoyle inférieur-amino, dialcoyle inférieur-amino ou alcoylène inférieur à 5 ou 6 chaînons -ou selon les cas asa, oxa-, thiaalcoylène-amino, et où ou bien $R_A$ et $R_B$ représentent ensemble un oxo, $R_C$ et $R_D$ représentent ensemble une liaison supplémentaire et $R_3$ est un hydroxy ou selon les cas $R_C$ est un hydrogène et $R_3$ et $R_D$ représentent ensemble un oxo et $R_E$ représente un hydrogène, alcoyle inférieur, hydroxy-alcoyle inférieur, alcoxy inférieur, alcoyle inférieur, dialcoyle inférieur-amino-alcoyle inférieur, alcoylène inférieur-amino à 5 à 7 chaînons -ou selon les cas asa-, oxa- ou thiaalcoylène-inférieur amino-alcoyle inférieur, alcényle inférieur, alcynyle inférieur, cycloalcoyle ayant de 3 à 8, de préférence de 5 à 7 chaînons ou un phénylalcoyle inférieur éventuellement substitué par un alcoyle inférieur, alcoxy inférieur et/ou halogène, un furylacoyle inférieur, thiénylalcoyle inférieur ou pyridylalcoyle inférieur, ou $R_A$ représente un hydroxy ou un alcoxy inférieur, $R_B$ représente avec $R_C$ ainsi que $R_D$ avec $R_E$ à chaque fois une liaison supplémentaire et $R_3$ est un hydroxy ou selon les cas $R_B$ et $R_C$ représentent ensemble une liaison supplémentaire et $R_3$ et $R_D$ représentent ensemble un oxo et $R_E$ représente un hydrogène, alcoyle inférieur, hydroxyalcoyle inférieur, alcoxy inférieur-alcoyle inférieur, dialcoyle inférieur-amino-alcoyle inférieur, alcoylène inférieur à 5 à 6 chaînons-amino -ou selon les cas asa-, oxa-, ou thiaalcoylène inférieur-amino-alcoyle inférieur, alcényle inférieur, alcynyle inférieur, cycloalcoyle à 3 à 8 chaînons ou phénylalcoyle inférieur éventuellement substitué par alcoyle inférieur, alcoxy inférieur et/ou halogène, furylalcoyle inférieur, thiénylalcoyle inférieur ou pyridylalcoyle inférieur,

où les radicaux "inférieur" présentent à chaque fois jusqu'à 7 atomes de carbone compris ou un de ses sels.

2. Composé selon la revendication 1, où X représente une liaison directe et $R_1$, $R_2$, $R_3$, $R_A$, $R_B$, $R_C$, $R_D$ et $R_E$ ont les significations données dans la revendication 1, $R_1$ est cependant différent d'un hydrogène, ou X représente un oxy, $R_1$ représente un alcoyle inférieur ou un phényle éventuellement substitué par un alcoyle inférieur, alcoxy inférieur, hydroxy et/ou halogène et $R_2$, $R_3$, $R_A$, $R_B$, $R_C$, $R_D$ et $R_E$ ont les significations données dans la revendication 1.

3. Composé selon la revendication 1 de formule

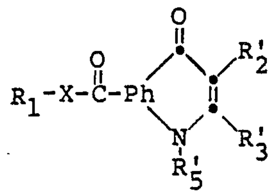

(Ia),

où $R_1$ représente un alcoyle inférieur ayant jusqu'à 7 atomes de carbone compris, un alcoxy inférieur-alcoyle inférieur-thio-, alcane inférieur-sulfinyle- ou alcane inférieur-sulfonyle-alcoyle inférieur ayant à chaque fois jusqu'à 4 atomes de carbone dans les parties alcoyle, un alcényle inférieur ayant jusqu'à 4 atomes de carbone compris, un cycloalcoyle à 3 à 8 chaînons, un phényle éventuellement substitué par un alcoyle inférieur ayant jusqu'à 4 atomes de carbone, un alcoxy inférieur ayant jusqu'à 4 atomes de carbone et/ou un halogène de numéro atomique allant jusqu'à 35 compris ou, selon les cas, un phénylalcoyle inférieur ayant jusqu'à 4 atomes de carbone dans la partie alcoyle, un furyle, thiényle ou pyridyle ou un furyl-, thiényl- ou pyridylalcoyle inférieur ayant jusqu'à 4 atomes de carbone compris dans la partie alcoyle et X représente un liaison directe ou $R_1$ représente un hydrogène ou un alcoyle inférieur ayant jusqu'à 7 atomes de carbone compris et X représente un oxy et où Ph représente un 1,2-phénylène présentant le groupe $R_1$—X—C(=O)— éventuellement substitué en outre par un alcoyle inférieur ayant jusqu'à 4 atomes de carbone compris, un alcoxy inférieur ayant jusqu'à 4 atomes de carbone compris, un hydroxy ou un halogène de numéro atomique allant jusqu'à 35 compris, $R'_2$ représente un carboxy, un alcoxy inférieur-carbonyle ayant jusqu'à 5 atomes de carbone compris, un hydroxy-alcoxy inférieur-carbonyle ayant jusqu'à 5 atomes de carbone compris, un alcoxy inférieur-alcoxy inférieur-carbonyle ayant jusqu'à 4 atomes de carbone dans les parties alcoyle, un dialcoyle inférieur-amino-alcoxy inférieur-carbonyle ayant à chaque fois jusqu'à 4 atomes de carbone dans la partie alcoyle et alcoxy, un carbamoyle, N-hydroxycarbamoyle ou N-alcoyle inférieur- ou selon les cas N,N-dialcoyle inférieur-carbamoyle ayant jusqu'à 4 atomes de carbone dans la partie alcoyle, $R'_3$ représente un hydroxy et $R'_5$ représente un hydrogène, un alcoyle inférieur ou un alcényle inférieur ayant jusqu'à 4 atomes de carbone compris, un phényle éventuellement substitué par un alcoyle inférieur ayant jusqu'à 4 atomes de carbone compris, un alcoxy inférieur ayant jusqu'à 4 atomes de carbone compris et/ou un halogène de numéro atomique allant jusqu'à 35 compris, un furyl-, thiényl- ou pyridyl-alcoyle inférieur ayant jusqu'à 4 atomes de carbone compris dans la partie alcoyle, un alcoxy inférieur-alcoyle inférieur ayant jusqu'à 4 atomes de carbone compris dans la partie alcoxy ou, selon les cas, alcoyle ou un dialcoyle inférieur-amino-alcoyle inférieur. où alcoyle inférieur présente jusqu'à 4 atomes de carbone compris, ou un de ses tautomères ou sels.

4. Composé selon la revendication 1 de formule

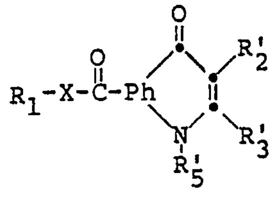

(Ia),

où $R_1$ représente un hydrogène, un alcoyle inférieur ayant jusqu'à 7 atomes de carbone compris, un alcoxy inférieur-, alcoyle inférieur-thio-, alcane inférieur-sulfinyle- ou alcane inférieur-sulfonyle-alcoyle inférieur ayant à chaque fois jusqu'à 4 atomes de carbone dans les parties alcoyle, un alcényle inférieur ayant jusqu'à 4 atomes de carbone compris ou un cycloalcoyle à 3 à 8 chaînons et X représente une liaison directe ou $R_1$ représente un hydrogène, un alcoyle inférieur ayant jusqu'à 7 atomes de carbone compris, un alcoxy inférieur-, alcoyle inférieur-thio-, alcane inférieur-sulfonyle- ou alcane inférieur-sulfinyle-alcoyle inférieur ayant à chaque fois jusqu'à 4 atomes de carbone compris dans les parties alcoyle, un alcényle inférieur ayant jusqu'à 4 atomes de carbone compris, ou un cycloalcoyle à 3 à 8 chaînons, un alcanoyloxy inférieur-méthyle ayant jusqu'à 7 atomes de carbone dans la partie alcanoyle ou un ω(ω-hydroxyalcoxy inférieur)-alcoyle inférieur, ω-(ω-alcoxy inférieur-alcoxy inférieur)-alcoyle inférieur, ω-[ω-hydroxyalcoxy inférieur)-alcoxy inférieur]- alcoyle inférieur ou ω-[ω-(ω-alcoxy inférieur-alcoxy inférieur)-alcoxy inférieur]-alcoyle inférieur ayant à chaque fois de 1 à 4 atomes de carbone dans la partie alcoyle et de 2 à 4 atomes de

carbone dans la partie alcoylène et X représente un oxy, Ph représente un 1,2-phénylène présentant le groupe $R_1$—X—C(=O)—, éventuellement substitué de façon additionnelle par un alcoyle inférieur ayant jusqu'à 4 atomes de carbone compris, un alcoxy inférieur ayant jusqu'à 4 atomes de carbone compris, un hydroxy ou un halogène dont le numéro atomique va jusqu'à 35 compris, $R'_2$ représente un carboxy, un alcoxy inférieur-carbonyle ayant jusqu'à 5 atomes de carbone compris, un alcoxy inférieure-alcoxy inférieur-carbonyle ayant jusqu'à 4 atomes de carbone compris dans les parties alcoyle, un carbamoyle, un N-hydroxycarbamoyle ou un N-alcoyle inférieur- ou, selon les cas, N,N-dialcoyle inférieur-carbamoyle ayant jusqu'à 4 atomes de carbone compris dans la partie alcoyle, $R'_3$ représente un hydroxy et $R'_5$ représente un hydrogène, un alcoyle inférieur, un alcényle inférieur ou un alcynyle inférieur ayant jusqu'à 4 atomes de carbone compris, ainsi que ses tautomères où le groupe hydroxy $R'_3$ se présente sous la forme tautomère-oxo, ou un sel d'un composé salificateur de formule (Ia) où d'un de ses tautomères.

5. Composé selon la revendication de formule

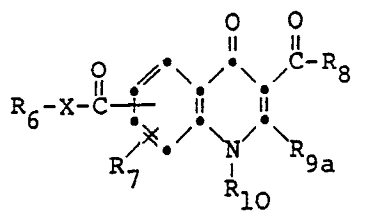

(II),

où X représente une liaison directe et $R_6$ représente un hydrogène ou un alcoyle inférieur ayant jusqu'à 7 atomes de carbone compris ou X représente un oxy et $R_6$ un hydrogène, un alcoyle inférieur ayant jusqu'à 7 atomes de carbone compris, un ω-hydroxy-alcoyle inférieur ayant de 2 à 4 atomes de carbone, un alcanoyloxy inférieur-méthyle ayant jusqu'à 7 atomes de carbone compris dans la partie alcanoyle ou un ω-(ω-hydroxyalcoxy inférieur)-alcoyle inférieur, ω-(ω-alcoxy inférieur-alcoxy inférieur)-alcoyle inférieur, ω-[ω-(ω-hydroxyalcoxy inférieur)-alcoxy inférieur]-alcoyle inférieur ou ω-[ω(ω-alcoxy inférieur-alcoxy inférieur)-alcoxy inférieur]-alcoyle inférieur avec à chaque fois de 1 à 4 atomes de carbone dans la partie alcoyle et de 2 à 4 atomes de carbone dans la partie alcoylène, $R_7$ représente un hydrogène, un alcoyle inférieur ayant jusqu'à 4 atomes de carbone compris, un alcoxy inférieur ayant jusqu'à 4 atomes de carbone compris, un halogène dont le numéro atomique va jusqu'à 35 ou un hydroxy, $R_5$ représente un hydroxy ou un alcoxy inférieur ayant jusqu'à 4 atomes de carbone compris, $R_{9a}$ représente un hydroxy, et $R_{10}$ représente un alcoyle inférieur, un alcényle inférieur ou un alcynyle inférieur avec jusqu'à 4 atomes de carbone compris, où un composé de 2-hydroxy-4-oxo-1,4-dihydroquinoléine de formule (II) peut également se présenter sous la forme tautomère 2-oxo-4-hydroxy-1,2-dihydro- ou 2,4-dioxo-1,2,3,4-tétrahydroquinoléine, ou un de ses sels.

6. Composé selon la revendication 5, où X représente un oxy, le groupe $R_6$—O—C(=O)— est lié en position 6, $R_6$ représente un hydrogène, un alcoyle inférieur ayant jusqu'à 7 atomes de carbone, un ω-hydroxyalcoyle inférieur ayant de 2 à 4 atomes de carbone, un alcanoyloxy inférieur-méthyle ayant jusqu'à 7 atomes de carbone dans la partie alcoyle ou un ω-(ω-hydroxy-alcoxy inférieur)-alcoyle inférieur, ω-(ω-alcoxy inférieur-alcoxy inférieur)-alcoyle inférieur, ω-[ω-ω-[ω(-hydroxyalcoxy inférieur)-alcoxy inférieur)-alcoyle inférieur ou ω-[ω-(ω-alcoxy inférieur-alcoxy inférieur)-alcoxy inférieur)-alcoyle inférieur avec à chaque fois de 1 à 4 atomes de carbone dans la partie alcoyle et de 2 à 4 atomes de carbone dans la partie alcoylène, $R_7$ représente un hydrogène et $R_{10}$ représente un alcényle inférieur ayant jusqu'à 4 atomes de carbone compris, ou X représente une liaison directe, $R_6$ représente un hydrogène ou un alcoyle inférieur ayant jusqu'à 7 atomes de carbone compris, $R_7$ représente un alcoyle inférieur ayant jusqu'à 4 atomes de carbone compris, où le groupe $R_6$—C(=O)— prend la position 6 et le groupe alcoyle inférieur $R_7$ prend la position 7, et $R_{10}$ représente un alcoyle inférieur ayant jusqu'à 4 atomes de carbone compris, où $R_8$ et $R_{9a}$ ont à chaque fois les significations données dans la revendication 5, ou selon les cas le tautomère 2-oxo-4-hydroxy-1,2-dihydroquinoléine ou 2,4-dioxo-1,2,3,4-tétrahydroquinoléine et/ou un de ses sels.

7. Composé selon la revendication 5, où X représente une liaison directe, $R_6$ représente un alcoyle inférieur ayant jusqu'à 7 atomes de carbone compris, $R_7$ représente un hydrogène ou un alcoyle inférieur ayant jusqu'à 4 atomes de carbone compris, où le groupe $R_6$—C(=O)— prend la position 6 et un groupe alcoyle $R_7$ prend la position 7, $R_8$ et $R_{9a}$ représentent un hydroxy et $R_{10}$ représente un alcoyle inférieur ou un alcényle inférieur ayant jusqu'à 4 atomes de carbone compris, ou, selon les cas, le tautomère de 2-oxo-4-hydroxy-1,2-dihydroquinoléine ou 2,4-dioxo-1,2,3,4-tétrahydroquinoléine et/ou un de ses sels.

8. Composé selon la revendication 5, où X représente un oxy, $R_6$ représente un hydrogène ou un alcoyle inférieur ayant jusqu'à 7 atomes de carbone compris, $R_7$ représente un hydrogène ou un alcoyle inférieur ayant jusqu'à 4 atomes de carbone compris, où le groupe $R_6$—O—C(=O)— prend la position 6 et un groupe alcoyle inférieur $R_7$ prend la position 7, $R_8$ et $R_{9a}$ ont les significations données dans la revendication 5, et $R_{10}$ représente un alcoyle inférieur ou un alcényle inférieur ayant jusqu'à 4 atomes de carbone compris, ou, selon les cas, le tautomère de 2-oxo-4-hydroxy-1,2-dihydroquinoléine ou 2,4-dioxo-1,2,3,4-tétrahydroquinoléine et/ou un de ses sels.

9. Ester éthylique de l'acide 6-butyryl-4-hydroxy-1,7-diméthyl-carbostiryl-3-carboxylique selon la revendication 1.

10. Ester éthylique de l'acide 6-butyryl-4-hydroxy-7-méthyl-1-propyl-carbostiryl-3-carboxylique selon la revendication 1.

11. Ester éthylique de l'acide éthyl-6-butyryl-4-hydroxy-7-méthyl-carbostiryl-3-carboxylique selon la revendication 1.

12. Ester éthylique de l'acide 1-allyl-6-butyryl-4-hydroxy-7-méthyl-carbostiryl-3-carboxylique selon la revendication 1.

13. Ester éthylique de l'acide éthyl-6-butyryl-4-hydroxy-carbostiryl-3-carboxylique selon la revendication 1.

14. Ester éthylique de l'acide 1-Butyl-6-butyryl-4-hydroxy-7-méthyl-carbostiryl-3-carboxylique selon la revendication 1.

15. Ester éthylique de l'acide 1-Allyl-6-méthoxy-carbonyl-4-hydroxy-carbostiryl-3-carboxylique selon la revendication 1.

16. Ester éthylique de l'acide 1-allyl-6-carboxy-4-hydroxy-carbostiryl-3-carboxylique selon la revendication 1, ou un de ses sels.

17. Tert.-butylester de l'acide 1-éthyl-6-butyryl-4-hydroxy-7-méthyl-carbostiryl-3-carboxylique selon la revendication 1.

18. Acide 1-éthyl-6-butyryl-4-hydroxy-7-méthyl-carbostiryl-3-carboxylique selon la revendication 1 ou un de ses sels.

19. Ester éthylique de l'acide 6-méthoxycarbonyl-1-propargyl-4-hydroxy-carbostiryl-3-carboxylique selon la revendication 1 ou un de ses sels.

20. 1-éthyl-6-butyryl-4-hydroxy-7-méthyl-carbostiryl-3-carboxylique selon la revendication 1.

21. Ester éthylique de l'acide 1-éthyl-6-formyl-4-hydroxy-7-méthyl-carbostyiryl-3-carboxylique selon la revendication 1.

22. Ester éthylique de l'acide 1-éthyl-6-méthoxy-carbonyl-4-hydroxy-carbostiryl-3-carboxylique selon la revendication 1.

23. Ester éthylique de l'acide 1-éthyl-6-carboxy-4-hydroxy-carbostiryl-3-carboxylique selon la revendication 1 ou un de ses sels.

24. Composé selon l'une des revendications 1 à 23, pour application dans un procédé de traitement prophylactique et/ou thérapeutique du corps animal ou humain.

25. Composés selon l'une des revendications 1 à 23, pour application selon la revendication 24 comme agent anti-allergique.

26. Préparations pharmaceutique contenant un composé selon l'une des revendications 1, 4 à 6 et 13 à 23, éventuellement sous forme de sels pharmaceutiquement acceptables, outre des additifs et supports pharmaceutiques habituels.

27. Préparations pharmaceutiques contenant un composé selon l'une des revendications 2, 3 et 7 à 12, éventuellement sous forme de sels pharmaceutiquement acceptables, outre des additifs et supports pharmaceutiques habituels.

28. Procédé de préparation de nouveaux dérivés de quinolinone de formule (I) selon la revendication 1 et de ses sels, caractérisé en ce qu'on cyclise de façon intramoléculaire un composé de formule

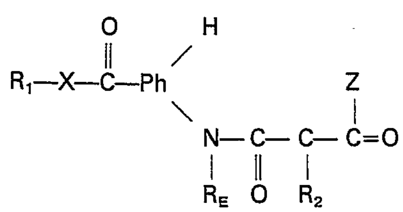

(IV)

où Z représente un radical séparable, ou un de ses tuatomères et/ou sels ou ce qu'on cyclise de façon intramoléculaire un composé de formule

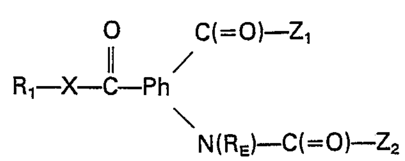

(VI)

où l'un des radicaux $Z_1$ et $Z_2$ représentent un groupe de formule $CH_2—R_2$ et l'autre un groupe hydroxy réactif éventuellement éthérifié ou estérifié, ou un de ses tautomères et/ou sels ou en ce qu'on condense de façon intramoléculaire un composé de formule

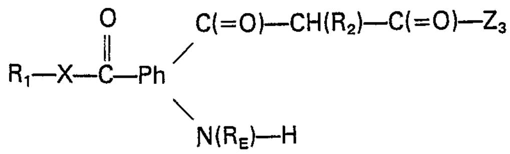

(IX)

où $Z_3$ représente un hydroxy ou un groupe hydroxy réactif modifié, ou un de ses tautomères et/ou sels ou en ce que dans un composé de formule

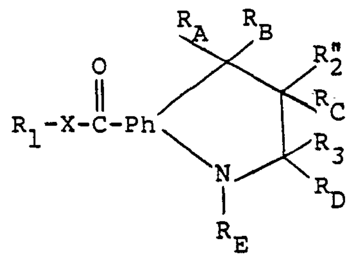

(XI),

où $R''_2$ représente un radical transformé en le groupe carboxy éventuellement estérifié ou amidé $R_2$, ou dans un de ses tautomères et/ou sels, on transforme le groupe $R''_2$ en le groupe carboxy éventuellement estérifié ou amidé $R_2$ ou en ce que dans un composé de formule

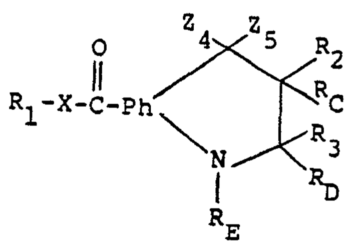

(XIII),

où $Z_4$ représente un radical transformable en hydroxy ou alcoxy inférieur et $Z_5$ représente ensemble avec $R_C$ ainsi que $R_D$ ensemble avec $R_E$ à chaque fois une liason supplémentaire, ou bien où $Z_4$ et $Z_5$ représentent chacun un radical monovalent ou ensemble un radical bivalent transformable en oxo et $R_C$ représente avec $R_D$ une liaison supplémentaire, ou dans un de ses sels on transforme un radical $Z_4$ transformable en hydroxy ou, selon les cas, en alcoxy inférieur, en hydroxy ou alcoxy inférieur ou, selon les cas, on transforme ensemble en oxo des radicaux $Z_4$ et $Z_5$ transformables en oxo ou en ce que dans un composé de formule

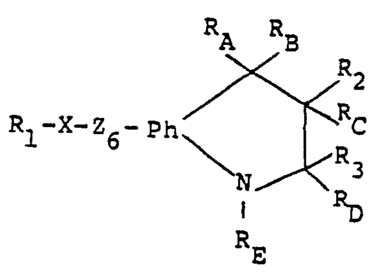

(XIX),

où $Z_6$ représente un radical transformable en le groupe carbonyle ou un de ses sels, on transforme $Z_6$ en le groupe carbonyle ou en ce que dans un comosé de formule

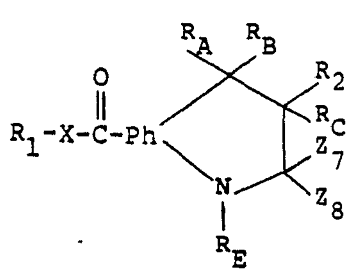

(XXI),

où $Z_7$ représente un radical transformable en hydroxy et $Z_8$ un radical $R_D$ ou $Z_7$ et $Z_8$ représentent à chaque fois un radical monovalent ou ensemble un radical bivalent transformable en oxo, on transforme $Z_7$ en hydroxy ou, selon les cas, $Z_7 + Z_8$ en oxo ou en ce que pour préparer des composés de formule (I) où X représente un oxy, dans un composé de formule

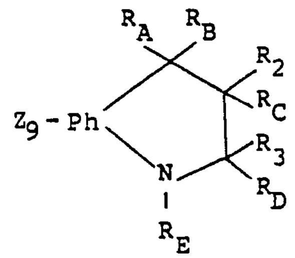

(XXIII),

où $Z_9$ représente un group carboxy fonctionnellement modifié différent du groupe $R_1—X—(=O)—$, on solvolyse $Z_9$ pour donner un groupe $R_1—O—C(=O)—$ correspondant ou en ce que pour préparer des composés de formule (I) où X représente une liaison directe, on fait réagir un composé de formule

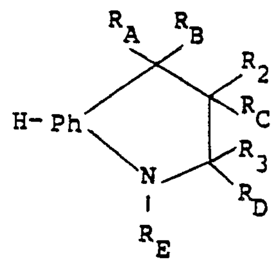

(XXV),

en présence d'un acide de Lewis avec un composé de formule

$R_1—COOH$ (XXVI)

ou d'un de ses dérivés fonctionnels appropriés ou en ce que pour préparer des composés de formule (I) où X est une liaison directe, on fait réagir un composé de formule XXVII

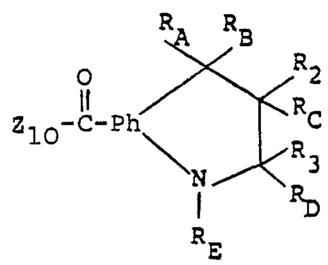

(XXVII),

où $Z_{10}$ représente un groupe hydroxy libre, éthérifié ou estérifié réactif, ou un groupe hétéroaryle approprié, lié par l'intermédiaire d'un atome d'azote du cycle, ou un de ses sels, avec un composé de formule

$R_1—M$ (XXVIII)

où M représente un radical métallique, pour donner un composé correspondant de formule (I) où $R_1$ est différent d'un hydrogène, ou un composé de formule (XXVII), où $Z_{10}$ est un hydroxy estérifié réactif, avec du boranate de bis-(triphénylphine)-cuivre pour donner un composé de formule (I) où $R_1$ est un hydrogène, et si on le désire on transforme le composé ainsi obtenu en un autre composé de formule (I) et/ou un composé salificateur obtenu en un sel ou un sel obtenu en le composé libre ou en un autre sel.

29. Composé de formule

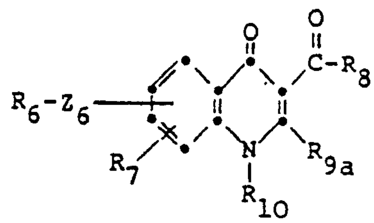

(XIXa),

où $Z_6$ représente un hydroxyméthylène et $R_6$ un alcoyle inférieur ayant jusqu'à 7 atomes de carbone compris, $R_7$ représente un hydrogène ou un alcoyle inférieur ayant jusqu'à 4 atomes de carbone compris, où le groupe $R_6$—$Z_6$ prend la position 6 et un groupe alcoyle inférieur $R_7$ prend la position 7, $R_2$ représente un hydroxy ou un alcoxy inférieur ayant jusqu'à 4 atomes de carbone compris, $R_{98}$ représente un hydroxy et $R_{10}$ représente un alcoyle inférieur ou un alcényle inférieur ayant jusqu'à 4 atomes de carbone compris, ou selon les cas le tautomère de 2-oxo-4-hydroxy-1,2-dihydroquinoléine ou de 2,4-dioxo-1,2,3,4-tétrahydroquinoléine et/ou un de ses sels.

30. Ester éthylique de l'acide 1-éthyl-4-hydroxy-6-(1-hydroxybutyl)-7-méthyl-carbostiryl-3-carboxylique selon la revendication 29.

31. Composé selon l'une des revendications 29 et 30 pour application dans un procédé de traitement prophylactique et/ou thérapeutique du corps animal ou humain.

32. Composé selon l'une des revendications 29 et 30 pour application selon la revendication 31 comme agent anti-allergique.

33. Préparations pharmaceutiques contenant un composé selon l'une des revendications 29 et 30, le cas échéant sous la forme d'un sel pharmaceutiquement acceptable, outre les additifs et supports pharmaceutiques habituels.

34. Procédé de préparation de dérivés de quinolinone de formule (XIXa) selon la revendication 29 et de ses sels, caractérisé en ce qu'on cyclise de façon intramoléculaire un composé de formule

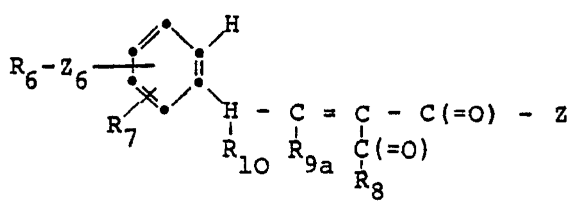

(IVa),

où Z représente un radical séparable, ou un de ses tautomères et/ou sels ou en ce qu'on cyclise de façon intramoléculaire un composé de formule

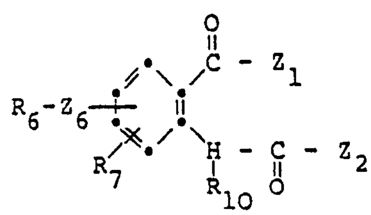

(VIa),

où l'un des radicaux $Z_1$ et $Z_2$ représentent un groupe de formule $CH_2$—C(=O)—$R_8$ et l'autre un groupe hydroxy éventuellement éthérifié ou estérifié réactif, ou un de ses tautomères et/ou sels, ou en ce qu'on condense de façon intramoléculaire un composé de formule

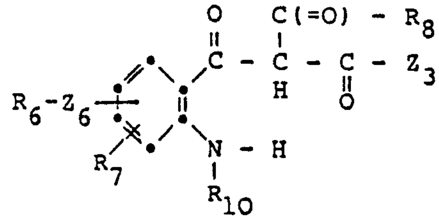

(IXa),

où $Z_3$ représente un hydroxy ou un groupe hydroxy réactif modifié ou un de ses tautomères et/ou sels ou en ce que dans un composé de formule

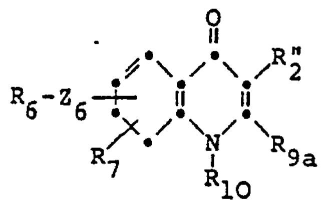

(XIa),

où $R''_2$ représente un radical transformable en le groupe carboxy éventuellement estérifié ou amidé —(C=O)—$R_8$, ou dans un de ses tautomères et/ou sels, on transforme le groupe $R''_2$ en le groupe carboxy éventuellement estérifié ou amidé —(C=O)—$R_8$ ou en ce que dans un composé de formules

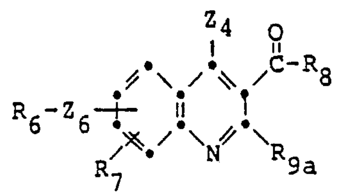

(XIIIa),

ou

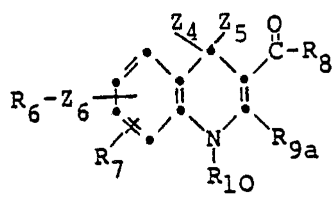

(XIIIb),

où $Z_4$ dans la formule (XIIIa) représente un radical transformable en hydroxy ou bien où dans la formule (XIIIb) $Z_4$ et $Z_5$ représentent chacun un radical monovalent ou ensemble un radical transformable en oxo, ou dans un de ses sels, on transforme un radical transformable en hydroxy $Z_4$ en hydroxy ou, selon les cas, on transforme ensemble les radicaux transformables en oxo $Z_4$ et $Z_5$ en oxo ou en ce que dans un composé de formules

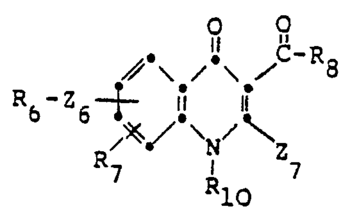

(XXIa),

ou

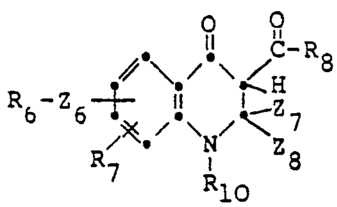

(XXIb),

où dans la formule (XXIa) $Z_1$ représente un radical transformable en hydroxy ou dans la formule (XXIb) $Z_7$ et $Z_8$ représentent à chaque fois un radical monovalent ou ensemble un radical bivalent transformable en oxo, on transforme $Z_7$ en hydroxy ou selon les cas $Z_7$ et $Z_8$ ensemble en oxo, ou en ce qu'on condense un composé de formule

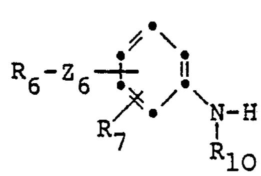

(Va)

avec un composé de formule

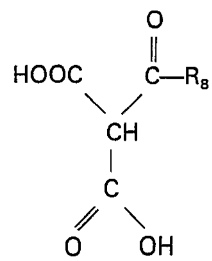

(XXa)

ou un de ses dérivés carboxy fonctionnels ou, selon les cas, un de ses sels, ou en ce que dans un composé de formule

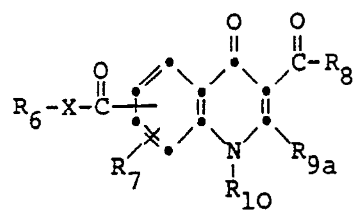

(II),

où X représente un liaison directe et $R_6$ un alcoyle inférieur ayant jusqu'à 7 atomes de carbone compris, on réduit le groupe de formule $R_6$—X—C(=O)— par traitement avec un réducteur approprié, comme un dihydrure de métaux légers, p. ex. le boranate de sodium ou le cyanoboranate de sodium ou avec un alcool secondaire, comme un alcanol secondaire ou un cycloalcanol, p. ex. avec l'isopropanol ou le cyclohexanol, en présence d'un alcoolate d'aluminium, p. ex. d'isopropanolate ou de cyclohexanolate d'aluminium, poor donner un groupe de formule $R_6$—$Z_6$ ou en ce qu'on fait réagir un aldéhyde de formule

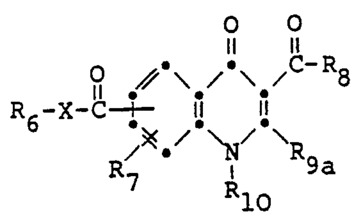

(II),

où $R_6$ représente un hydrogène et X est une liaison directe, avec un composé métallique d'un alcane inférieur ayant jusqu'à 7 atomes de carbone compris de formule $R_6$—H, comme un composé d'halogène magnésium ou de lithium de ce corps, p. ex. avec un alcoyle inférieur-lithium, et si on le désire, en ce que dans un composé de formule (XIXa) que l'on peut obtenir selon le procédé, on solvolyse un alcoxycarbonyle inférieur —C(=O)—$R_8$ en carboxy, on estérifie un carboxy —C(=O)—$R_8$ en alcoxycarbonyle inférieur et/ou en ce qu'on transforme un sel que l'on peut obtenir selon le procédé en le composé libre ou un composé libre que l'on peut obtenir selon le procédé en un sel.

35. Les composés que l'on peut obtenir selon le procédé d'une des revendications 28, 29 et 34.

36. Application de composés selon l'une des revendications 1 à 25 et 29 à 32, comme médicaments ou pour préparer des médicaments par un moyen non chimique.

**Revendications pour l'Etat contractant: AT**

1. Procédé de préparation de dérivés de quinoléine

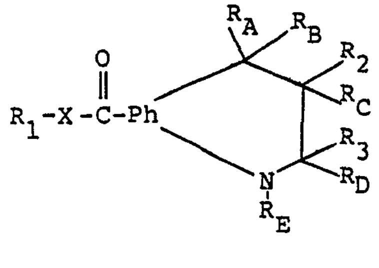

(I),

où $R_1$ représente un alcoyle inférieur éventuellement substitué par un hydroxy, alcoxy inférieur, hydroxyalcoxy inférieur, alcoxy inférieur-alcoxy inférieur, hydroxyalcoxy inférieur-alcoxy inférieur, alcoxy inférieur-alcoxy inférieur, alcoyle inférieur-thio, alcane inférieur sulfinyler ou alcane inférieur-sulfonyle, un

alcanoyloxyméthyle inférieur, un alcényle inférieur ou cycloalcoyle, un phényle ou phénylalcoyle inférieur éventuellement substitué dans la fraction phényle par un alcoyle inférieur, un alcoxy inférieur, et/ou un halogène, un furyle, thiényle ou pyridyle ou furyl-alcoyle inférieur, thiénylalcoyle inférieur, pyridylalcoyle inférieur ou hydrogène, X représente une liaison directe ou un oxy, Ph représente un 1,2-phénylène éventuellement substitué en outre par un alcoyle inférieur, alcoxy inférieur, hydroxy et/ou halogène, $R_2$ représente un carboxy, alcoxycarbonyle inférieur, hydroxycarbonyle inférieur, alcoxy inférieur-alcoxy inférieur-carbonyle, dialcoyle inférieur-aminoalcoxy inférieur, ou alkcoylène-mino inférieur à 5 ou 6 chaînons ou, selon les cas, asa- oxa- ou thiaalcoylène inférieur-aminoalcoxy inférieur-carbonyle ou un carboxy amidé, qui présente comme groupe amino un amino, hydroxyamino, alcoyle inférieur- amino, dialcoyle inférieur-amino ou alcoylène inférieur à 5 ou 6 chaînons -ou selon les cas asa, oxa-, thiaalcoylène-amino, et où ou bien $R_A$ et $R_B$ représentent ensemble un oxo, $R_C$ et $R_D$ représentent ensemble une liaison supplémentaire et $R_3$ est un hydroxy ou selon les cas $R_C$ est un hydrogène et $R_3$ et $R_D$ représentent ensemble un oxo et $R_E$ représente un hydrogène, alcoyle inférieur, hydroxy-alcoyle inférieur, alcoxy inférieur, alcoyle inférieur, dialcoyle inférieur-amino-alcoyle inférieur, alcoylène inférieur-amino à 5 à 7 chaînons -ou selon les cas asa-, oxa- ou thiaalcoylène-inférieur amino-alcoyle inférieur, alcényle inférieur, alcynyle inférieur, cycloalcoyle ayant de 3 à 8, de préférence de 5 à 7 chaînons ou un phénylalcoyle inférieur éventuellement substitué par un alcoyle inférieur, alcoxy inférieur et/ou halogène, un furylalcoyle inférieur, thiénylalcoyle inférieur ou pyridylalcoyle inférieur, ou $R_A$ représente un hydroxy ou un alcoxy inférieur $R_B$ représente avec $R_C$ ainsi que $R_D$ avec $R_E$ à chaque fois une liaison supplémentaire et $R_3$ est un hydroxy ou selon les cas $R_B$ et $R_C$ représentent ensemble une liaison supplémentaire et $R_3$ $R_D$ représentent ensemble un oxo et $R_E$ représente un hydrogène, alcoyle inférieur, hdroxyalcoyle inférieur, alcoxy inférieur-alcoyle inférieur, dialcoyle inférieur-amino-alcoyle inférieur, alcoylène inférieur à 5 à 6 chaînons-amino -ou selon les cas asa-, oxa-, ou thiaalcoylène inférieur-amino-alcoyle inférieur, alcényle inférieur, alcynyle inférieur, cycloalcoyle à 3 à 8 chaînons ou phénylalcoyle inférieur éventuellement substitué par alcoyle inférieur, alcoxy inférieur et/ou halogène, furylalcoyle inférieur, thiénylalcoyle inférieur ou pyridylalcoyle inférieur, où les radicaux "inférieur" présentent à chaque fois jusqu'à 7 atomes de carbone compris, un thiénylalcoyle inférieur ou un pyridylalcoyle inférieur, et de leurs sels, caractérisé en ce qu'on cyclise de façon intramoléculaire un composé de formule

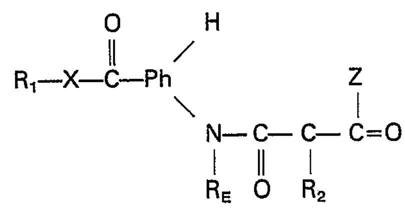

(IV),

où Z représente un radical séparable, ou un de ses tautomères et/ou sels ou en ce qu'on cyclise de façon intramoléculaire un composé de formule

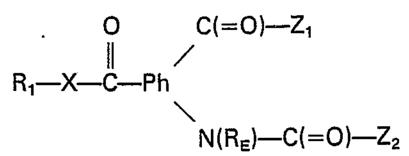

(VI)

où l'un des radicaux $Z_1$ et $Z_2$ représentent un groupe de formule $CH_2$—$R_2$ et l'autre un groupe hydroxy réactif éventuellement éthérifié ou estérifié, ou un de ses tautomères et/ou sels ou en ce qu'on condense de façon intramoléculaire un composé de formule

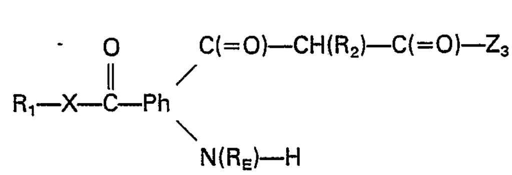

(IX)

où $Z_3$ représente un hydroxy ou un groupe hydroxy réactif modifié, ou un de ses tautomères et/ou sels ou en ce que dans un composé de formule

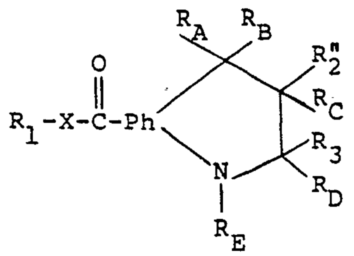

(XI),

où $R''_2$ représente un radical transformé en le groupe carboxy éventuellement estérifié ou amidé $R_2$, ou dans un de ses tautomères et/ou sels, on transforme le groupe $R''_2$ en le groupe carboxy éventuellement estérifié ou amidé $R_2$ ou en ce que dans un composé de formule

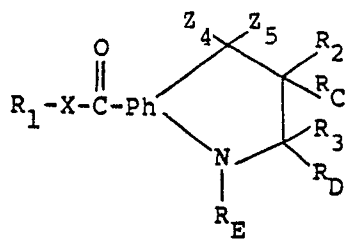

(XIII),

où $Z_4$ représente un radical transformable en hydroxy ou alcoxy inférieur et $Z_5$ représente ensemble avec $R_C$ ainsi que $R_D$ ensemble avec $R_E$ à chaque fois une liaison supplémentaire, ou bien où $Z_4$ et $Z_5$ représentent chacun un radical monovalent ou ensemble un radical bivalent transformable en oxo et $R_C$ représente avec $R_D$ une liaison supplémentaire, ou dans un de ses sels on transforme un radical $Z_4$ transfeormable en hydroxy ou, selon les cas, en alcoxy inférieur, en hydroxy ou alcoxy inférieur ou, selon les cas, on transforme ensemble en oxo des radicaux $Z_4$ et $Z_5$ transformables en oxo ou en ce que dans un composé de formule

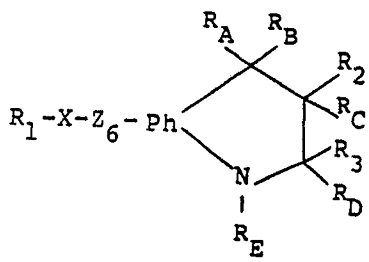

(XIX),

où $Z_6$ représente un radical transformable en le groupe carbonyle ou un de ses sels, on transforme $Z_6$ en le groupe carbonyle ou en ce que dans un composé de formule

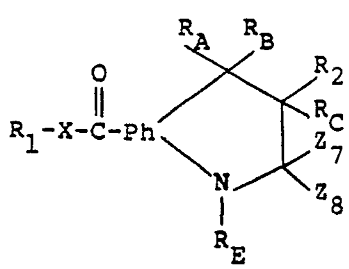

(XXI),

où $Z_7$ représente un radical transformable en hydroxy et $Z_8$ un radical $R_D$ ou $Z_7$ et $Z_8$ représentent à chaque fois un radical monovalent ou ensemble un radical bivalent transformable en oxo, on transforme $Z_7$ en hydroxy ou, selon les cas, $Z_7 + Z_8$ en oxo ou en ce que pour préparer des composés de formule (I) où X représente un oxy, dans un composé de formule

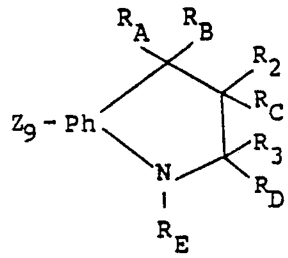

(XXIII),

où $Z_9$ représente un groupe carboxy fonctionnellement modifié différent du groupe $R_1$—X—(=O)—, on solvolyse $Z_9$ pour donner un groupe $R_1$—O—C(=O)— correspondant ou en ce que pour préparer des composés de formule (I) où X représente une liaison directe, on fait réagir un composé de formule

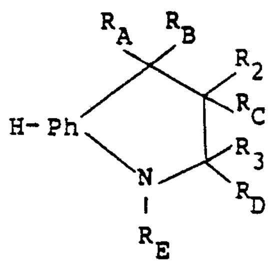

(XXV),

en présence d'un acide de Lewis avec un composé de formule

$R_1$—COOH (XXVI)

ou d'un de ses dérivés fonctionnels appropriés ou en ce que pour préparer des composés de formule (I) où X est une liaison directe, on fait réagir un composé de formule XXVII

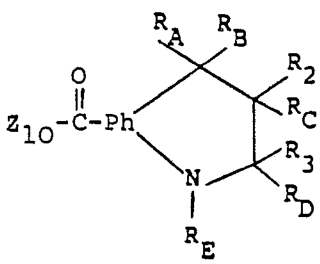

(XXVII),

où $Z_{10}$ représente un groupe hydroxy libre, éthérifié ou estérifié réactif, ou un groupe hétéroaryle approprié, lié par l'intermédiaire d'un atome d'azote du cycle, ou un de ses sels, avec un composé de formule

$R_1$—M (XXVIII)

où M représente un radical métallique, pour donner un composé correspondant de formule (I) où $R_1$ est différent d'un hydrogène, ou un composé de formule (XXVII), où $Z_{10}$ est un hydroxy estérifié réactif, avec du boranate de bis-(triphénylphine)-cuivre pour donner un composé de formule (I) où $R_1$ est un hydrogène, et si on le désire on transforme le composé ainsi obtenu en un autre composé de formule (I) et/ou un composé salificateur obtenu en un sel ou un sel obtenu en le composé libre ou en un autre sel.

2. Procédé selon la revendication 1, caractérisé en ce qu'on prépare un composé de formule

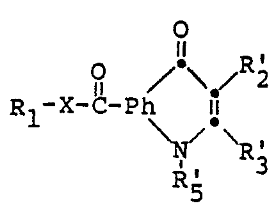

(Ia),

où $R_1$ représente un alcoyle inférieur ayant jusqu'à 7 atomes de carbone compris, un alcoxy inférieur-

alcoyle inférieur-thio-, alcane inférieure-sulfinyle- ou alcane inférieur-sulfonyle-alcoyle inférieur ayant à chaque fois jusqu'à 4 atomes de carbone dans les paties alcoyle, un alcényle inférieur ayant jusqu'à 4 atomes de carbone compris, un cycloalcoyle à 3 à 8 chaînons, un phényle éventuellement substitué par un alcoyle inférieur ayant jusqu'à 4 atomes de carbone, un alcoxy inférieur ayant jusqu'à 4 atomes de carbone et/ou un halogène de numéro atomique allant jusqu'à 35 compris ou, selon les cas, un phénylalcoyle inférieur ayant jusqu'à 4 atomes de carbone dans la partie alcoyle, un furyle, thiényle ou pyridyle ou un furyl-, thiényl- ou pyridylalcoyle inférieur ayant jusqu'à 4 atomes de carbone compris dans a partie alcoyle et X représente une liaison directe ou $R_1$ représente un hydrogène ou un alcoyle inférieur ayant jusqu'à 7 atomes de carbone compris et X représente un oxy et où Ph représente un 1,2-phénylène présentant le groupe $R_1—X—C(=O)—$ éventuellement substitué en outre par un alcoyle inférieur ayant jusqu'à 4 atomes de carbone compris, un alcoxy inférieur ayant jusqu'à 4 atomes de carbone compris, un hydroxy ou un halogène de numéro atomique allant jusqu'à 35 compris, $R'_2$ représente un carboxy, un alcoxy inférieur-carbonyle ayant jusqu'à 5 atomes de carbone compris, un hydroxyalcoxy inférieur-carbonyle ayant jusqu'à 5 atomes de carbone compris, un alcoxy inférieur-alcoxy inférieur-carbonyle ayant jusqu'à 4 atomes de carbone dans les parties alcoyle, un dialcoyle inférieur-amino-alcoxy inférieur-carbonyle ayant à chaque fois jusqu'à 4 atomes de carbone dans la partie alcoyle et alcoxy, un carbamoyle, N-hydroxycarbamoyle ou N-alcoyle inférieur ou selon les cas N,N-dialcoyle inférieur-carbamoyle ayant jusqu'à 4 atomes de carbone dans la partie alcoyle, $R'_3$ représente un hydroxy et $R'_5$ représente un hydrogène, un alcoyle inférieur ou un alcényle inférieur ayant jusqu'à 4 atomes de carbone compris, un phényle éventuellement substitué par un alcoyle inférieur ayant jusqu'à 4 atomes de crbone compris, un alcoxy inférieur ayant jusqu'à 4 atomes de carbone compris et/ou un halogène de numéro atomique allant jusqu'à 35 compris, un furyl-, thiényl- ou pyridylalcoyle inférieur ayant jusqu'à 4 atomes de carbone compris dans la partie alcoyle, un alcoxy inférieur-alcoyle inférieur ayant jusqu'à 4 atomes de carbone compris dans la partie alcoxy ou, selon les cas, alcoyle ou un dialcoyle inférieur-amino-alcoyle inférieur, où alcoyle inférieur présente jusqu'à 4 atomes de carbone compris, ou un de ses tautomères ou sels.

3. Procédé selon la revendication 1, caractérisé en ce qu'on prépare un composé de formule

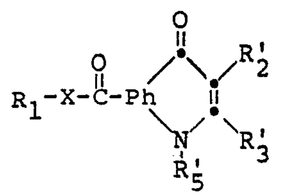

(Ia),

où $R_1$ représente un hydrogène, un alcoyle inférieur ayant jusqu'à 7 atomes de carbone compris, un alcoxy inférieur-, alcoyle inférieur-thio-, alcane inférieur-sulfinyle- ou alcane inférieur-sulfonyle-alcoyle inférieur ayant à chaque fois jusqu'à 4 atomes de carbone dans les parties alcoyle, un alcényle inférieur ayant jusqu'à 4 atomes de carbone compris ou un cycloalcoyle à 3 à 8 chaînons et X représente une liaison directe ou $R_1$ représente un hydrogène, un alcoyle inférieur ayant jusqu'à 7 atomes de carbone compris, un alcoxy inférieur-, alcoyle inférieur-thio-, alcane inférieur-sulfonyle- ou alcane inférieur-sulfinyle-alcoyle inférieur ayant à chaque fois jusqu'à 4 atomes de carbone compris dans les parties alcoyle, un alcényle inférieur ayant jusqu'à 4 atomes de carbone compris, ou un cycloalcoyle à 3 à 8 chaînons, un alcanoyloxy inférieur-méthyle ayant jusqu'à 7 atomes de carbone dans la partie alcanoyle ou un ω(ω-hydroxyalcoxy inférieur)-alcoyle inférieur, ω-(ω-alcoxy inférieur-alcoxy inférieur)-alcoyle inférieur, ω-[ω-hydroxyalcoxy inférieur)-alcoxy inférieur]- alcoyle inférieur ou ω-[ω-(ω-alcoxy inférieur-alcoxy inférieur)-alcoxy inférieur]-alcoyle inférieur ayant à chaque fois de 1 à 4 atomes de carbone dans la partie alcoyle et de 2 à 4 atomes de carbone dans la partie alcoylène et X représente un oxy, Ph représente un 1,2-phénylène présentant le groupe $R_1—X—C(=O)—$, éventuellement substitué de façon additionnelle par un alcoyle inférieur ayant jusqu'à 4 atomes de carbone compris, un alcoxy inférieur ayant jusqu'à 4 atomes de carbone compris, un hydroxy ou un halogène dont le numéro atomique va jusqu'à 35 compris, $R'_2$ représente un carboxy, un alcoxy inférieur-carbonyle ayant jusqu'à 5 atomes de carbone compris, un alcoxy inférieure-alcoxy inférieur-carbonyle ayant jusqu'à 4 atomes de carbone compris dans les parties alcoyle, un carbamoyle, un N-hydroxycarbamoyle ou un N-alcoyle inférieur- ou, selon les cas, N,N-dialcoyle inférieur-carbamoyle ayant jusqu'à 4 atomes de carbone compris dans la partie alcoyle, $R'_3$ représente un hydroxy et $R'_5$ représente un hydrogène, un alcoyle inférieur, un alcényle inférieur ou un alcynyle inférieur ayant jusqu'à 4 atomes de carbone compris, ainsi que ses tautomères où le groupe hydroxy $R'_3$ se présente sous la forme oxo-tautomère, ou un de ses sels ou tautomères.

4. Procédé selon la revendication 1, caractérisé en ce qu'on prépare un composé de formule

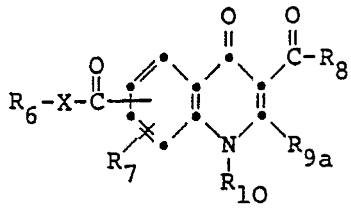 (II),

où X représente une liaison directe et $R_6$ représente un hydrogène ou un alcoyle inférieur ayant jusqu'à 7 atomes de carbone compris ou X représente un oxy et $R_6$ un hydrogène, un alcoyle inférieur ayant jusqu'à 7 atomes de carbone compris, un ω-hydroxy-alcoyle inférieur ayant de 2 à 4 atomes de carbone, un alcanoyloxy inférieur-méthyle ayant jusqu'à 7 atomes de carbone compris dans la partie alcanoyle ou un ω-(ω-hydroxyalcoxy inférieur)-alcoyle inférieur, ω-(ω-alcoxy inférieur-alcoxy inférieur)-alcoyle inférieur, ω-[ω-(ω-hydroxyalcoxy inférieur)-alcoxy inférieur]-alcoyle inférieur ou ω-[ω(ω-alcoxy inférieur-alcoxy inférieur)-alcoxy inférieur]-alcoyle inférieur avec à chaque fois de 1 à 4 atomes de carbone dans la partie alcoyle et de 2 à 4 atomes de carbone dans la patie alcoylène, $R_7$ représente un hydrogène, un alcoyle inférieur ayant jusqu'à 4 atomes de carbone compris, un alcoxy inférieur ayant jusqu'à 4 atomes de carbone compris, un halogène dont le numéro atomique va jusqu'à 35 ou un hydroxy, $R_8$ représente un hydroxy ou un alcoxy inférieur ayant jusqu'à 4 atomes de carbone compris, $R_{9a}$ représente un hydroxy, et $R_{10}$ représente un alcoyle inférieur, un alcényle inférieur ou un alcynyle inférieur avec jusqu'à 4 atomes de carbone compris, où un composé de 2-hydroxy-4-oxo-1,4-dihydroquinoléine de formule (II) peut également se présenter sous la forme tautomère 2-oxo-4-hydroxy-1,2-dihydro- ou 2,4-dioxo-1,2,3,4-tétrahydroquinoléine, ou un de ses sels.

5. Procédé selon la revendication 4 caractérisé en ce qu'on prépare un composé de formule (II) où X représente un oxy, le groupe $R_6$—O—C(=O)— est lié en position 6, $R_6$ représente un hydrogène, un alcoyle inférieur ayant jusqu'à 7 atomes de carbone, un ω-hydroxyalcoyle inférieur ayant de 2 à 4 atomes de carbone, un alcanoyloxy inférieur-méthyle ayant jusqu'à 7 atomes de carbone dans la partie alcoyle ou un ω-(ω-hydroxy alcoxy inférieur)-alcoyle inférieur, ω-(ω-alcoxy inférieur-alcoxy inférieur)-alcoyle inférieur, ω-[ω-ω-[ω-(ω-hydroxyalcoxy inférieur)-alcoxy inférieur)-alxoyle inférieur ou ω-[ω-(ω-alcoxy inférieur-alcoxy inférieur)-alcoxy inférieur]-alcoyle inférieur avec à chaque fois de 1 à 4 atomes de carbone dans la partie alcoyle et de 2 à 4 atomes de carbone dans la partie alcoylène, $R_7$ représente un hydrogène et $R_{10}$ représente un alcényle inférieur ayant jusqu'à 4 atomes de carbone compris, ou X représente une liaison directe, $R_6$ représente un hydrogène ou un alcoyle inférieur ayant jusqu'à 7 atomes de carbone compris, $R_7$ représente un alcoyle inférieur ayant jusqu'à 4 atomes de carbone compris, où le groupe $R_6$—C(=O)— prend la position 6 et le groupe alcoyle inférieur $R_7$ prend la position 7, et $R_{10}$ représente un alcoyle inférieur ayant jusqu'à 4 atomes de carbone compris, où $R_8$ et $R_{9a}$ ont à chaque fois les significations données dans la revendication 5, ou selon les cas le tautomère 2-oxo-4-hydroxy-1,2-dihydroquinoléine ou 2,4-dioxo-1,2,3,4-tétrahydroquinoléine et/ou un de ses sels.

6. Procédé selon la revendication 4, caractérisé en ce qu'on prépare un composé de formule (II) où X représente une liaison directe, $R_6$ représente un alcoyle inférieur ayant jusqu'à 6 atomes de carbone compris, $R_7$ représente un hydrogène ou un alcoyle inférieur ayant jusqu'à 4 atomes de carbone compris, où le groupe $R_6$—C(=O)— prend la position 6 et un groupe alcoyle $R_7$ prend la position 7, $R_8$ et $R_{9a}$ représentent un hydroxy et $R_{10}$ représente un alcoyle inférieur ou un alcényle inférieur ayant jusqu'à 4 atomes de carbone compris, ou, selon les cas, le tautomère de 2-oxo-4-hydroxy-1,2-dihydroquinoléine ou 2,4-dioxo-1,2,3,4-tétrahydroquinoléine et/ou un de ses sels.

7. Procédé selon la revendication 4, caractérisé en ce qu'on prépare un composé de formule (II), où X représente un oxy, $R_6$ représente un hydrogène ou un alcoyle inférieur ayant jusqu'à 7 atomes de carbone compris, $R_7$ représente un hydrogène ou un alcoyle inférieur ayant jusqu'à 4 atomes de carbone compris, où le groupe $R_6$—O—C(=O)— prend la position 6 et un groupe alcoyle inférieur $R_7$ prend la position 7, $R_8$ et $R_{9a}$ ont les significations données dans la revendiction 5, et $R_{10}$ représente un alcoyle inférieur ou un alcényle inférieur ayant jusqu'à 4 atomes de carbone compris, ou, selon les cas, le tautomère de 2-oxo-4-hydroxy-1,2-dihydroquinoléine ou 2,4-dioxo-1,2,3,4-tétrahydroquinoléine et/ou un de ses sels.

8. Procédé selon la revendication 1, caractérisé en ce qu'on prépare l'ester étrhylique de l'acide 6-butyryl-4-hydroxy-1,7-diméthyl-carbostiryl-3-carboxylique.

9. Procédé selon la revendication 1, caractérisé en ce qu'on prépare l'éster éthylique de l'acide 6-butyryl-4-hydroxy-7-methyl-1-propyl-carbostiryl-3-carboxylique.

10. Procédé selon la revendication 1, caractérisé en ce qu'on prépare l'éster éthylique de l'acide 1-éthyl-6-butyryl-4-hydroxy-7-méthyl-carbostiryl-3-carboxylique.

11. Procédé selon la revendication 1, caractérisé en ce qu'on prépare l'éster éthylique de l'acide 1-allyl-6-butyryl-4-hydroxy-7-méthyl-carbostiryl-3-carboxylique.

12. Procédé selon la revendication 1, caractérisé en ce qu'on prépare l'éster éthylique de l'acide 1-éthyl-6-butyryl-4-hydroxy-carbostiryl-3-carboxylique.

13. Procédé selon la revendication 1, caractérisé en ce qu'on prépare l'éster éthylique de l'acide 1-butyl-6-butyryl-4-hydroxy-7-méthyl-carbostiryl-3-carboxylique.

14. Procédé selon la revendication 1, caractérisé en ce qu'on prépare l'éster éthylique de l'acide 1-allyl-6-méthoxycarbonyl-4-hydroxy-carbostiryl-3-carboxylique.

15. Procédé selon la revendication 1, caractérisé en ce qu'on prépare l'éster éthylique de l'acide 1-allyl-6-carboxy-4-hydroxy-carbostiryl-3-carboxylique, ou un de ses sels.

16. Procédé selon la revendication 1, caractérisé en ce qu'on prépare le tert.-butylester de l'acide 1-éthyl-6-butyryl-4-hydroxy-7-méthyl-carbostiryl-3-carboxylique.

17. Procédé selon la revendication 1, caractérisé en ce qu'on prépare l'acide 1-éthyl-6-butyryl-4-hydroxy-7-méthyl-carbostiryl-3-carboxylique ou un de ses sels.

18. Procédé selon la revendication 1, caractérisé en ce qu'on prépare l'éster éthylique de l'acide 6-méthoxycarbonyl-1-propargyl-4-hydroxy-carbostiryl-3-carboxylique ou un de ses sels.

19. Procédé selon la revendication 1, caractérisé en ce qu'on prépare le 1-éthyl-6-butyryl-4-hydroxy-7-méthyl-carbostiryl-3-carboxamide.

20. Procédé selon la revendication 1, caractérisé en ce qu'on prépare l'éster éthylique de l'acide 1-éthyl-6-formyl-4-hydroxy-7-méthyl-carbostiryl-3-carboxylique.

21. Procédé selon la revendication 1, caractérisé en ce qu'on prépare l'éster éthylique de l'acide 1-éthyl-6-méthoxycarbonyl-4-hydroxy-carbostiryl-3-carboxylique.

22. Procédé selon la revendication 1, caractérisé en ce qu'on prépare l'éster éthylique de l'acide 1-éthyl-6-carboxy-4-hydroxy-carbostiryl-3-carboxylique.

23. Procédé de préparation de préparations pharmaceutiques, caractérisé en ce qu'on met sous une forme appropriée à l'administration pharmaceutique, un composé que l'on peut obtenir selon l'une des revendications 1 à 22, éventuellement sous la forme d'un sel pharmaceutiquement acceptable, en le mélangeant avec des additifs et supports pharmaceutiques habituels.

24. Procédé selon la revendication 23 de préparation d'agents anti-allergiques.

25. Procédé de préparation de nouveaux dérivés de quinolinone de formule

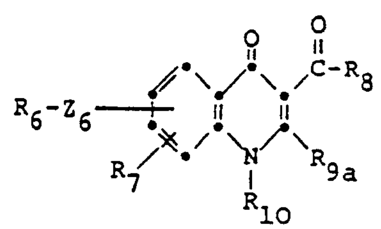

(XIXa),

où $Z_6$ représente un hydroxyméthylène et $R_6$ ou alcoyle inférieur ayant jusqu'à 7 atomes de carbone compris, $R_7$ représente un hydrogène ou un alcoyle inférieur ayant jusqu'à 4 atomes de carbone compris, où le groupe $R_6—Z_6$ prend la position 6 et un groupe alcoyle inférieur $R_7$ prend la position 7, $R_8$ représente un hydroxy ou un alcoxy inférieur ayant jusqu'à 4 atomes de carbone compris, $R_{9a}$ représente un hydroxy et $R_{10}$ représente un alcoyle inférieur ou un alcényle inférieur ayant jusqu'à 4 atomes de carbone compris, ou selon les cas leurs tautomères de 2-oxo-4-hydroxy-1,2-dihydroquinoléine ou de 2,4-dioxo-1,2,3,4-tétrahydroquinoléine et/ou un de ses sels, caractérisé en ce qu'on cyclise de façon intramoléculaire un composé de formule (IVa)

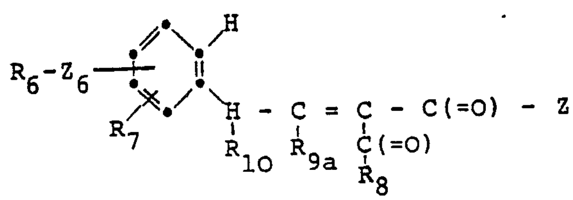

(IVa),

où Z représente un radical séparable, ou un de ses tautomères et/ou sels ou en ce qu'on cyclise de façon intramoléculaire un composé de formule

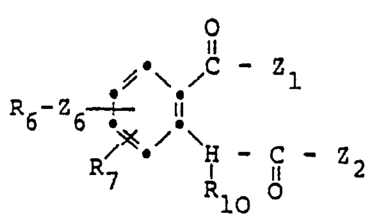

(VIa),

où l'un des radicaux $Z_1$ et $Z_2$ représentent un groupe de formule $CH_2—C(=O)—R_8$ et l'autre un groupe

hydroxy éventuellement éthérifié ou estérifié réactif, ou un de ses tautomères et/ou sels, ou en ce qu'on condense de façon intramoléculaire un composé de formule

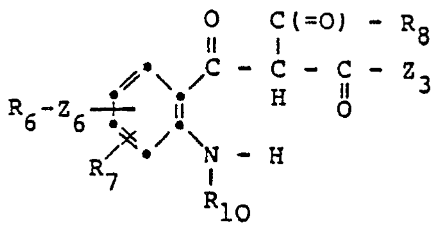

(IXa),

où $Z_3$ représente un hydroxy ou un groupe hydroxy réactif modifié ou un de ses tautomères et/ou sels ou en ce que dans un composé de formule

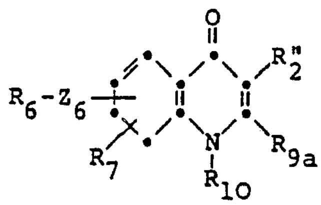

(XIa),

où $R''_2$ représente un radical transformable en le groupe carboxy éventuellement estérifié ou amidé $-C(=O)-R_8$, ou dans un de ses tautomères et/ou sels, on transforme le groupe $R''_2$ en le groupe carboxy éventuellement estérifié ou amidé $-C(=O)-R_8$ ou en ce que dans un composé de formules

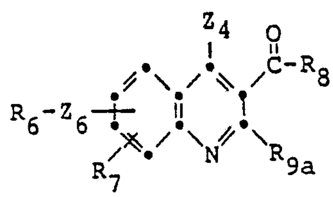

(XIIIa),

ou

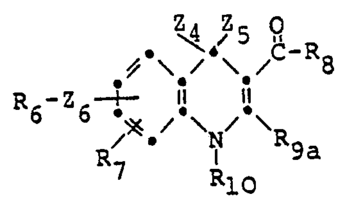

(XIIIb),

où $Z_4$ dans la formule (XIIIa) représente un radical transformable en hydroxy ou bien où dans la formule (XIIIb) $Z_4$ et $Z_5$ représentent chacun un radical monovalent ou ensemble un radical transformable en oxo, ou dans un de ses sels, on transforme un radical transformable en hydroxy $Z_4$ en hydroxy ou, selon les cas, on transforme ensemble les radicaux transformables en oxo $Z_4$ et $Z_5$ en oxo ou en ce que dans un composé de formules

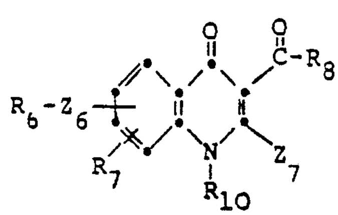

(XXIa),

ou

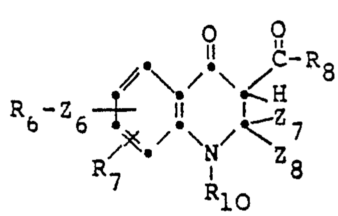

(XXIb),

où dans la formule (XXIa) $Z_1$ représente un radical transformable en hydroxy ou dans la formule (XXIb) $Z_7$ et $Z_8$ représentent à chaque fois un radical monovalent ou ensemble un radical bivalent transformable en oxo, on transforme $Z_7$ en hydroxy ou selon les cas $Z_7$ et $Z_8$ ensemble en oxo, ou en ce qu'on condense un composé de formule

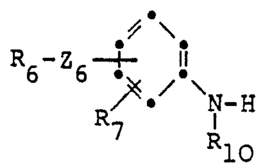

(Va)

avec un composé de formule

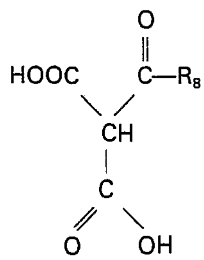

(XXa)

ou un de ses dérivés carboxy fonctionnels ou, selon les cas, un de ses sels, ou en ce que dans un composé de formule

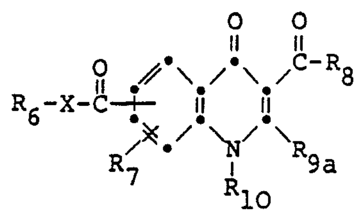

(II),

où X représente une liaison directe et $R_6$ un alcoyle inférieur ayant jusqu'à 7 atomes de carbone compris, on réduit le groupe de formule $R_6$—X—C(=O)— par traitement avec un réducteur approprié, comme un dihydrure de métaux légers, p. ex. le boranate de sodium ou le cyanoboranate de sodium ou avec un alcool secondaire, comme un alcanol secondaire ou un cycloalcanol, p. ex. avec l'isopropanol ou le cyclohexanol, en présence d'un alcoolate d'aluminium p. ex. d'isopropanolate ou de cyclohexanolate d'aluminium, pour donner un groupe de formule $R_6$—$Z_6$ ou en ce qu'on fait réagir un aldéhyde de formule

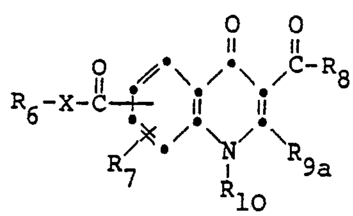

(II),

où $R_6$ représente un hydrogène et X est une liaison directe, avec un composé métallique d'un alcane inférieur ayant jusqu'à 7 atomes de carbone compris de formule $R_6$—H, comme un composé d'halogène magnésium ou de lithium de ce corps, p. ex. avec un alcoyle inférieur-lithium, et si on le désire, en ce que dans un composé de formule (XIXa) que l'on peut obtenir selon le procédé, on solvolyse un alcoxy-carbonyle inférieur —C(=O)—$R_8$ en carboxy, on estérifie un carboxy —C(=O)—$R_8$ en alcoxycarbonyle inférieur et/ou en ce qu'on transforme un sel que l'on peut obtenir selon le procédé en le composé libre ou un composé libre que l'on peut obtenir selon le procédé en un sel.

26. Procédé selon la revendication 25, caractérisé en ce qu'on prépare l'ester éthylique de l'acide 1-éthyl-4-hydroxy-6-(1-hydroxybutyl)-7-méthyl-carbostiryl-3-carboxylique.

27. Procédé de préparation de préparations pharmaceutiques, caractérisé en ce qu'on met sous une forme appropriée à l'administration pharmaceutique un composé que l'on peut obtenir selon l'une des revendications 25 et 26 ou un de ses sels pharmaceutiquement acceptables en le mélangeant avec des additifs et supports pharmaceutiques habituels.